# EUROPEAN PATENT SPECIFICATION

(11) **EP 1 544 299 B1**
(45) Date of publication and mention of the grant of the patent: **17.12.2008**
(21) Application number: 05001328.3
(22) Date of filing: 23.05.2000
(51) Int. Cl.: C12N 15/12, C12N 15/62, C12N 5/10, C07K 14/71, A61K 38/17, A61P 43/00

(54) **VEGF receptor chimeras for the treatment of eye disorders characterized by vascular permeability**
VEGF-Rezeptorchimären zur behandlung von Augenkrankheiten, welche durch vaskuläre Permeabilität charakterisiert sind.
Chimère du récepteur de VEGF pour le traitement de maladies des yeux caracterisées par la perméabilité vasculaire

(30) Priority: 08.06.1999 US 138133 P
(43) Date of publication of application: 22.06.2005
(62) Divisional of application: 00932721.4
(73) Proprietor: REGENERON PHARMACEUTICALS, INC., Tarrytown, NY 10591-6707 (US)
(72) Inventor: Papadopoulos, Nicholas J., Butler, NJ 07405 (US); Wiegand, Stanley J., Croton-on-Hudson, NY 10520 (US)
(74) Representative: Duckworth, Timothy John

(56) References cited:
- WO-A-97/44453
- SAISHIN Y ET AL. : "VEGF-TRAPR1R2 suppresses choroidal neovascularisation and VEGF-induced breakdown of the blood-retinal barrier" J. CELLULAR PHYSIOLOGY, vol. 195, 2003, pages 241-248,
- WANG Q. ET AL.: "The expression of angiogenic factors and their receptors during retinal neovascularization: effects of VEGF Trap administration." INVEST. OPHTALMOL. VIS. SCI., vol. 44, 2003, page E2902,
- CAO J. ET AL.: "Inhibition of corneal neovascularization and inflammation by VEGF Trap" INVEST. OPHTALMOL. VIS. SCI., vol. 43, 2002, page E1863,
- BOULTON M., FOREMAN D., WILLIAMS G AND MCLEOD D.: "VEGF localisation in diabetic retinopathy." BR J OPHTHALMOL, vol. 82, 1998, pages 561-568,

### SEQUENCE LISTING

<110> Regeneron Pharmaceuticals, Inc.
<120> USE OF MODIFIED CHIMERIC POLYPEPTIDES
<130> N.79986C TJD
<150> PCT/US00/14142
   <151> 2000-05-23
<150> 60/138,133
   <151> 1999-06-08
<160> 36
<170> FastSEQ for Windows Version 3.0
<210> 1
   <211> 1704
   <212> DNA
   <213> Homo sapiens
<220>
   <221> CDS
   <222> (1)...(1701)
<400> 1
   atg gtc agc tac tgg gac acc ggg gtc ctg ctg tgc gcg ctg ctc agc       48
   Met Val Ser Tyr Trp Asp Thr Gly Val Leu Leu Cys Ala Leu Leu Ser
    1               5                  10                  15
   tgt ctg ctt ctc aca gga tct agt tca ggt tca aaa tta aaa gat cct       96
   Cys Leu Leu Leu Thr Gly Ser Ser Ser Gly Ser Lys Leu Lys Asp Pro
               20                  25                  30
   gaa ctg agt tta aaa ggc acc cag cac atc atg caa gca ggc cag aca      144
   Glu Leu Ser Leu Lys Gly Thr Gln His Ile Met Gln Ala Gly Gln Thr
           35                  40                  45
   ctg cat ctc caa tgc agg ggg gaa gca gcc cat aaa tgg tct ttg cct      192
   Leu His Leu Gln Cys Arg Gly Glu Ala Ala His Lys Trp Ser Leu Pro
       50                  55                  60
   gaa atg gtg agt aag gaa agc gaa agg ctg agc ata act aaa tct gcc      240
   Glu Met Val Ser Lys Glu Ser Glu Arg Leu Ser Ile Thr Lys Ser Ala
   65                  70                  75                  80
   tgt gga aga aat ggc aaa caa ttc tgc agt act tta acc ttg aac aca      288
   Cys Gly Arg Asn Gly Lys Gln Phe Cys Ser Thr Leu Thr Leu Asn Thr
                   85                  90                  95
   gct caa gca aac cac act ggc ttc tac agc tgc aaa tat cta gct gta      336
   Ala Gln Ala Asn His Thr Gly Phe Tyr Ser Cys Lys Tyr Leu Ala Val
               100                 105                 110
   cct act tca aag aag aag gaa aca gaa tct gca atc tat ata ttt att      384
   Pro Thr Ser Lys Lys Lys Glu Thr Glu Ser Ala Ile Tyr Ile Phe Ile
           115                 120                 125
   agt gat aca ggt aga cct ttc gta gag atg tac agt gaa atc ccc gaa      432
   Ser Asp Thr Gly Arg Pro Phe Val Glu Met Tyr Ser Glu Ile Pro Glu
       130                 135                 140
   att ata cac atg act gaa gga agg gag ctc gtc att ccc tgc cgg gtt 480
   Ile Ile His Met Thr Glu Gly Arg Glu Leu Val Ile Pro Cys Arg Val
   145 150 155 160
   acg tca cct aac atc act gtt act tta aaa aag ttt cca ctt gac act 528
   Thr Ser Pro Asn Ile Thr Val Thr Leu Lys Lys Phe Pro Leu Asp Thr
   165 170 175
   ttg atc cct gat gga aaa cgc ata atc tgg gac agt aga aag ggc ttc 576
   Leu Ile Pro Asp Gly Lys Arg Ile Ile Trp Asp Ser Arg Lys Gly Phe
   180 185 190
   atc ata tca aat gca acg tac aaa gaa ata ggg ctt ctg acc tgt gaa 624
   Ile Ile Ser Asn Ala Thr Tyr Lys Glu Ile Gly Leu Leu Thr Cys Glu
   195 200 205
   gca aca gtc aat ggg cat ttg tat aag aca aac tat ctc aca cat cga 672
   Ala Thr Val Asn Gly His Leu Tyr Lys Thr Asn Tyr Leu Thr His Arg
   210 215 220
   caa acc aat aca atc ata gat gtc caa ata agc aca cca cgc cca gtc 720
   Gln Thr Asn Thr Ile Ile Asp Val Gln Ile Ser Thr Pro Arg Pro Val
   225 230 235 240
   aaa tta ctt aga ggc cat act ctt gtc ctc aat tgt act gct acc act 768
   Lys Leu Leu Arg Gly His Thr Leu Val Leu Asn Cys Thr Ala Thr Thr
   245 250 255
   ccc ttg aac acg aga gtt caa atg acc tgg agt tac cct gat gaa aaa 816
   Pro Leu Asn Thr Arg Val Gln Met Thr Trp Ser Tyr Pro Asp Glu Lys
   260 265 270
   aat aag aga gct tcc gta agg cga cga att gac caa agc aat tcc cat 864
   Asn Lys Arg Ala Ser Val Arg Arg Arg Ile Asp Gln Ser Asn Ser His
   275 280 285
   gcc aac ata ttc tac agt gtt ctt act att gac aaa atg cag aac aaa 912
   Ala Asn Ile Phe Tyr Ser Val Leu Thr Ile Asp Lys Met Gln Asn Lys
   290 295 300
   gac aaa gga ctt tat act tgt cgt gta agg agt gga cca tca ttc aaa 960
   Asp Lys Gly Leu Tyr Thr Cys Arg Val Arg Ser Gly Pro Ser Phe Lys
   305 310 315 320
   tct gtt aac acc tca gtg cat ata tat gat aaa gca ggc ccg ggc gag 1008
   Ser Val Asn Thr Ser Val His Ile Tyr Asp Lys Ala Gly Pro Gly Glu
   325 330 335
   ccc aaa tct tgt gac aaa act cac aca tgc cca ccg tgc cca gca cct 1056
   Pro Lys Ser Cys Asp Lys Thr His Thr Cys Pro Pro Cys Pro Ala Pro
   340 345 350
   gaa ctc ctg ggg gga ccg tca gtc ttc ctc ttc ccc cca aaa ccc aag 1104
   Glu Leu Leu Gly Gly Pro Ser Val Phe Leu Phe Pro Pro Lys Pro Lys
   355 360 365
   gac acc ctc atg atc tcc cgg acc cct gag gtc aca tgc gtg gtg gtg 1152
   Asp Thr Leu Met Ile Ser Arg Thr Pro Glu Val Thr Cys Val Val Val
   370 375 380
   gac gtg agc cac gaa gac cct gag gtc aag ttc aac tgg tac gtg gac      1200
   Asp Val Ser His Glu Asp Pro Glu Val Lys Phe Asn Trp Tyr Val Asp
   385                 390                 395                 400
   ggc gtg gag gtg cat aat gcc aag aca aag ccg cgg gag gag cag tac      1248
   Gly Val Glu Val His Asn Ala Lys Thr Lys Pro Arg Glu Glu Gln Tyr
                   405                 410                 415
   aac agc acg tac cgt gtg gtc agc gtc ctc acc gtc ctg cac cag gac      1296
   Asn Ser Thr Tyr Arg Val Val Ser Val Leu Thr Val Leu His Gln Asp
               420                 425                 430
   tgg ctg aat ggc aag gag tac aag tgc aag gtc tcc aac aaa gcc ctc      1344
   Trp Leu Asn Gly Lys Glu Tyr Lys Cys Lys Val Ser Asn Lys Ala Leu
           435                 440                 445
   cca gcc ccc atc gag aaa acc atc tcc aaa gcc aaa ggg cag ccc cga      1392
   Pro Ala Pro Ile Glu Lys Thr Ile Ser Lys Ala Lys Gly Gln Pro Arg
       450                 455                 460
   gaa cca cag gtg tac acc ctg ccc cca tcc cgg gat gag ctg acc aag      1440
   Glu Pro Gln Val Tyr Thr Leu Pro Pro Ser Arg Asp Glu Leu Thr Lys
   465                 470                 475                 480
   aac cag gtc agc ctg acc tgc ctg gtc aaa ggc ttc tat ccc agc gac      1488
   Asn Gln Val Ser Leu Thr Cys Leu Val Lys Gly Phe Tyr Pro Ser Asp
                   485                 490                 495
   atc gcc gtg gag tgg gag agc aat ggg cag ccg gag aac aac tac aag      1536
   Ile Ala Val Glu Trp Glu Ser Asn Gly Gln Pro Glu Asn Asn Tyr Lys
               500                 505                 510
   acc acg cct ccc gtg ctg gac tcc gac ggc tcc ttc ttc ctc tac agc      1584
   Thr Thr Pro Pro Val Leu Asp Ser Asp Gly Ser Phe Phe Leu Tyr Ser
           515                 520                 525
   aag ctc acc gtg gac aag agc agg tgg cag cag ggg aac gtc ttc tca      1632
   Lys Leu Thr Val Asp Lys Ser Arg Trp Gln Gln Gly Asn Val Phe Ser
       530                 535                 540
   tgc tcc gtg atg cat gag gct ctg cac aac cac tac acg cag aag agc      1680
   Cys Ser Val Met His Glu Ala Leu His Asn His Tyr Thr Gln Lys Ser
   545                 550                 555                 560
   ctc tcc ctg tct ccg ggt aaa tga                                      1704
   Leu Ser Leu Ser Pro Gly Lys
                   565
<210> 2
   <211> 567
   <212> PRT
   <213> Homo sapiens
<400> 2
   Met Val Ser Tyr Trp Asp Thr Gly Val Leu Leu Cys Ala Leu Leu Ser
    1                5                 10                  15
   Cys Leu Leu Leu Thr Gly Ser Ser Ser Gly Ser Lys Leu Lys Asp Pro
               20                  25                  30
   Glu Leu Ser Leu Lys Gly Thr Gln His Ile Met Gln Ala Gly Gln Thr
           35                  40                  45
   Leu His Leu Gln Cys Arg Gly Glu Ala Ala His Lys Trp Ser Leu Pro
       50                  55                  60
   Glu Met Val Ser Lys Glu Ser Glu Arg Leu Ser Ile Thr Lys Ser Ala
   65                  70                  75                  80
   Cys Gly Arg Asn Gly Lys Gln Phe Cys Ser Thr Leu Thr Leu Asn Thr
                   85                  90                  95
   Ala Gln Ala Asn His Thr Gly Phe Tyr Ser Cys Lys Tyr Leu Ala Val
               100                 105                 110
   Pro Thr Ser Lys Lys Lys Glu Thr Glu Ser Ala Ile Tyr Ile Phe Ile
           115                 120                 125
   Ser Asp Thr Gly Arg Pro Phe Val Glu Met Tyr Ser Glu Ile Pro Glu
       130                 135                 140
   Ile Ile His Met Thr Glu Gly Arg Glu Leu Val Ile Pro Cys Arg Val
   145                 150                 155                 160
   Thr Ser Pro Asn Ile Thr Val Thr Leu Lys Lys Phe Pro Leu Asp Thr
                   165                 170                 175
   Leu Ile Pro Asp Gly Lys Arg Ile Ile Trp Asp Ser Arg Lys Gly Phe
               180                 185                 190
   Ile Ile Ser Asn Ala Thr Tyr Lys Glu Ile Gly Leu Leu Thr Cys Glu
           195                 200                 205
   Ala Thr Val Asn Gly His Leu Tyr Lys Thr Asn Tyr Leu Thr His Arg
       210                 215                 220
   Gln Thr Asn Thr Ile Ile Asp Val Gln Ile Ser Thr Pro Arg Pro Val
   225                 230                 235                 240
   Lys Leu Leu Arg Gly His Thr Leu Val Leu Asn Cys Thr Ala Thr Thr
                   245                 250                 255
   Pro Leu Asn Thr Arg Val Gln Met Thr Trp Ser Tyr Pro Asp Glu Lys
               260                 265                 270
   Asn Lys Arg Ala Ser Val Arg Arg Arg Ile Asp Gln Ser Asn Ser His
           275                 280                 285
   Ala Asn Ile Phe Tyr Ser Val Leu Thr Ile Asp Lys Met Gln Asn Lys
       290                 295                 300
   Asp Lys Gly Leu Tyr Thr Cys Arg Val Arg Ser Gly Pro Ser Phe Lys
   305                 310                 315                 320
   Ser Val Asn Thr Ser Val His Ile Tyr Asp Lys Ala Gly Pro Gly Glu
                   325                 330                 335
   Pro Lys Ser Cys Asp Lys Thr His Thr Cys Pro Pro Cys Pro Ala Pro
               340                 345                 350
   Glu Leu Leu Gly Gly Pro Ser Val Phe Leu Phe Pro Pro Lys Pro Lys
           355                 360                 365
   Asp Thr Leu Met Ile Ser Arg Thr Pro Glu Val Thr Cys Val Val Val
       370                 375                 380
   Asp Val Ser His Glu Asp Pro Glu Val Lys Phe Asn Trp Tyr Val Asp
   385                 390                 395                 400
   Gly Val Glu Val His Asn Ala Lys Thr Lys Pro Arg Glu Glu Gln Tyr
                   405                 410                 415
   Asn Ser Thr Tyr Arg Val Val Ser Val Leu Thr Val Leu His Gln Asp
               420                 425                 430
   Trp Leu Asn Gly Lys Glu Tyr Lys Cys Lys Val Ser Asn Lys Ala Leu
           435                 440                 445
   Pro Ala Pro Ile Glu Lys Thr Ile Ser Lys Ala Lys Gly Gln Pro Arg
       450                 455                 460
   Glu Pro Gln Val Tyr Thr Leu Pro Pro Ser Arg Asp Glu Leu Thr Lys
   465                 470                 475                 480
   Asn Gln Val Ser Leu Thr Cys Leu Val Lys Gly Phe Tyr Pro Ser Asp
                   485                 490                 495
   Ile Ala Val Glu Trp Glu Ser Asn Gly Gln Pro Glu Asn Asn Tyr Lys
               500                 505                 510
   Thr Thr Pro Pro Val Leu Asp Ser Asp Gly Ser Phe Phe Leu Tyr Ser
           515                 520                 525
   Lys Leu Thr Val Asp Lys Ser Arg Trp Gln Gln Gly Asn Val Phe Ser
       530                 535                 540
   Cys Ser Val Met His Glu Ala Leu His Asn His Tyr Thr Gln Lys Ser
   545 550 555 560
   Leu Ser Leu Ser Pro Gly Lys
   565
<210> 3
   <211> 1674
   <212> DNA
   <213> Homo sapiens
<220>
   <221> CDS
   <222> (1)...(1671)
<400> 3
   atg gtc agc tac tgg gac acc ggg gtc ctg ctg tgc gcg ctg ctc agc       48
   Met Val Ser Tyr Trp Asp Thr Gly Val Leu Leu Cys Ala Leu Leu Ser
    1               5                  10                  15
   tgt ctg ctt ctc aca gga tct agt tca ggt tca aaa tta aaa gat cct       96
   Cys Leu Leu Leu Thr Gly Ser Ser Ser Gly Ser Lys Leu Lys Asp Pro
               20                  25                  30
   gaa ctg agt tta aaa ggc acc cag cac atc atg caa gca ggc cag aca      144
   Glu Leu Ser Leu Lys Gly Thr Gln His Ile Met Gln Ala Gly Gln Thr
           35                  40                  45
   ctg cat ctc caa tgc agg ggg gaa gca gcc cat aaa tgg tct ttg cct      192
   Leu His Leu Gln Cys Arg Gly Glu Ala Ala His Lys Trp Ser Leu Pro
       50                  55                  60
   gaa atg gtg agt aag gaa agc gaa agg ctg agc ata act aaa tct gcc      240
   Glu Met Val Ser Lys Glu Ser Glu Arg Leu Ser Ile Thr Lys Ser Ala
   65                  70                  75                  80
   tgt gga aga aat ggc aaa caa ttc tgc agt act tta acc ttg aac aca      288
   Cys Gly Arg Asn Gly Lys Gln Phe Cys Ser Thr Leu Thr Leu Asn Thr
                   85                  90                  95
   gct caa gca aac cac act ggc ttc tac agc tgc aaa tat cta gct gta      336
   Ala Gln Ala Asn His Thr Gly Phe Tyr Ser Cys Lys Tyr Leu Ala Val
               100                 105                 110
   cct act tca aag aag aag gaa aca gaa tct gca atc tat ata ttt att      384
   Pro Thr Ser Lys Lys Lys Glu Thr Glu Ser Ala Ile Tyr Ile Phe Ile
           115                 120                 125
   agt gat aca ggt aga cct ttc gta gag atg tac agt gaa atc ccc gaa      432
   Ser Asp Thr Gly Arg Pro Phe Val Glu Met Tyr Ser Glu Ile Pro Glu
       130                 135                 140
   att ata cac atg act gaa gga agg gag ctc gtc att ccc tgc cgg gtt      480
   Ile Ile His Met Thr Glu Gly Arg Glu Leu Val Ile Pro Cys Arg Val
   145                 150                 155                 160
   acg tca cct aac atc act gtt act tta aaa aag ttt cca ctt gac act      528
   Thr Ser Pro Asn Ile Thr Val Thr Leu Lys Lys Phe Pro Leu Asp Thr
                   165                 170                 175
   ttg atc cct gat gga aaa cgc ata atc tgg gac agt aga aag ggc ttc      576
   Leu Ile Pro Asp Gly Lys Arg Ile Ile Trp Asp Ser Arg Lys Gly Phe
               180                 185                 190
   atc ata tca aat gca acg tac aaa gaa ata ggg ctt ctg acc tgt gaa      624
   Ile Ile Ser Asn Ala Thr Tyr Lys Glu Ile Gly Leu Leu Thr Cys Glu
           195                 200                 205
   gca aca gtc aat ggg cat ttg tat aag aca aac tat ctc aca cat cga      672
   Ala Thr Val Asn Gly His Leu Tyr Lys Thr Asn Tyr Leu Thr His Arg
       210                 215                 220
   caa acc aat aca atc ata gat gtc caa ata agc aca cca cgc cca gtc      720
   Gln Thr Asn Thr Ile Ile Asp Val Gln Ile Ser Thr Pro Arg Pro Val
   225                 230                 235                 240
   aaa tta ctt aga ggc cat act ctt gtc ctc aat tgt act gct acc act      768
   Lys Leu Leu Arg Gly His Thr Leu Val Leu Asn Cys Thr Ala Thr Thr
                   245                 250                 255
   ccc ttg aac acg aga gtt caa atg acc tgg agt tac cct gat gaa att      816
   Pro Leu Asn Thr Arg Val Gln Met Thr Trp Ser Tyr Pro Asp Glu Ile
               260                 265                 270
   gac caa agc aat tcc cat gcc aac ata ttc tac agt gtt ctt act att      864
   Asp Gln Ser Asn Ser His Ala Asn Ile Phe Tyr Ser Val Leu Thr Ile
           275                 280                 285
   gac aaa atg cag aac aaa gac aaa gga ctt tat act tgt cgt gta agg      912
   Asp Lys Met Gln Asn Lys Asp Lys Gly Leu Tyr Thr Cys Arg Val Arg
       290                 295                 300
   agt gga cca tca ttc aaa tct gtt aac acc tca gtg cat ata tat gat      960
   Ser Gly Pro Ser Phe Lys Ser Val Asn Thr Ser Val His Ile Tyr Asp
   305                 310                 315                 320
   aaa gca ggc ccg ggc gag ccc aaa tct tgt gac aaa act cac aca tgc     1008
   Lys Ala Gly Pro Gly Glu Pro Lys Ser Cys Asp Lys Thr His Thr Cys
                   325                 330                 335
   cca ccg tgc cca gca cct gaa ctc ctg ggg gga ccg tca gtc ttc ctc     1056
   Pro Pro Cys Pro Ala Pro Glu Leu Leu Gly Gly Pro Ser Val Phe Leu
               340                 345                 350
   ttc ccc cca aaa ccc aag gac acc ctc atg atc tcc cgg acc cct gag     1104
   Phe Pro Pro Lys Pro Lys Asp Thr Leu Met Ile Ser Arg Thr Pro Glu
           355                 360                 365
   gtc aca tgc gtg gtg gtg gac gtg agc cac gaa gac cct gag gtc aag     1152
   Val Thr Cys Val Val Val Asp Val Ser His Glu Asp Pro Glu Val Lys
       370                 375                 380
   ttc aac tgg tac gtg gac ggc gtg gag gtg cat aat gcc aag aca aag     1200
   Phe Asn Trp Tyr Val Asp Gly Val Glu Val His Asn Ala Lys Thr Lys
   385                 390                 395                 400
   ccg cgg gag gag cag tac aac agc acg tac cgt gtg gtc agc gtc ctc     1248
   Pro Arg Glu Glu Gln Tyr Asn Ser Thr Tyr Arg Val Val Ser Val Leu
                   405                 410                 415
   acc gtc ctg cac cag gac tgg ctg aat ggc aag gag tac aag tgc aag     1296
   Thr Val Leu His Gln Asp Trp Leu Asn Gly Lys Glu Tyr Lys Cys Lys
               420                 425                 430
   gtc tcc aac aaa gcc ctc cca gcc ccc atc gag aaa acc atc tcc aaa     1344
   Val Ser Asn Lys Ala Leu Pro Ala Pro Ile Glu Lys Thr Ile Ser Lys
           435                 440                 445
   gcc aaa ggg cag ccc cga gaa cca cag gtg tac acc ctg ccc cca tcc        1392
   Ala Lys Gly Gln Pro Arg Glu Pro Gln Val Tyr Thr Leu Pro Pro Ser
       450                 455                     460
   cgg gat gag ctg acc aag aac cag gtc agc ctg acc tgc ctg gtc aaa        1440
   Arg Asp Glu Leu Thr Lys Asn Gln Val Ser Leu Thr Cys Leu Val Lys
   465                 470                     475                 480
   ggc ttc tat ccc agc gac atc gcc gtg gag tgg gag agc aat ggg cag        1488
   Gly Phe Tyr Pro Ser Asp Ile Ala Val Glu Trp Glu Ser Asn Gly Gln
                   485                     490                 495
   ccg gag aac aac tac aag acc acg cct ccc gtg ctg gac tcc gac ggc        1536
   Pro Glu Asn Asn Tyr Lys Thr Thr Pro Pro Val Leu Asp Ser Asp Gly
               500                     505                 510
   tcc ttc ttc ctc tac agc aag ctc acc gtg gac aag agc agg tgg cag        1584
   Ser Phe Phe Leu Tyr Ser Lys Leu Thr Val Asp Lys Ser Arg Trp Gln
           515                     520                 525
   cag ggg aac gtc ttc tca tgc tcc gtg atg cat gag gct ctg cac aac        1632
   Gln Gly Asn Val Phe Ser Cys Ser Val Met His Glu Ala Leu His Asn
       530                 535                     540
   cac tac acg cag aag agc ctc tcc ctg tct ccg ggt aaa tga                1674
   His Tyr Thr Gln Lys Ser Leu Ser Leu Ser Pro Gly Lys
   545                 550                     555
<210> 4
   <211> 557
   <212> PRT
   <213> Homo sapiens
<400> 4
   Met Val Ser Tyr Trp Asp Thr Gly Val Leu Leu Cys Ala Leu Leu Ser
    1               5                  10                  15
   Cys Leu Leu Leu Thr Gly Ser Ser Ser Gly Ser Lys Leu Lys Asp Pro
               20                  25                  30
   Glu Leu Ser Leu Lys Gly Thr Gln His Ile Met Gln Ala Gly Gln Thr
           35                  40                  45
   Leu His Leu Gln Cys Arg Gly Glu Ala Ala His Lys Trp Ser Leu Pro
       50                  55                  60
   Glu Met Val Ser Lys Glu Ser Glu Arg Leu Ser Ile Thr Lys Ser Ala
   65                  70                  75                  80
   Cys Gly Arg Asn Gly Lys Gln Phe Cys Ser Thr Leu Thr Leu Asn Thr
                   85                  90                  95
   Ala Gln Ala Asn His Thr Gly Phe Tyr Ser Cys Lys Tyr Leu Ala Val
               100                 105                 110
   Pro Thr Ser Lys Lys Lys Glu Thr Glu Ser Ala Ile Tyr Ile Phe Ile
           115                 120                 125
   Ser Asp Thr Gly Arg Pro Phe Val Glu Met Tyr Ser Glu Ile Pro Glu
       130                 135                 140
   Ile Ile His Met Thr Glu Gly Arg Glu Leu Val Ile Pro Cys Arg Val
   145                 150                 155                 160
   Thr Ser Pro Asn Ile Thr Val Thr Leu Lys Lys Phe Pro Leu Asp Thr
                   165                 170                 175
   Leu Ile Pro Asp Gly Lys Arg Ile Ile Trp Asp Ser Arg Lys Gly Phe
               180                 185                 190
   Ile Ile Ser Asn Ala Thr Tyr Lys Glu Ile Gly Leu Leu Thr Cys Glu
           195                 200                 205
   Ala Thr Val Asn Gly His Leu Tyr Lys Thr Asn Tyr Leu Thr His Arg
       210                     215                     220
   Gln Thr Asn Thr Ile Ile Asp Val Gln Ile Ser Thr Pro Arg Pro Val
   225                     230                     235                 240
   Lys Leu Leu Arg Gly His Thr Leu Val Leu Asn Cys Thr Ala Thr Thr
                   245                     250                     255
   Pro Leu Asn Thr Arg Val Gln Met Thr Trp Ser Tyr Pro Asp Glu Ile
               260                     265                     270
   Asp Gln Ser Asn Ser His Ala Asn Ile Phe Tyr Ser Val Leu Thr Ile
           275                     280                     285
   Asp Lys Met Gln Asn Lys Asp Lys Gly Leu Tyr Thr Cys Arg Val Arg
       290                     295                     300
   Ser Gly Pro Ser Phe Lys Ser Val Asn Thr Ser Val His Ile Tyr Asp
   305                     310                     315                 320
   Lys Ala Gly Pro Gly Glu Pro Lys Ser Cys Asp Lys Thr His Thr Cys
                   325                     330                     335
   Pro Pro Cys Pro Ala Pro Glu Leu Leu Gly Gly Pro Ser Val Phe Leu
               340                     345                     350
   Phe Pro Pro Lys Pro Lys Asp Thr Leu Met Ile Ser Arg Thr Pro Glu
           355                     360                     365
   Val Thr Cys Val Val Val Asp Val Ser His Glu Asp Pro Glu Val Lys
       370                     375                     380
   Phe Asn Trp Tyr Val Asp Gly Val Glu Val His Asn Ala Lys Thr Lys
   385                     390                     395                 400
   Pro Arg Glu Glu Gln Tyr Asn Ser Thr Tyr Arg Val Val Ser Val Leu
                   405                     410                     415
   Thr Val Leu His Gln Asp Trp Leu Asn Gly Lys Glu Tyr Lys Cys Lys
               420                     425                     430
   Val Ser Asn Lys Ala Leu Pro Ala Pro Ile Glu Lys Thr Ile Ser Lys
           435                     440                     445
   Ala Lys Gly Gln Pro Arg Glu Pro Gln Val Tyr Thr Leu Pro Pro Ser
       450                     455                     460
   Arg Asp Glu Leu Thr Lys Asn Gln Val Ser Leu Thr Cys Leu Val Lys
   465                     470                     475                 480
   Gly Phe Tyr Pro Ser Asp Ile Ala Val Glu Trp Glu Ser Asn Gly Gln
                   485                     490                     495
   Pro Glu Asn Asn Tyr Lys Thr Thr Pro Pro Val Leu Asp Ser Asp Gly
               500                     505                     510
   Ser Phe Phe Leu Tyr Ser Lys Leu Thr Val Asp Lys Ser Arg Trp Gln
           515                     520                     525
   Gln Gly Asn Val Phe Ser Cys Ser Val Met His Glu Ala Leu His Asn
       530                     535                     540
   His Tyr Thr Gln Lys Ser Leu Ser Leu Ser Pro Gly Lys
   545                     550                     555
<210> 5
   <211> 1359
   <212> DNA
   <213> Homo sapiens
<220>
   <221> CDS
   <222> (1)...(1356)
<400> 5
   atg gtc agc tac tgg gac acc ggg gtc ctg ctg tgc gcg ctg ctc agc        48
   Met Val Ser Tyr Trp Asp Thr Gly Val Leu Leu Cys Ala Leu Leu Ser
   1               5                   10                  15
   tgt ctg ctt ctc aca gga tct agt tcc gga ggt aga cct ttc gta gag        96
   Cys Leu Leu Leu Thr Gly Ser Ser Ser Gly Gly Arg Pro Phe Val Glu
               20                  25                  30
   atg tac agt gaa atc ccc gaa att ata cac atg act gaa gga agg gag       144
   Met Tyr Ser Glu Ile Pro Glu Ile Ile His Met Thr Glu Gly Arg Glu
           35                  40                  45
   ctc gtc att ccc tgc cgg gtt acg tca cct aac atc act gtt act tta      192
   Leu Val Ile Pro Cys Arg Val Thr Ser Pro Asn Ile Thr Val Thr Leu
       50                  55                  60
   aaa aag ttt cca ctt gac act ttg atc cct gat gga aaa cgc ata atc      240
   Lys Lys Phe Pro Leu Asp Thr Leu Ile Pro Asp Gly Lys Arg Ile Ile
   65                  70                  75                  80
   tgg gac agt aga aag ggc ttc atc ata tca aat gca acg tac aaa gaa      288
   Trp Asp Ser Arg Lys Gly Phe Ile Ile Ser Asn Ala Thr Tyr Lys Glu
                   85                  90                  95
   ata ggg ctt ctg acc tgt gaa gca aca gtc aat ggg cat ttg tat aag      336
   Ile Gly Leu Leu Thr Cys Glu Ala Thr Val Asn Gly His Leu Tyr Lys
               100                 105                 110
   aca aac tat ctc aca cat cga caa acc aat aca atc ata gat gtc caa      384
   Thr Asn Tyr Leu Thr His Arg Gln Thr Asn Thr Ile Ile Asp Val Gln
           115                 120                 125
   ata agc aca cca cgc cca gtc aaa tta ctt aga ggc cat act ctt gtc      432
   Ile Ser Thr Pro Arg Pro Val Lys Leu Leu Arg Gly His Thr Leu Val
       130                 135                 140
   ctc aat tgt act gct acc act ccc ttg aac acg aga gtt caa atg acc      480
   Leu Asn Cys Thr Ala Thr Thr Pro Leu Asn Thr Arg Val Gln Met Thr
   145                 150                 155                 160
   tgg agt tac cct gat gaa att gac caa agc aat tcc cat gcc aac ata      528
   Trp Ser Tyr Pro Asp Glu Ile Asp Gln Ser Asn Ser His Ala Asn Ile
                   165                 170                 175
   ttc tac agt gtt ctt act att gac aaa atg cag aac aaa gac aaa gga      576
   Phe Tyr Ser Val Leu Thr Ile Asp Lys Met Gln Asn Lys Asp Lys Gly
               180                 185                 190
   ctt tat act tgt cgt gta agg agt gga cca tca ttc aaa tct gtt aac      624
   Leu Tyr Thr Cys Arg Val Arg Ser Gly Pro Ser Phe Lys Ser Val Asn
           195                 200                 205
   acc tca gtg cat ata tat gat aaa gca ggc ccg ggc gag ccc aaa tct      672
   Thr Ser Val His Ile Tyr Asp Lys Ala Gly Pro Gly Glu Pro Lys Ser
       210                 215                 220
   tgt gac aaa act cac aca tgc cca ccg tgc cca gca cct gaa ctc ctg      720
   Cys Asp Lys Thr His Thr Cys Pro Pro Cys Pro Ala Pro Glu Leu Leu
   225                 230                 235                 240
   ggg gga ccg tca gtc ttc ctc ttc ccc cca aaa ccc aag gac acc ctc      768
   Gly Gly Pro Ser Val Phe Leu Phe Pro Pro Lys Pro Lys Asp Thr Leu
                   245                 250                 255
   atg atc tcc cgg acc cct gag gtc aca tgc gtg gtg gtg gac gtg agc      816
   Met Ile Ser Arg Thr Pro Glu Val Thr Cys Val Val Val Asp Val Ser
               260                 265                 270
   cac gaa gac cct gag gtc aag ttc aac tgg tac gtg gac ggc gtg gag      864
   His Glu Asp Pro Glu Val Lys Phe Asn Trp Tyr Val Asp Gly Val Glu
           275                 280                 285
   gtg cat aat gcc aag aca aag ccg cgg gag gag cag tac aac agc acg       912
   Val His Asn Ala Lys Thr Lys Pro Arg Glu Glu Gln Tyr Asn Ser Thr
       290                 295                 300
   tac cgt gtg gtc agc gtc ctc acc gtc ctg cac cag gac tgg ctg aat       960
   Tyr Arg Val Val Ser Val Leu Thr Val Leu His Gln Asp Trp Leu Asn
   305                 310                 315                 320
   ggc aag gag tac aag tgc aag gtc tcc aac aaa gcc ctc cca gcc ccc      1008
   Gly Lys Glu Tyr Lys Cys Lys Val Ser Asn Lys Ala Leu Pro Ala Pro
                   325                 330                 335
   atc gag aaa acc atc tcc aaa gcc aaa ggg cag ccc cga gaa cca cag      1056
   Ile Glu Lys Thr Ile Ser Lys Ala Lys Gly Gln Pro Arg Glu Pro Gln
               340                 345                 350
   gtg tac acc ctg ccc cca tcc cgg gat gag ctg acc aag aac cag gtc      1104
   Val Tyr Thr Leu Pro Pro Ser Arg Asp Glu Leu Thr Lys Asn Gln Val
           355                 360                 365
   agc ctg acc tgc ctg gtc aaa ggc ttc tat ccc agc gac atc gcc gtg      1152
   Ser Leu Thr Cys Leu Val Lys Gly Phe Tyr Pro Ser Asp Ile Ala Val
       370                 375                 380
   gag tgg gag agc aat ggg cag ccg gag aac aac tac aag acc acg cct      1200
   Glu Trp Glu Ser Asn Gly Gln Pro Glu Asn Asn Tyr Lys Thr Thr Pro
   385                 390                 395                 400
   ccc gtg ctg gac tcc gac ggc tcc ttc ttc ctc tac agc aag ctc acc      1248
   Pro Val Leu Asp Ser Asp Gly Ser Phe Phe Leu Tyr Ser Lys Leu Thr
                   405                 410                 415
   gtg gac aag agc agg tgg cag cag ggg aac gtc ttc tca tgc tcc gtg      1296
   Val Asp Lys Ser Arg Trp Gln Gln Gly Asn Val Phe Ser Cys Ser Val
               420                 425                 430
   atg cat gag gct ctg cac aac cac tac acg cag aag agc ctc tcc ctg      1344
   Met His Glu Ala Leu His Asn His Tyr Thr Gln Lys Ser Leu Ser Leu
           435                 440                 445
   tct ccg ggt aaa tga                                                  1359
   Ser Pro Gly Lys
       450
<210> 6
   <211> 452
   <212> PRT
   <213> Homo sapiens
<400> 6
   Met Val Ser Tyr Trp Asp Thr Gly Val Leu Leu Cys Ala Leu Leu Ser
    1               5                  10                  15
   Cys Leu Leu Leu Thr Gly Ser Ser Ser Gly Gly Arg Pro Phe Val Glu
               20                  25                  30
   Met Tyr Ser Glu Ile Pro Glu Ile Ile His Met Thr Glu Gly Arg Glu
           35                  40                  45
   Leu Val Ile Pro Cys Arg Val Thr Ser Pro Asn Ile Thr Val Thr Leu
       50                  55                  60
   Lys Lys Phe Pro Leu Asp Thr Leu Ile Pro Asp Gly Lys Arg Ile Ile
   65                  70                  75                  80
   Trp Asp Ser Arg Lys Gly Phe Ile Ile Ser Asn Ala Thr Tyr Lys Glu
                       85                  90                  95
   Ile Gly Leu Leu Thr Cys Glu Ala Thr Val Asn Gly His Leu Tyr Lys
               100                 105                 110
   Thr Asn Tyr Leu Thr His Arg Gln Thr Asn Thr Ile Ile Asp Val Gln
           115                 120                 125
   Ile Ser Thr Pro Arg Pro Val Lys Leu Leu Arg Gly His Thr Leu Val
       130                 135                 140
   Leu Asn Cys Thr Ala Thr Thr Pro Leu Asn Thr Arg Val Gln Met Thr
   145                 150                 155                 160
   Trp Ser Tyr Pro Asp Glu Ile Asp Gln Ser Asn Ser His Ala Asn Ile
                   165                 170                 175
   Phe Tyr Ser Val Leu Thr Ile Asp Lys Met Gln Asn Lys Asp Lys Gly
               180                 185                 190
   Leu Tyr Thr Cys Arg Val Arg Ser Gly Pro Ser Phe Lys Ser Val Asn
           195                 200                 205
   Thr Ser Val His Ile Tyr Asp Lys Ala Gly Pro Gly Glu Pro Lys Ser
       210                 215                 220
   Cys Asp Lys Thr His Thr Cys Pro Pro Cys Pro Ala Pro Glu Leu Leu
   225                 230                 235                 240
   Gly Gly Pro Ser Val Phe Leu Phe Pro Pro Lys Pro Lys Asp Thr Leu
                   245                 250                 255
   Met Ile Ser Arg Thr Pro Glu Val Thr Cys Val Val Val Asp Val Ser
               260                 265                 270
   His Glu Asp Pro Glu Val Lys Phe Asn Trp Tyr Val Asp Gly Val Glu
           275                 280                 285
   Val His Asn Ala Lys Thr Lys Pro Arg Glu Glu Gln Tyr Asn Ser Thr
       290                 295                 300
   Tyr Arg Val Val Ser Val Leu Thr Val Leu His Gln Asp Trp Leu Asn
   305                 310                 315                 320
   Gly Lys Glu Tyr Lys Cys Lys Val Ser Asn Lys Ala Leu Pro Ala Pro
                   325                 330                 335
   Ile Glu Lys Thr Ile Ser Lys Ala Lys Gly Gln Pro Arg Glu Pro Gln
               340                 345                 350
   Val Tyr Thr Leu Pro Pro Ser Arg Asp Glu Leu Thr Lys Asn Gln Val
           355                 360                 365
   Ser Leu Thr Cys Leu Val Lys Gly Phe Tyr Pro Ser Asp Ile Ala Val
       370                 375                 380
   Glu Trp Glu Ser Asn Gly Gln Pro Glu Asn Asn Tyr Lys Thr Thr Pro
   385                 390                 395                 400
   Pro Val Leu Asp Ser Asp Gly Ser Phe Phe Leu Tyr Ser Lys Leu Thr
                   405                 410                 415
   Val Asp Lys Ser Arg Trp Gln Gln Gly Asn Val Phe Ser Cys Ser Val
               420                 425                 430
   Met His Glu Ala Leu His Asn His Tyr Thr Gln Lys Ser Leu Ser Leu
           435                 440                 445
   Ser Pro Gly Lys
       450
<210> 7
   <211> 1389
   <212> DNA
   <213> Homo sapiens
<220>
   <221> CDS
   <222> (1)...(1386)
<400> 7
   atg gtc agc tac tgg gac acc ggg gtc ctg ctg tgc gcg ctg ctc agc        48
   Met Val Ser Tyr Trp Asp Thr Gly Val Leu Leu Cys Ala Leu Leu Ser
    1               5                  10                  15
   tgt ctg ctt ctc aca gga tct agt tcc gga ggt aga cct ttc gta gag        96
   Cys Leu Leu Leu Thr Gly Ser Ser Ser Gly Gly Arg Pro Phe Val Glu
                   20                       25                        30
   atg tac agt gaa atc ccc gaa att ata cac atg act gaa gga agg gag      144
   Met Tyr Ser Glu Ile Pro Glu Ile Ile His Met Thr Glu Gly Arg Glu
               35                  40                  45
   ctc gtc att ccc tgc cgg gtt acg tca cct aac atc act gtt act tta      192
   Leu Val Ile Pro Cys Arg Val Thr Ser Pro Asn Ile Thr Val Thr Leu
           50                  55                  60
   aaa aag ttt cca ctt gac act ttg atc cct gat gga aaa cgc ata atc      240
   Lys Lys Phe Pro Leu Asp Thr Leu Ile Pro Asp Gly Lys Arg Ile Ile
    65                 70                  75                  80
   tgg gac agt aga aag ggc ttc atc ata tca aat gca acg tac aaa gaa      288
   Trp Asp Ser Arg Lys Gly Phe Ile Ile Ser Asn Ala Thr Tyr Lys Glu
                   85                  90                  95
   ata ggg ctt ctg acc tgt gaa gca aca gtc aat ggg cat ttg tat aag      336
   Ile Gly Leu Leu Thr Cys Glu Ala Thr Val Asn Gly His Leu Tyr Lys
               100                 105                 110
   aca aac tat ctc aca cat cga caa acc aat aca atc ata gat gtc caa      384
   Thr Asn Tyr Leu Thr His Arg Gln Thr Asn Thr Ile Ile Asp Val Gln
           115                 120                 125
   ata agc aca cca cgc cca gtc aaa tta ctt aga ggc cat act ctt gtc      432
   Ile Ser Thr Pro Arg Pro Val Lys Leu Leu Arg Gly His Thr Leu Val
       130                 135                 140
   ctc aat tgt act gct acc act ccc ttg aac acg aga gtt caa atg acc      480
   Leu Asn Cys Thr Ala Thr Thr Pro Leu Asn Thr Arg Val Gln Met Thr
   145                 150                 155                 160
   tgg agt tac cct gat gaa aaa aat aag aga gct tcc gta agg cga cga      528
   Trp Ser Tyr Pro Asp Glu Lys Asn Lys Arg Ala Ser Val Arg Arg Arg
                   165                 170                 175
   att gac caa agc aat tcc cat gcc aac ata ttc tac agt gtt ctt act      576
   Ile Asp Gln Ser Asn Ser His Ala Asn Ile Phe Tyr Ser Val Leu Thr
               180                 185                 190
   att gac aaa atg cag aac aaa gac aaa gga ctt tat act tgt cgt gta      624
   Ile Asp Lys Met Gln Asn Lys Asp Lys Gly Leu Tyr Thr Cys Arg Val
           195                 200                 205
   agg agt gga cca tca ttc aaa tct gtt aac acc tca gtg cat ata tat      672
   Arg Ser Gly Pro Ser Phe Lys Ser Val Asn Thr Ser Val His Ile Tyr
       210                 215                 220
   gat aaa gca ggc ccg ggc gag ccc aaa tct tgt gac aaa act cac aca      720
   Asp Lys Ala Gly Pro Gly Glu Pro Lys Ser Cys Asp Lys Thr His Thr
   225                 230                 235                 240
   tgc cca ccg tgc cca gca cct gaa ctc ctg ggg gga ccg tca gtc ttc      768
   Cys Pro Pro Cys Pro Ala Pro Glu Leu Leu Gly Gly Pro Ser Val Phe
                   245                 250                 255
   ctc ttc ccc cca aaa ccc aag gac acc ctc atg atc tcc cgg acc cct      816
   Leu Phe Pro Pro Lys Pro Lys Asp Thr Leu Met Ile Ser Arg Thr Pro
               260                 265                 270
   gag gtc aca tgc gtg gtg gtg gac gtg agc cac gaa gac cct gag gtc      864
   Glu Val Thr Cys Val Val Val Asp Val Ser His Glu Asp Pro Glu Val
           275                 280                 285
   aag ttc aac tgg tac gtg gac ggc gtg gag gtg cat aat gcc aag aca      912
   Lys Phe Asn Trp Tyr Val Asp Gly Val Glu Val His Asn Ala Lys Thr
       290                 295                 300
   aag ccg cgg gag gag cag tac aac agc acg tac cgt gtg gtc agc gtc      960
   Lys Pro Arg Glu Glu Gln Tyr Asn Ser Thr Tyr Arg Val Val Ser Val
   305                 310                 315                 320
   ctc acc gtc ctg cac cag gac tgg ctg aat ggc aag gag tac aag tgc     1008
   Leu Thr Val Leu His Gln Asp Trp Leu Asn Gly Lys Glu Tyr Lys Cys
                   325                 330                 335
   aag gtc tcc aac aaa gcc ctc cca gcc ccc atc gag aaa acc atc tcc     1056
   Lys Val Ser Asn Lys Ala Leu Pro Ala Pro Ile Glu Lys Thr Ile Ser
               340                 345                 350
   aaa gcc aaa ggg cag ccc cga gaa cca cag gtg tac acc ctg ccc cca     1104
   Lys Ala Lys Gly Gln Pro Arg Glu Pro Gln Val Tyr Thr Leu Pro Pro
           355                 360                 365
   tcc cgg gat gag ctg acc aag aac cag gtc agc ctg acc tgc ctg gtc     1152
   Ser Arg Asp Glu Leu Thr Lys Asn Gln Val Ser Leu Thr Cys Leu Val
       370                 375                 380
   aaa ggc ttc tat ccc agc gac atc gcc gtg gag tgg gag agc aat ggg     1200
   Lys Gly Phe Tyr Pro Ser Asp Ile Ala Val Glu Trp Glu Ser Asn Gly
   385                 390                 395                 400
   cag ccg gag aac aac tac aag acc acg cct ccc gtg ctg gac tcc gac     1248
   Gln Pro Glu Asn Asn Tyr Lys Thr Thr Pro Pro Val Leu Asp Ser Asp
                   405                 410                 415
   ggc tcc ttc ttc ctc tac agc aag ctc acc gtg gac aag agc agg tgg     1296
   Gly Ser Phe Phe Leu Tyr Ser Lys Leu Thr Val Asp Lys Ser Arg Trp
               420                 425                 430
   cag cag ggg aac gtc ttc tca tgc tcc gtg atg cat gag gct ctg cac     1344
   Gln Gln Gly Asn Val Phe Ser Cys Ser Val Met His Glu Ala Leu His
           435                 440                 445
   aac cac tac acg cag aag agc ctc tcc ctg tct ccg ggt aaa             1386
   Asn His Tyr Thr Gln Lys Ser Leu Ser Leu Ser Pro Gly Lys
       450                 455                 460
   tga                                                                 1389
<210> 8
   <211> 462
   <212> PRT
   <213> Homo sapiens
<400> 8
   Met Val Ser Tyr Trp Asp Thr Gly Val Leu Leu Cys Ala Leu Leu Ser
    1               5                  10                  15
   Cys Leu Leu Leu Thr Gly Ser Ser Ser Gly Gly Arg Pro Phe Val Glu
               20                  25                  30
   Met Tyr Ser Glu Ile Pro Glu Ile Ile His Met Thr Glu Gly Arg Glu
           35                  40                  45
   Leu Val Ile Pro Cys Arg Val Thr Ser Pro Asn Ile Thr Val Thr Leu
       50                  55                  60
   Lys Lys Phe Pro Leu Asp Thr Leu Ile Pro Asp Gly Lys Arg Ile Ile
   65                  70                  75                  80
   Trp Asp Ser Arg Lys Gly Phe Ile Ile Ser Asn Ala Thr Tyr Lys Glu
                   85                  90                  95
   Ile Gly Leu Leu Thr Cys Glu Ala Thr Val Asn Gly His Leu Tyr Lys
               100                 105                 110
   Thr Asn Tyr Leu Thr His Arg Gln Thr Asn Thr Ile Ile Asp Val Gln
           115                 120                 125
   Ile Ser Thr Pro Arg Pro Val Lys Leu Leu Arg Gly His Thr Leu Val
       130                 135                 140
   Leu Asn Cys Thr Ala Thr Thr Pro Leu Asn Thr Arg Val Gln Met Thr
   145                 150                 155                 160
   Trp Ser Tyr Pro Asp Glu Lys Asn Lys Arg Ala Ser Val Arg Arg Arg
                   165                 170                 175
   Ile Asp Gln Ser Asn Ser His Ala Asn Ile Phe Tyr Ser Val Leu Thr
               180                 185                 190
   Ile Asp Lys Met Gln Asn Lys Asp Lys Gly Leu Tyr Thr Cys Arg Val
           195                 200                 205
   Arg Ser Gly Pro Ser Phe Lys Ser Val Asn Thr Ser Val His Ile Tyr
       210                 215                 220
   Asp Lys Ala Gly Pro Gly Glu Pro Lys Ser Cys Asp Lys Thr His Thr
   225                 230                 235                 240
   Cys Pro Pro Cys Pro Ala Pro Glu Leu Leu Gly Gly Pro Ser Val Phe
                   245                 250                 255
   Leu Phe Pro Pro Lys Pro Lys Asp Thr Leu Met Ile Ser Arg Thr Pro
               260                 265                 270
   Glu Val Thr Cys Val Val Val Asp Val Ser His Glu Asp Pro Glu Val
           275                 280                 285
   Lys Phe Asn Trp Tyr Val Asp Gly Val Glu Val His Asn Ala Lys Thr
       290                 295                 300
   Lys Pro Arg Glu Glu Gln Tyr Asn Ser Thr Tyr Arg Val Val Ser Val
   305                 310                 315                 320
   Leu Thr Val Leu His Gln Asp Trp Leu Asn Gly Lys Glu Tyr Lys Cys
                   325                 330                 335
   Lys Val Ser Asn Lys Ala Leu Pro Ala Pro Ile Glu Lys Thr Ile Ser
               340                 345                 350
   Lys Ala Lys Gly Gln Pro Arg Glu Pro Gln Val Tyr Thr Leu Pro Pro
           355                 360                 365
   Ser Arg Asp Glu Leu Thr Lys Asn Gln Val Ser Leu Thr Cys Leu Val
       370                 375                 380
   Lys Gly Phe Tyr Pro Ser Asp Ile Ala Val Glu Trp Glu Ser Asn Gly
   385                 390                 395                 400
   Gln Pro Glu Asn Asn Tyr Lys Thr Thr Pro Pro Val Leu Asp Ser Asp
                   405                 410                 415
   Gly Ser Phe Phe Leu Tyr Ser Lys Leu Thr Val Asp Lys Ser Arg Trp
               420                 425                 430
   Gln Gln Gly Asn Val Phe Ser Cys Ser Val Met His Glu Ala Leu His
           435                 440                 445
   Asn His Tyr Thr Gln Lys Ser Leu Ser Leu Ser Pro Gly Lys
       450                 455                 460
<210> 9
   <211> 1704
   <212> DNA
   <213> Homo sapiens
<220>
   <221> CDS
   <222> (1)...(1701)
<400> 9
   atg gtc agc tac tgg gac acc ggg gtc ctg ctg tgc gcg ctg ctc agc 48
   Met Val Ser Tyr Trp Asp Thr Gly Val Leu Leu Cys Ala Leu Leu Ser
    1               5                  10                  15
   tgt ctg ctt ctc aca gga tct agt tca ggt tca aaa tta aaa gat cct        96
   Cys Leu Leu Leu Thr Gly Ser Ser Ser Gly Ser Lys Leu Lys Asp Pro
               20                  25                  30
   gaa ctg agt tta aaa ggc acc cag cac atc atg caa gca ggc cag aca       144
   Glu Leu Ser Leu Lys Gly Thr Gln His Ile Met Gln Ala Gly Gln Thr
           35                  40                  45
   ctg cat ctc caa tgc agg ggg gaa gca gcc cat aaa tgg tct ttg cct       192
   Leu His Leu Gln Cys Arg Gly Glu Ala Ala His Lys Trp Ser Leu Pro
       50                  55                  60
   gaa atg gtg agt aag gaa agc gaa agg ctg agc ata act aaa tct gcc       240
   Glu Met Val Ser Lys Glu Ser Glu Arg Leu Ser Ile Thr Lys Ser Ala
   65                  70                  75                  80
   tgt gga aga aat ggc aaa caa ttc tgc agt act tta acc ttg aac aca       288
   Cys Gly Arg Asn Gly Lys Gln Phe Cys Ser Thr Leu Thr Leu Asn Thr
                   85                  90                  95
   gct caa gca aac cac act ggc ttc tac agc tgc aaa tat cta gct gta       336
   Ala Gln Ala Asn His Thr Gly Phe Tyr Ser Cys Lys Tyr Leu Ala Val
               100                 105                 110
   cct act tca aag aag aag gaa aca gaa tct gca atc tat ata ttt att       384
   Pro Thr Ser Lys Lys Lys Glu Thr Glu Ser Ala Ile Tyr Ile Phe Ile
           115                 120                 125
   agt gat aca ggt aga cct ttc gta gag atg tac agt gaa atc ccc gaa       432
   Ser Asp Thr Gly Arg Pro Phe Val Glu Met Tyr Ser Glu Ile Pro Glu
       130                 135                 140
   att ata cac atg act gaa gga agg gag ctc gtc att ccc tgc cgg gtt       480
   Ile Ile His Met Thr Glu Gly Arg Glu Leu Val Ile Pro Cys Arg Val
   145                 150                 155                 160
   acg tca cct aac atc act gtt act tta aaa aag ttt cca ctt gac act       528
   Thr Ser Pro Asn Ile Thr Val Thr Leu Lys Lys Phe Pro Leu Asp Thr
                   165                 170                 175
   ttg atc cct gat gga aaa cgc ata atc tgg gac agt aga aag ggc ttc       576
   Leu Ile Pro Asp Gly Lys Arg Ile Ile Trp Asp Ser Arg Lys Gly Phe
               180                 185                 190
   atc ata tca aat gca acg tac aaa gaa ata ggg ctt ctg acc tgt gaa       624
   Ile Ile Ser Asn Ala Thr Tyr Lys Glu Ile Gly Leu Leu Thr Cys Glu
           195                 200                 205
   gca aca gtc aat ggg cat ttg tat aag aca aac tat ctc aca cat cga       672
   Ala Thr Val Asn Gly His Leu Tyr Lys Thr Asn Tyr Leu Thr His Arg
       210                 215                 220
   caa acc aat aca atc ata gat gtc caa ata agc aca cca cgc cca gtc       720
   Gln Thr Asn Thr Ile Ile Asp Val Gln Ile Ser Thr Pro Arg Pro Val
   225                 230                 235                 240
   aaa tta ctt aga ggc cat act ctt gtc ctc aat tgt act gct acc act       768
   Lys Leu Leu Arg Gly His Thr Leu Val Leu Asn Cys Thr Ala Thr Thr
                       245                 250                 255
   ccc ttg aac acg aga gtt caa atg acc tgg agt tac cct gat gaa aaa       816
   Pro Leu Asn Thr Arg Val Gln Met Thr Trp Ser Tyr Pro Asp Glu Lys
                   260                 265                 270
   aat aag aac gct tcc gta agg cga cga att gac caa agc aat tcc cat       864
   Asn Lys Asn Ala Ser Val Arg Arg Arg Ile Asp Gln Ser Asn Ser His
           275                 280                 285
   gcc aac ata ttc tac agt gtt ctt act att gac aaa atg cag aac aaa       912
   Ala Asn Ile Phe Tyr Ser Val Leu Thr Ile Asp Lys Met Gln Asn Lys
       290                 295                 300
   gac aaa gga ctt tat act tgt cgt gta agg agt gga cca tca ttc aaa       960
   Asp Lys Gly Leu Tyr Thr Cys Arg Val Arg Ser Gly Pro Ser Phe Lys
   305                 310                 315                 320
   tct gtt aac acc tca gtg cat ata tat gat aaa gca ggc ccg ggc gag      1008
   Ser Val Asn Thr Ser Val His Ile Tyr Asp Lys Ala Gly Pro Gly Glu
                   325                 330                 335
   ccc aaa tct tgt gac aaa act cac aca tgc cca ccg tgc cca gca cct      1056
   Pro Lys Ser Cys Asp Lys Thr His Thr Cys Pro Pro Cys Pro Ala Pro
               340                 345                 350
   gaa ctc ctg ggg gga ccg tca gtc ttc ctc ttc ccc cca aaa ccc aag      1104
   Glu Leu Leu Gly Gly Pro Ser Val Phe Leu Phe Pro Pro Lys Pro Lys
           355                 360                 365
   gac acc ctc atg atc tcc cgg acc cct gag gtc aca tgc gtg gtg gtg      1152
   Asp Thr Leu Met Ile Ser Arg Thr Pro Glu Val Thr Cys Val Val Val
       370                 375                 380
   gac gtg agc cac gaa gac cct gag gtc aag ttc aac tgg tac gtg gac      1200
   Asp Val Ser His Glu Asp Pro Glu Val Lys Phe Asn Trp Tyr Val Asp
   385                 390                 395                 400
   ggc gtg gag gtg cat aat gcc aag aca aag ccg cgg gag gag cag tac      1248
   Gly Val Glu Val His Asn Ala Lys Thr Lys Pro Arg Glu Glu Gln Tyr
                   405                 410                 415
   aac agc acg tac cgt gtg gtc agc gtc ctc acc gtc ctg cac cag gac      1296
   Asn Ser Thr Tyr Arg Val Val Ser Val Leu Thr Val Leu His Gln Asp
               420                 425                 430
   tgg ctg aat ggc aag gag tac aag tgc aag gtc tcc aac aaa gcc ctc      1344
   Trp Leu Asn Gly Lys Glu Tyr Lys Cys Lys Val Ser Asn Lys Ala Leu
           435                 440                 445
   cca gcc ccc atc gag aaa acc atc tcc aaa gcc aaa ggg cag ccc cga      1392
   Pro Ala Pro Ile Glu Lys Thr Ile Ser Lys Ala Lys Gly Gln Pro Arg
       450                 455                 460
   gaa cca cag gtg tac acc ctg ccc cca tcc cgg gat gag ctg acc aag      1440
   Glu Pro Gln Val Tyr Thr Leu Pro Pro Ser Arg Asp Glu Leu Thr Lys
   465                 470                 475                 480
   aac cag gtc agc ctg acc tgc ctg gtc aaa ggc ttc tat ccc agc gac      1488
   Asn Gln Val Ser Leu Thr Cys Leu Val Lys Gly Phe Tyr Pro Ser Asp
                   485                 490                 495
   atc gcc gtg gag tgg gag agc aat ggg cag ccg gag aac aac tac aag      1536
   Ile Ala Val Glu Trp Glu Ser Asn Gly Gln Pro Glu Asn Asn Tyr Lys
               500                 505                 510
   acc acg cct ccc gtg ctg gac tcc gac ggc tcc ttc ttc ctc tac agc      1584
   Thr Thr Pro Pro Val Leu Asp Ser Asp Gly Ser Phe Phe Leu Tyr Ser
           515                 520                 525
   aag ctc acc gtg gac aag agc agg tgg cag cag ggg aac gtc ttc tca      1632
   Lys Leu Thr Val Asp Lys Ser Arg Trp Gln Gln Gly Asn Val Phe Ser
       530                 535                 540
   tgc tcc gtg atg cat gag gct ctg cac aac cac tac acg cag aag agc      1680
   Cys Ser Val Met His Glu Ala Leu His Asn His Tyr Thr Gln Lys Ser
   545                 550                 555                 560
   ctc tcc ctg tct ccg ggt aaa tga                                      1704
   Leu Ser Leu Ser Pro Gly Lys
                   565
<210> 10
   <211> 567
   <212> PRT
   <213> Homo sapiens
<400> 10
   Met Val Ser Tyr Trp Asp Thr Gly Val Leu Leu Cys Ala Leu Leu Ser
    1               5                  10                  15
   Cys Leu Leu Leu Thr Gly Ser Ser Ser Gly Ser Lys Leu Lys Asp Pro
               20                  25                  30
   Glu Leu Ser Leu Lys Gly Thr Gln His Ile Met Gln Ala Gly Gln Thr
           35                  40                  45
   Leu His Leu Gln Cys Arg Gly Glu Ala Ala His Lys Trp Ser Leu Pro
       50                  55                  60
   Glu Met Val Ser Lys Glu Ser Glu Arg Leu Ser Ile Thr Lys Ser Ala
   65                  70                  75                  80
   Cys Gly Arg Asn Gly Lys Gln Phe Cys Ser Thr Leu Thr Leu Asn Thr
                   85                  90                  95
   Ala Gln Ala Asn His Thr Gly Phe Tyr Ser Cys Lys Tyr Leu Ala Val
               100                 105                 110
   Pro Thr Ser Lys Lys Lys Glu Thr Glu Ser Ala Ile Tyr Ile Phe Ile
           115                 120                 125
   Ser Asp Thr Gly Arg Pro Phe Val Glu Met Tyr Ser Glu Ile Pro Glu
       130                 135                 140
   Ile Ile His Met Thr Glu Gly Arg Glu Leu Val Ile Pro Cys Arg Val
   145                 150                 155                 160
   Thr Ser Pro Asn Ile Thr Val Thr Leu Lys Lys Phe Pro Leu Asp Thr
                   165                 170                 175
   Leu Ile Pro Asp Gly Lys Arg Ile Ile Trp Asp Ser Arg Lys Gly Phe
               180                 185                 190
   Ile Ile Ser Asn Ala Thr Tyr Lys Glu Ile Gly Leu Leu Thr Cys Glu
           195                 200                 205
   Ala Thr Val Asn Gly His Leu Tyr Lys Thr Asn Tyr Leu Thr His Arg
       210                 215                 220
   Gln Thr Asn Thr Ile Ile Asp Val Gln Ile Ser Thr Pro Arg Pro Val
   225                 230                 235                 240
   Lys Leu Leu Arg Gly His Thr Leu Val Leu Asn Cys Thr Ala Thr Thr
                   245                 250                 255
   Pro Leu Asn Thr Arg Val Gln Met Thr Trp Ser Tyr Pro Asp Glu Lys
               260                 265                 270
   Asn Lys Asn Ala Ser Val Arg Arg Arg Ile Asp Gln Ser Asn Ser His
           275                     280                     285
   Ala Asn Ile Phe Tyr Ser Val Leu Thr Ile Asp Lys Met Gln Asn Lys
       290                     295                     300
   Asp Lys Gly Leu Tyr Thr Cys Arg Val Arg Ser Gly Pro Ser Phe Lys
   305                     310                     315                     320
   Ser Val Asn Thr Ser Val His Ile Tyr Asp Lys Ala Gly Pro Gly Glu
                   325                     330                     335
   Pro Lys Ser Cys Asp Lys Thr His Thr Cys Pro Pro Cys Pro Ala Pro
               340                     345                     350
   Glu Leu Leu Gly Gly Pro Ser Val Phe Leu Phe Pro Pro Lys Pro Lys
           355                     360                     365
   Asp Thr Leu Met Ile Ser Arg Thr Pro Glu Val Thr Cys Val Val Val
       370                     375                     380
   Asp Val Ser His Glu Asp Pro Glu Val Lys Phe Asn Trp Tyr Val Asp
   385                     390                     395                     400
   Gly Val Glu Val His Asn Ala Lys Thr Lys Pro Arg Glu Glu Gln Tyr
                   405                     410                     415
   Asn Ser Thr Tyr Arg Val Val Ser Val Leu Thr Val Leu His Gln Asp
               420                     425                     430
   Trp Leu Asn Gly Lys Glu Tyr Lys Cys Lys Val Ser Asn Lys Ala Leu
           435                     440                     445
   Pro Ala Pro Ile Glu Lys Thr Ile Ser Lys Ala Lys Gly Gln Pro Arg
       450                     455                     460
   Glu Pro Gln Val Tyr Thr Leu Pro Pro Ser Arg Asp Glu Leu Thr Lys
   465                     470                     475                     480
   Asn Gln Val Ser Leu Thr Cys Leu Val Lys Gly Phe Tyr Pro Ser Asp
                   485                     490                     495
   Ile Ala Val Glu Trp Glu Ser Asn Gly Gln Pro Glu Asn Asn Tyr Lys
               500                     505                     510
   Thr Thr Pro Pro Val Leu Asp Ser Asp Gly Ser Phe Phe Leu Tyr Ser
           515                     520                     525
   Lys Leu Thr Val Asp Lys Ser Arg Trp Gln Gln Gly Asn Val Phe Ser
       530                     535                     540
   Cys Ser Val Met His Glu Ala Leu His Asn His Tyr Thr Gln Lys Ser
   545                     550                     555                     560
   Leu Ser Leu Ser Pro Gly Lys
                   565
<210> 11
   <211> 1453
   <212> DNA
   <213> Homo sapiens
<220>
   <221> CDS
   <222> (69)...(1442)
<400> 11
   aagcttgggc tgcaggtcga tcgactctag aggatcgatc cccgggcgag ctcgaattcg      60
   caaccacc atg gtc agc tac tgg gac acc ggg gtc ctg ctg tgc gcg ctg      110
            Met Val Ser Tyr Trp Asp Thr Gly Val Leu Leu Cys Ala Leu
             1               5                  10
   ctc agc tgt ctg ctt ctc aca gga tct agt tcc gga ggt aga cct ttc       158
   Leu Ser Cys Leu Leu Leu Thr Gly Ser Ser Ser Gly Gly Arg Pro Phe
    15                  20                  25                  30
   gta gag atg tac agt gaa atc ccc gaa att ata cac atg act gaa gga       206
   Val Glu Met Tyr Ser Glu Ile Pro Glu Ile Ile His Met Thr Glu Gly
                    35                  40                  45
   agg gag ctc gtc att ccc tgc cgg gtt acg tca cct aac atc act gtt       254
   Arg Glu Leu Val Ile Pro Cys Arg Val Thr Ser Pro Asn Ile Thr Val
                50                  55                  60
   act tta aaa aag ttt cca ctt gac act ttg atc cct gat gga aaa cgc     302
   Thr Leu Lys Lys Phe Pro Leu Asp Thr Leu Ile Pro Asp Gly Lys Arg
           65                  70                  75
   ata atc tgg gac agt aga aag ggc ttc atc ata tca aat gca acg tac     350
   Ile Ile Trp Asp Ser Arg Lys Gly Phe Ile Ile Ser Asn Ala Thr Tyr
       80                  85                  90
   aaa gaa ata ggg ctt ctg acc tgt gaa gca aca gtc aat ggg cat ttg     398
   Lys Glu Ile Gly Leu Leu Thr Cys Glu Ala Thr Val Asn Gly His Leu
   95                  100                 105                 110
   tat aag aca aac tat ctc aca cat cga caa acc aat aca atc ata gat     446
   Tyr Lys Thr Asn Tyr Leu Thr His Arg Gln Thr Asn Thr Ile Ile Asp
                   115                 120                 125
   gtg gtt ctg agt ccg tct cat gga att gaa cta tct gtt gga gaa aag     494
   Val Val Leu Ser Pro Ser His Gly Ile Glu Leu Ser Val Gly Glu Lys
               130                 135                 140
   ctt gtc tta aat tgt aca gca aga act gaa cta aat gtg ggg att gac     542
   Leu Val Leu Asn Cys Thr Ala Arg Thr Glu Leu Asn Val Gly Ile Asp
           145                 150                 155
   ttc aac tgg gaa tac cct tct tcg aag cat cag cat aag aaa ctt gta     590
   Phe Asn Trp Glu Tyr Pro Ser Ser Lys His Gln His Lys Lys Leu Val
       160                 165                 170
   aac cga gac cta aaa acc cag tct ggg agt gag atg aag aaa ttt ttg     638
   Asn Arg Asp Leu Lys Thr Gln Ser Gly Ser Glu Met Lys Lys Phe Leu
   175                 180                 185                 190
   agc acc tta act ata gat ggt gta acc cgg agt gac caa gga ttg tac     686
   Ser Thr Leu Thr Ile Asp Gly Val Thr Arg Ser Asp Gln Gly Leu Tyr
                   195                 200                 205
   acc tgt gca gca tcc agt ggg ctg atg acc aag aag aac agc aca ttt     734
   Thr Cys Ala Ala Ser Ser Gly Leu Met Thr Lys Lys Asn Ser Thr Phe
               210                 215                 220
   gtc agg gtc cat gaa aag ggc ccg ggc gac aaa act cac aca tgc cca     782
   Val Arg Val His Glu Lys Gly Pro Gly Asp Lys Thr His Thr Cys Pro
           225                 230                 235
   ccg tgc cca gca cct gaa ctc ctg ggg gga ccg tca gtc ttc ctc ttc     830
   Pro Cys Pro Ala Pro Glu Leu Leu Gly Gly Pro Ser Val Phe Leu Phe
       240                 245                 250
   ccc cca aaa ccc aag gac acc ctc atg atc tcc cgg acc cct gag gtc     878
   Pro Pro Lys Pro Lys Asp Thr Leu Met Ile Ser Arg Thr Pro Glu Val
   255                 260                 265                 270
   aca tgc gtg gtg gtg gac gtg agc cac gaa gac cct gag gtc aag ttc     926
   Thr Cys Val Val Val Asp Val Ser His Glu Asp Pro Glu Val Lys Phe
                   275                 280                 285
   aac tgg tac gtg gac ggc gtg gag gtg cat aat gcc aag aca aag ccg     974
   Asn Trp Tyr Val Asp Gly Val Glu Val His Asn Ala Lys Thr Lys Pro
               290                 295                 300
   cgg gag gag cag tac aac agc acg tac cgt gtg gtc agc gtc ctc acc        1022
   Arg Glu Glu Gln Tyr Asn Ser Thr Tyr Arg Val Val Ser Val Leu Thr
           305                 310                 315
   gtc ctg cac cag gac tgg ctg aat ggc aag gag tac aag tgc aag gtc        1070
   Val Leu His Gln Asp Trp Leu Asn Gly Lys Glu Tyr Lys Cys Lys Val
       320                 325                 330
   tcc aac aaa gcc ctc cca gcc ccc atc gag aaa acc atc tcc aaa gcc        1118
   Ser Asn Lys Ala Leu Pro Ala Pro Ile Glu Lys Thr Ile Ser Lys Ala
   335                 340                 345                 350
   aaa ggg cag ccc cga gaa cca cag gtg tac acc ctg ccc cca tcc cgg        1166
   Lys Gly Gln Pro Arg Glu Pro Gln Val Tyr Thr Leu Pro Pro Ser Arg
                   355                 360                 365
   gat gag ctg acc aag aac cag gtc agc ctg acc tgc ctg gtc aaa ggc        1214
   Asp Glu Leu Thr Lys Asn Gln Val Ser Leu Thr Cys Leu Val Lys Gly
               370                 375                 380
   ttc tat ccc agc gac atc gcc gtg gag tgg gag agc aat ggg cag ccg        1262
   Phe Tyr Pro Ser Asp Ile Ala Val Glu Trp Glu Ser Asn Gly Gln Pro
           385                 390                 395
   gag aac aac tac aag acc acg cct ccc gtg ctg gac tcc gac ggc tcc        1310
   Glu Asn Asn Tyr Lys Thr Thr Pro Pro Val Leu Asp Ser Asp Gly Ser
       400                 405                 410
   ttc ttc ctc tat agc aag ctc acc gtg gac aag agc agg tgg cag cag        1358
   Phe Phe Leu Tyr Ser Lys Leu Thr Val Asp Lys Ser Arg Trp Gln Gln
   415                 420                 425                 430
   ggg aac gtc ttc tca tgc tcc gtg atg cat gag gct ctg cac aac cac        1406
   Gly Asn Val Phe Ser Cys Ser Val Met His Glu Ala Leu His Asn His
                   435                 440                 445
   tac acg cag aag agc ctc tcc ctg tct ccg ggt aaa tgagcggccg             1452
   Tyr Thr Gln Lys Ser Leu Ser Leu Ser Pro Gly Lys
               450                 455
   c                                                                      1453
<210> 12
   <211> 458
   <212> PRT
   <213> Homo sapiens
<400> 12
   Met Val Ser Tyr Trp Asp Thr Gly Val Leu Leu Cys Ala Leu Leu Ser
    1               5                  10                  15
   Cys Leu Leu Leu Thr Gly Ser Ser Ser Gly Gly Arg Pro Phe Val Glu
               20                  25                  30
   Met Tyr Ser Glu Ile Pro Glu Ile Ile His Met Thr Glu Gly Arg Glu
           35                  40                  45
   Leu Val Ile Pro Cys Arg Val Thr Ser Pro Asn Ile Thr Val Thr Leu
       50                  55                  60
   Lys Lys Phe Pro Leu Asp Thr Leu Ile Pro Asp Gly Lys Arg Ile Ile
   65                  70                  75                  80
   Trp Asp Ser Arg Lys Gly Phe Ile Ile Ser Asn Ala Thr Tyr Lys Glu
                   85                  90                  95
   Ile Gly Leu Leu Thr Cys Glu Ala Thr Val Asn Gly His Leu Tyr Lys
               100                 105                 110
   Thr Asn Tyr Leu Thr His Arg Gln Thr Asn Thr Ile Ile Asp Val Val
           115                 120                 125
   Leu Ser Pro Ser His Gly Ile Glu Leu Ser Val Gly Glu Lys Leu Val
       130                 135                 140
   Leu Asn Cys Thr Ala Arg Thr Glu Leu Asn Val Gly Ile Asp Phe Asn
   145                 150                 155                 160
   Trp Glu Tyr Pro Ser Ser Lys His Gln His Lys Lys Leu Val Asn Arg
                   165                 170                 175
   Asp Leu Lys Thr Gln Ser Gly Ser Glu Met Lys Lys Phe Leu Ser Thr
               180                 185                 190
   Leu Thr Ile Asp Gly Val Thr Arg Ser Asp Gln Gly Leu Tyr Thr Cys
           195                 200                 205
   Ala Ala Ser Ser Gly Leu Met Thr Lys Lys Asn Ser Thr Phe Val Arg
       210                 215                 220
   Val His Glu Lys Gly Pro Gly Asp Lys Thr His Thr Cys Pro Pro Cys
   225                 230                 235                 240
   Pro Ala Pro Glu Leu Leu Gly Gly Pro Ser Val Phe Leu Phe Pro Pro
                   245                 250                 255
   Lys Pro Lys Asp Thr Leu Met Ile Ser Arg Thr Pro Glu Val Thr Cys
               260                 265                 270
   Val Val Val Asp Val Ser His Glu Asp Pro Glu Val Lys Phe Asn Trp
           275                 280                 285
   Tyr Val Asp Gly Val Glu Val His Asn Ala Lys Thr Lys Pro Arg Glu
       290                 295                 300
   Glu Gln Tyr Asn Ser Thr Tyr Arg Val Val Ser Val Leu Thr Val Leu
   305                 310                 315                 320
   His Gln Asp Trp Leu Asn Gly Lys Glu Tyr Lys Cys Lys Val Ser Asn
                   325                 330                 335
   Lys Ala Leu Pro Ala Pro Ile Glu Lys Thr Ile Ser Lys Ala Lys Gly
               340                 345                 350
   Gln Pro Arg Glu Pro Gln Val Tyr Thr Leu Pro Pro Ser Arg Asp Glu
           355                 360                 365
   Leu Thr Lys Asn Gln Val Ser Leu Thr Cys Leu Val Lys Gly Phe Tyr
       370                 375                 380
   Pro Ser Asp Ile Ala Val Glu Trp Glu Ser Asn Gly Gln Pro Glu Asn
   385                 390                 395                 400
   Asn Tyr Lys Thr Thr Pro Pro Val Leu Asp Ser Asp Gly Ser Phe Phe
                   405                 410                 415
   Leu Tyr Ser Lys Leu Thr Val Asp Lys Ser Arg Trp Gln Gln Gly Asn
               420                 425                 430
   Val Phe Ser Cys Ser Val Met His Glu Ala Leu His Asn His Tyr Thr
           435                 440                 445
   Gln Lys Ser Leu Ser Leu Ser Pro Gly Lys
       450                 455
<210> 13
   <211> 1444
   <212> DNA
   <213> Homo sapiens
<220>
   <221> CDS
   <222> (69)...(1433)
<400> 13
   aagcttgggc tgcaggtcga tcgactctag aggatcgatc cccgggcgag ctcgaattcg      60
   caaccacc atg gtc agc tac tgg gac acc ggg gtc ctg ctg tgc gcg ctg     110
            Met Val Ser Tyr Trp Asp Thr Gly Val Leu Leu Cys Ala Leu
             1               5                  10
   ctc agc tgt ctg ctt ctc aca gga tct agt tcc gga ggt aga cct ttc      158
   Leu Ser Cys Leu Leu Leu Thr Gly Ser Ser Ser Gly Gly Arg Pro Phe
    15                  20                  25                  30
   gta gag atg tac agt gaa atc ccc gaa att ata cac atg act gaa gga      206
   Val Glu Met Tyr Ser Glu Ile Pro Glu Ile Ile His Met Thr Glu Gly
                   35                  40                  45
   agg gag ctc gtc att ccc tgc cgg gtt acg tca cct aac atc act gtt       254
   Arg Glu Leu Val Ile Pro Cys Arg Val Thr Ser Pro Asn Ile Thr Val
               50                  55                  60
   act tta aaa aag ttt cca ctt gac act ttg atc cct gat gga aaa cgc       302
   Thr Leu Lys Lys Phe Pro Leu Asp Thr Leu Ile Pro Asp Gly Lys Arg
           65                  70                  75
   ata atc tgg gac agt aga aag ggc ttc atc ata tca aat gca acg tac       350
   Ile Ile Trp Asp Ser Arg Lys Gly Phe Ile Ile Ser Asn Ala Thr Tyr
       80                  85                  90
   aaa gaa ata ggg ctt ctg acc tgt gaa gca aca gtc aat ggg cat ttg       398
   Lys Glu Ile Gly Leu Leu Thr Cys Glu Ala Thr Val Asn Gly His Leu
   95                  100                 105                 110
   tat aag aca aac tat ctc aca cat cga caa acc aat aca atc ata gat       446
   Tyr Lys Thr Asn Tyr Leu Thr His Arg Gln Thr Asn Thr Ile Ile Asp
                   115                 120                 125
   atc cag ctg ttg ccc agg aag tcg ctg gag ctg ctg gta ggg gag aag       494
   Ile Gln Leu Leu Pro Arg Lys Ser Leu Glu Leu Leu Val Gly Glu Lys
               130                 135                 140
   ctg gtc ctc aac tgc acc gtg tgg gct gag ttt aac tca ggt gtc acc       542
   Leu Val Leu Asn Cys Thr Val Trp Ala Glu Phe Asn Ser Gly Val Thr
           145                 150                 155
   ttt gac tgg gac tac cca ggg aag cag gca gag cgg ggt aag tgg gtg       590
   Phe Asp Trp Asp Tyr Pro Gly Lys Gln Ala Glu Arg Gly Lys Trp Val
       160                 165                 170
   ccc gag cga cgc tcc caa cag acc cac aca gaa ctc tcc agc atc ctg       638
   Pro Glu Arg Arg Ser Gln Gln Thr His Thr Glu Leu Ser Ser Ile Leu
   175                 180                 185                 190
   acc atc cac aac gtc agc cag cac gac ctg ggc tcg tat gtg tgc aag       686
   Thr Ile His Asn Val Ser Gln His Asp Leu Gly Ser Tyr Val Cys Lys
                   195                 200                 205
   gcc aac aac ggc atc cag cga ttt cgg gag agc acc gag gtc att gtg       734
   Ala Asn Asn Gly Ile Gln Arg Phe Arg Glu Ser Thr Glu Val Ile Val
               210                 215                 220
   cat gaa aat ggc ccg ggc gac aaa act cac aca tgc cca ccg tgc cca       782
   His Glu Asn Gly Pro Gly Asp Lys Thr His Thr Cys Pro Pro Cys Pro
           225                 230                 235
   gca cct gaa ctc ctg ggg gga ccg tca gtc ttc ctc ttc ccc cca aaa       830
   Ala Pro Glu Leu Leu Gly Gly Pro Ser Val Phe Leu Phe Pro Pro Lys
       240                 245                 250
   ccc aag gac acc ctc atg atc tcc cgg acc cct gag gtc aca tgc gtg       878
   Pro Lys Asp Thr Leu Met Ile Ser Arg Thr Pro Glu Val Thr Cys Val
   255                 260                 265                 270
   gtg gtg gac gtg agc cac gaa gac cct gag gtc aag ttc aac tgg tac       926
   Val Val Asp Val Ser His Glu Asp Pro Glu Val Lys Phe Asn Trp Tyr
                       275                      280                      285
   gtg gac ggc gtg gag gtg cat aat gcc aag aca aag ccg cgg gag gag      974
   Val Asp Gly Val Glu Val His Asn Ala Lys Thr Lys Pro Arg Glu Glu
               290                     295                     300
   cag tac aac agc acg tac cgt gtg gtc agc gtc ctc acc gtc ctg cac     1022
   Gln Tyr Asn Ser Thr Tyr Arg Val Val Ser Val Leu Thr Val Leu His
           305                     310                     315
   cag gac tgg ctg aat ggc aag gag tac aag tgc aag gtc tcc aac aaa     1070
   Gln Asp Trp Leu Asn Gly Lys Glu Tyr Lys Cys Lys Val Ser Asn Lys
       320                     325                     330
   gcc ctc cca gcc ccc atc gag aaa acc atc tcc aaa gcc aaa ggg cag     1118
   Ala Leu Pro Ala Pro Ile Glu Lys Thr Ile Ser Lys Ala Lys Gly Gln
   335                     340                     345                 350
   ccc cga gaa cca cag gtg tac acc ctg ccc cca tcc cgg gat gag ctg     1166
   Pro Arg Glu Pro Gln Val Tyr Thr Leu Pro Pro Ser Arg Asp Glu Leu
                   355                     360                     365
   acc aag aac cag gtc agc ctg acc tgc ctg gtc aaa ggc ttc tat ccc     1214
   Thr Lys Asn Gln Val Ser Leu Thr Cys Leu Val Lys Gly Phe Tyr Pro
               370                     375                     380
   agc gac atc gcc gtg gag tgg gag agc aat ggg cag ccg gag aac aac     1262
   Ser Asp Ile Ala Val Glu Trp Glu Ser Asn Gly Gln Pro Glu Asn Asn
           385                     390                     395
   tac aag acc acg cct ccc gtg ctg gac tcc gac ggc tcc ttc ttc ctc     1310
   Tyr Lys Thr Thr Pro Pro Val Leu Asp Ser Asp Gly Ser Phe Phe Leu
       400                     405                     410
   tat agc aag ctc acc gtg gac aag agc agg tgg cag cag ggg aac gtc     1358
   Tyr Ser Lys Leu Thr Val Asp Lys Ser Arg Trp Gln Gln Gly Asn Val
   415                     420                     425                 430
   ttc tca tgc tcc gtg atg cat gag gct ctg cac aac cac tac acg cag     1406
   Phe Ser Cys Ser Val Met His Glu Ala Leu His Asn His Tyr Thr Gln
                   435                     440                     445
   aag agc ctc tcc ctg tct ccg ggt aaa tgagcggccg c                    1444
   Lys Ser Leu Ser Leu Ser Pro Gly Lys
               450                     455
<210> 14
   <211> 455
   <212> PRT
   <213> Homo sapiens
<400> 14
   Met Val Ser Tyr Trp Asp Thr Gly Val Leu Leu Cys Ala Leu Leu Ser
    1              5                   10                  15
   Cys Leu Leu Leu Thr Gly Ser Ser Ser Gly Gly Arg Pro Phe Val Glu
               20                  25                  30
   Met Tyr Ser Glu Ile Pro Glu Ile Ile His Met Thr Glu Gly Arg Glu
           35                  40                  45
   Leu Val Ile Pro Cys Arg Val Thr Ser Pro Asn Ile Thr Val Thr Leu
       50                  55                  60
   Lys Lys Phe Pro Leu Asp Thr Leu Ile Pro Asp Gly Lys Arg Ile Ile
   65                  70                  75                  80
   Trp Asp Ser Arg Lys Gly Phe Ile Ile Ser Asn Ala Thr Tyr Lys Glu
   85 90 95
   Ile Gly Leu Leu Thr Cys Glu Ala Thr Val Asn Gly His Leu Tyr Lys
   100 105 110
   Thr Asn Tyr Leu Thr His Arg Gln Thr Asn Thr Ile Ile Asp Ile Gln
   115 120 125
   Leu Leu Pro Arg Lys Ser Leu Glu Leu Leu Val Gly Glu Lys Leu Val
   130 135 140
   Leu Asn Cys Thr Val Trp Ala Glu Phe Asn Ser Gly Val Thr Phe Asp
   145 150 155 160
   Trp Asp Tyr Pro Gly Lys Gln Ala Glu Arg Gly Lys Trp Val Pro Glu
   165 170 175
   Arg Arg Ser Gln Gln Thr His Thr Glu Leu Ser Ser Ile Leu Thr Ile
   180 185 190
   His Asn Val Ser Gln His Asp Leu Gly Ser Tyr Val Cys Lys Ala Asn
   195 200 205
   Asn Gly Ile Gln Arg Phe Arg Glu Ser Thr Glu Val Ile Val His Glu
   210 215 220
   Asn Gly Pro Gly Asp Lys Thr His Thr Cys Pro Pro Cys Pro Ala Pro
   225 230 235 240
   Glu Leu Leu Gly Gly Pro Ser Val Phe Leu Phe Pro Pro Lys Pro Lys
   245 250 255
   Asp Thr Leu Met Ile Ser Arg Thr Pro Glu Val Thr Cys Val Val Val
   260 265 270
   Asp Val Ser His Glu Asp Pro Glu Val Lys Phe Asn Trp Tyr Val Asp
   275 280 285
   Gly Val Glu Val His Asn Ala Lys Thr Lys Pro Arg Glu Glu Gln Tyr
   290 295 300
   Asn Ser Thr Tyr Arg Val Val Ser Val Leu Thr Val Leu His Gln Asp
   305 310 315 320
   Trp Leu Asn Gly Lys Glu Tyr Lys Cys Lys Val Ser Asn Lys Ala Leu
   325 330 335
   Pro Ala Pro Ile Glu Lys Thr Ile Ser Lys Ala Lys Gly Gln Pro Arg
   340 345 350
   Glu Pro Gln Val Tyr Thr Leu Pro Pro Ser Arg Asp Glu Leu Thr Lys
   355 360 365
   Asn Gln Val Ser Leu Thr Cys Leu Val Lys Gly Phe Tyr Pro Ser Asp
   370 375 380
   Ile Ala Val Glu Trp Glu Ser Asn Gly Gln Pro Glu Asn Asn Tyr Lys
   385 390 395 400
   Thr Thr Pro Pro Val Leu Asp Ser Asp Gly Ser Phe Phe Leu Tyr Ser
   405 410 415
   Lys Leu Thr Val Asp Lys Ser Arg Trp Gln Gln Gly Asn Val Phe Ser
   420 425 430
   Cys Ser Val Met His Glu Ala Leu His Asn His Tyr Thr Gln Lys Ser
   435 440 445
   Leu Ser Leu Ser Pro Gly Lys
   450 455
<210> 15
   <211> 1377
   <212> DNA
   <213> Homo sapiens
<220>
   <221> CDS
   <222> (1)...(1374)
<400> 15
   atg gtc agc tac tgg gac acc ggg gtc ctg ctg tgc gcg ctg ctc agc        48
   Met Val Ser Tyr Trp Asp Thr Gly Val Leu Leu Cys Ala Leu Leu Ser
    1              5                   10                  15
   tgt ctg ctt ctc aca gga tct agt tcc gga agt gat acc ggt aga cct        96
   Cys Leu Leu Leu Thr Gly Ser Ser Ser Gly Ser Asp Thr Gly Arg Pro
               20                  25                  30
   ttc gta gag atg tac agt gaa atc ccc gaa att ata cac atg act gaa      144
   Phe Val Glu Met Tyr Ser Glu Ile Pro Glu Ile Ile His Met Thr Glu
           35                  40                  45
   gga agg gag ctc gtc att ccc tgc cgg gtt acg tca cct aac atc act      192
   Gly Arg Glu Leu Val Ile Pro Cys Arg Val Thr Ser Pro Asn Ile Thr
       50                  55                  60
   gtt act tta aaa aag ttt cca ctt gac act ttg atc cct gat gga aaa      240
   Val Thr Leu Lys Lys Phe Pro Leu Asp Thr Leu Ile Pro Asp Gly Lys
   65                  70                  75                  80
   cgc ata atc tgg gac agt aga aag ggc ttc atc ata tca aat gca acg      288
   Arg Ile Ile Trp Asp Ser Arg Lys Gly Phe Ile Ile Ser Asn Ala Thr
                   85                  90                  95
   tac aaa gaa ata ggg ctt ctg acc tgt gaa gca aca gtc aat ggg cat      336
   Tyr Lys Glu Ile Gly Leu Leu Thr Cys Glu Ala Thr Val Asn Gly His
               100                 105                 110
   ttg tat aag aca aac tat ctc aca cat cga caa acc aat aca atc ata      384
   Leu Tyr Lys Thr Asn Tyr Leu Thr His Arg Gln Thr Asn Thr Ile Ile
           115                 120                 125
   gat gtg gtt ctg agt ccg tct cat gga att gaa cta tct gtt gga gaa      432
   Asp Val Val Leu Ser Pro Ser His Gly Ile Glu Leu Ser Val Gly Glu
       130                 135                 140
   aag ctt gtc tta aat tgt aca gca aga act gaa cta aat gtg ggg att      480
   Lys Leu Val Leu Asn Cys Thr Ala Arg Thr Glu Leu Asn Val Gly Ile
   145                 150                 155                 160
   gac ttc aac tgg gaa tac cct tct tcg aag cat cag cat aag aaa ctt      528
   Asp Phe Asn Trp Glu Tyr Pro Ser Ser Lys His Gln His Lys Lys Leu
                   165                 170                 175
   gta aac cga gac cta aaa acc cag tct ggg agt gag atg aag aaa ttt      576
   Val Asn Arg Asp Leu Lys Thr Gln Ser Gly Ser Glu Met Lys Lys Phe
               180                 185                 190
   ttg agc acc tta act ata gat ggt gta acc cgg agt gac caa gga ttg      624
   Leu Ser Thr Leu Thr Ile Asp Gly Val Thr Arg Ser Asp Gln Gly Leu
           195                 200                 205
   tac acc tgt gca gca tcc agt ggg ctg atg acc aag aag aac agc aca      672
   Tyr Thr Cys Ala Ala Ser Ser Gly Leu Met Thr Lys Lys Asn Ser Thr
       210                 215                 220
   ttt gtc agg gtc cat gaa aag gac aaa act cac aca tgc cca ccg tgc      720
   Phe Val Arg Val His Glu Lys Asp Lys Thr His Thr Cys Pro Pro Cys
   225                 230                 235                 240
   cca gca cct gaa ctc ctg ggg gga ccg tca gtc ttc ctc ttc ccc cca      768
   Pro Ala Pro Glu Leu Leu Gly Gly Pro Ser Val Phe Leu Phe Pro Pro
                   245                 250                 255
   aaa ccc aag gac acc ctc atg atc tcc cgg acc cct gag gtc aca tgc      816
   Lys Pro Lys Asp Thr Leu Met Ile Ser Arg Thr Pro Glu Val Thr Cys
                   260                 265                 270
   gtg gtg gtg gac gtg agc cac gaa gac cct gag gtc aag ttc aac tgg      864
   Val Val Val Asp Val Ser His Glu Asp Pro Glu Val Lys Phe Asn Trp
           275                 280                 285
   tac gtg gac ggc gtg gag gtg cat aat gcc aag aca aag ccg cgg gag      912
   Tyr Val Asp Gly Val Glu Val His Asn Ala Lys Thr Lys Pro Arg Glu
       290                 295                 300
   gag cag tac aac agc acg tac cgt gtg gtc agc gtc ctc acc gtc ctg      960
   Glu Gln Tyr Asn Ser Thr Tyr Arg Val Val Ser Val Leu Thr Val Leu
   305                 310                 315                 320
   cac cag gac tgg ctg aat ggc aag gag tac aag tgc aag gtc tcc aac     1008
   His Gln Asp Trp Leu Asn Gly Lys Glu Tyr Lys Cys Lys Val Ser Asn
                   325                 330                 335
   aaa gcc ctc cca gcc ccc atc gag aaa acc atc tcc aaa gcc aaa ggg     1056
   Lys Ala Leu Pro Ala Pro Ile Glu Lys Thr Ile Ser Lys Ala Lys Gly
               340                 345                 350
   cag ccc cga gaa cca cag gtg tac acc ctg ccc cca tcc cgg gat gag     1104
   Gln Pro Arg Glu Pro Gln Val Tyr Thr Leu Pro Pro Ser Arg Asp Glu
           355                 360                 365
   ctg acc aag aac cag gtc agc ctg acc tgc ctg gtc aaa ggc ttc tat     1152
   Leu Thr Lys Asn Gln Val Ser Leu Thr Cys Leu Val Lys Gly Phe Tyr
       370                 375                 380
   ccc agc gac atc gcc gtg gag tgg gag agc aat ggg cag ccg gag aac     1200
   Pro Ser Asp Ile Ala Val Glu Trp Glu Ser Asn Gly Gln Pro Glu Asn
   385                 390                 395                 400
   aac tac aag acc acg cct ccc gtg ctg gac tcc gac ggc tcc ttc ttc     1248
   Asn Tyr Lys Thr Thr Pro Pro Val Leu Asp Ser Asp Gly Ser Phe Phe
                   405                 410                 415
   ctc tac agc aag ctc acc gtg gac aag agc agg tgg cag cag ggg aac     1296
   Leu Tyr Ser Lys Leu Thr Val Asp Lys Ser Arg Trp Gln Gln Gly Asn
               420                 425                 430
   gtc ttc tca tgc tcc gtg atg cat gag gct ctg cac aac cac tac acg     1344
   Val Phe Ser Cys Ser Val Met His Glu Ala Leu His Asn His Tyr Thr
           435                 440                 445
   cag aag agc ctc tcc ctg tct ccg ggt aaa tga                         1377
   Gln Lys Ser Leu Ser Leu Ser Pro Gly Lys
       450                 455
<210> 16
   <211> 458
   <212> PRT
   <213> Homo sapiens
<400> 16
   Met Val Ser Tyr Trp Asp Thr Gly Val Leu Leu Cys Ala Leu Leu Ser
    1               5                  10                  15
   Cys Leu Leu Leu Thr Gly Ser Ser Ser Gly Ser Asp Thr Gly Arg Pro
               20                  25                  30
   Phe Val Glu Met Tyr Ser Glu Ile Pro Glu Ile Ile His Met Thr Glu
           35                  40                  45
   Gly Arg Glu Leu Val Ile Pro Cys Arg Val Thr Ser Pro Asn Ile Thr
       50                  55                  60
   Val Thr Leu Lys Lys Phe Pro Leu Asp Thr Leu Ile Pro Asp Gly Lys
   65                  70                  75                  80
   Arg Ile Ile Trp Asp Ser Arg Lys Gly Phe Ile Ile Ser Asn Ala Thr
                   85                  90                  95
   Tyr Lys Glu Ile Gly Leu Leu Thr Cys Glu Ala Thr Val Asn Gly His
               100                 105                 110
   Leu Tyr Lys Thr Asn Tyr Leu Thr His Arg Gln Thr Asn Thr Ile Ile
           115                 120                 125
   Asp Val Val Leu Ser Pro Ser His Gly Ile Glu Leu Ser Val Gly Glu
       130                 135                 140
   Lys Leu Val Leu Asn Cys Thr Ala Arg Thr Glu Leu Asn Val Gly Ile
   145                 150                 155                 160
   Asp Phe Asn Trp Glu Tyr Pro Ser Ser Lys His Gln His Lys Lys Leu
                   165                 170                 175
   Val Asn Arg Asp Leu Lys Thr Gln Ser Gly Ser Glu Met Lys Lys Phe
               180                 185                 190
   Leu Ser Thr Leu Thr Ile Asp Gly Val Thr Arg Ser Asp Gln Gly Leu
           195                 200                 205
   Tyr Thr Cys Ala Ala Ser Ser Gly Leu Met Thr Lys Lys Asn Ser Thr
       210                 215                 220
   Phe Val Arg Val His Glu Lys Asp Lys Thr His Thr Cys Pro Pro Cys
   225                 230                 235                 240
   Pro Ala Pro Glu Leu Leu Gly Gly Pro Ser Val Phe Leu Phe Pro Pro
                   245                 250                 255
   Lys Pro Lys Asp Thr Leu Met Ile Ser Arg Thr Pro Glu Val Thr Cys
               260                 265                 270
   Val Val Val Asp Val Ser His Glu Asp Pro Glu Val Lys Phe Asn Trp
           275                 280                 285
   Tyr Val Asp Gly Val Glu Val His Asn Ala Lys Thr Lys Pro Arg Glu
       290                 295                 300
   Glu Gln Tyr Asn Ser Thr Tyr Arg Val Val Ser Val Leu Thr Val Leu
   305                 310                 315                 320
   His Gln Asp Trp Leu Asn Gly Lys Glu Tyr Lys Cys Lys Val Ser Asn
                   325                 330                 335
   Lys Ala Leu Pro Ala Pro Ile Glu Lys Thr Ile Ser Lys Ala Lys Gly
               340                 345                 350
   Gln Pro Arg Glu Pro Gln Val Tyr Thr Leu Pro Pro Ser Arg Asp Glu
           355                 360                 365
   Leu Thr Lys Asn Gln Val Ser Leu Thr Cys Leu Val Lys Gly Phe Tyr
       370                 375                 380
   Pro Ser Asp Ile Ala Val Glu Trp Glu Ser Asn Gly Gln Pro Glu Asn
   385                 390                 395                 400
   Asn Tyr Lys Thr Thr Pro Pro Val Leu Asp Ser Asp Gly Ser Phe Phe
                   405                 410                 415
   Leu Tyr Ser Lys Leu Thr Val Asp Lys Ser Arg Trp Gln Gln Gly Asn
               420                 425                 430
   Val Phe Ser Cys Ser Val Met His Glu Ala Leu His Asn His Tyr Thr
           435                 440                 445
   Gln Lys Ser Leu Ser Leu Ser Pro Gly Lys
       450                 455
<210> 17
   <211> 430
   <212> PRT
   <213> Homo sapiens
<400> 17
   Gly Arg Pro Phe Val Glu Met Tyr Ser Glu Ile Pro Glu Ile Ile His
   1               5                   10                  15
   Met Thr Glu Gly Arg Glu Leu Val Ile Pro Cys Arg Val Thr Ser Pro
               20                  25                  30
   Asn Ile Thr Val Thr Leu Lys Lys Phe Pro Leu Asp Thr Leu Ile Pro
           35                  40                  45
   Asp Gly Lys Arg Ile Ile Trp Asp Ser Arg Lys Gly Phe Ile Ile Ser
       50                  55                  60
   Asn Ala Thr Tyr Lys Glu Ile Gly Leu Leu Thr Cys Glu Ala Thr Val
   65                  70                  75                  80
   Asn Gly His Leu Tyr Lys Thr Asn Tyr Leu Thr His Arg Gln Thr Asn
                   85                  90                  95
   Thr Ile Ile Asp Val Val Leu Ser Pro Ser His Gly Ile Glu Leu Ser
               100                 105                 110
   Val Gly Glu Lys Leu Val Leu Asn Cys Thr Ala Arg Thr Glu Leu Asn
           115                 120                 125
   Val Gly Ile Asp Phe Asn Trp Glu Tyr Pro Ser Ser Lys His Gln His
       130                 135                 140
   Lys Lys Leu Val Asn Arg Asp Leu Lys Thr Gln Ser Gly Ser Glu Met
   145                 150                 155                 160
   Lys Lys Phe Leu Ser Thr Leu Thr Ile Asp Gly Val Thr Arg Ser Asp
                   165                 170                 175
   Gln Gly Leu Tyr Thr Cys Ala Ala Ser Ser Gly Leu Met Thr Lys Lys
               180                 185                 190
   Asn Ser Thr Phe Val Arg Val His Glu Lys Gly Pro Gly Asp Lys Thr
           195                 200                 205
   His Thr Cys Pro Pro Cys Pro Ala Pro Glu Leu Leu Gly Gly Pro Ser
       210                 215                 220
   Val Phe Leu Phe Pro Pro Lys Pro Lys Asp Thr Leu Met Ile Ser Arg
   225                 230                 235                 240
   Thr Pro Glu Val Thr Cys Val Val Val Asp Val Ser His Glu Asp Pro
                   245                 250                 255
   Glu Val Lys Phe Asn Trp Tyr Val Asp Gly Val Glu Val His Asn Ala
               260                 265                 270
   Lys Thr Lys Pro Arg Glu Glu Gln Tyr Asn Ser Thr Tyr Arg Val Val
           275                 280                 285
   Ser Val Leu Thr Val Leu His Gln Asp Trp Leu Asn Gly Lys Glu Tyr
       290                 295                 300
   Lys Cys Lys Val Ser Asn Lys Ala Leu Pro Ala Pro Ile Glu Lys Thr
   305                 310                 315                 320
   Ile Ser Lys Ala Lys Gly Gln Pro Arg Glu Pro Gln Val Tyr Thr Leu
                   325                 330                 335
   Pro Pro Ser Arg Asp Glu Leu Thr Lys Asn Gln Val Ser Leu Thr Cys
               340                 345                 350
   Leu Val Lys Gly Phe Tyr Pro Ser Asp Ile Ala Val Glu Trp Glu Ser
           355                 360                 365
   Asn Gly Gln Pro Glu Asn Asn Tyr Lys Thr Thr Pro Pro Val Leu Asp
       370                 375                 380
   Ser Asp Gly Ser Phe Phe Leu Tyr Ser Lys Leu Thr Val Asp Lys Ser
   385                 390                 395                 400
   Arg Trp Gln Gln Gly Asn Val Phe Ser Cys Ser Val Met His Glu Ala
                   405                 410                 415
   Leu His Asn His Tyr Thr Gln Lys Ser Leu Ser Pro Gly Lys
               420                 425                 430
<210> 18
   <211> 36
   <212> DNA
   <213> Artificial Sequence
<220>
   <223> primer
<400> 18
   gactagcagt ccggaggtag acctttcgta gagatg 36
<210> 19
   <211> 33
   <212> DNA
   <213> Artificial Sequence
<220>
   <223> primer
<400> 19
   cggactcaga accacatcta tgattgtatt ggt 33
<210> 20
   <211> 7
   <212> PRT
   <213> Homo sapiens
<400> 20
   Gly Arg Pro Phe Val Glu Met
   1 5
<210> 21
   <211> 35
   <212> DNA
   <213> Artificial Sequence
<220>
   <223> primer
<400> 21
   acaatcatag atgtggttct gagtccgtct catgg 35
<210> 22
   <211> 38
   <212> DNA
   <213> Artificial Sequence
<220>
   <223> primer
<400> 22
   gataatgccc gggccctttt catggaccct gacaaatg 38
<210> 23
   <211> 6
   <212> PRT
   <213> Homo sapiens
<400> 23
   Val Arg Val His Glu Lys
   1 5
<210> 24
   <211> 36
   <212> DNA
   <213> Artificial Sequence
<220>
   <223> primer
<400> 24
   gactagcagt ccggaggtag acctttcgta gagatg 36
<210> 25
   <211> 38
   <212> DNA
   <213> Artificial Sequence
<220>
   <223> primer
<400> 25
   ttcctgggca acagctggat atctatgatt gtattggt 38
<210> 26
   <211> 4
   <212> PRT
   <213> Homo sapiens
<400> 26
   Ile Gln Leu Leu
   1
<210> 27
   <211> 39
   <212> DNA
   <213> Artificial Sequence
<220>
   <223> primer
<400> 27
   atccagctgt tgcccaggaa gtcgctggag ctgctggta 39
<210> 28
   <211> 39
   <212> DNA
   <213> Artificial Sequence
<220>
   <223> primer
<400> 28
   attttcatgc acaatgacct cggtgctctc ccgaaatcg 39
<210> 29
   <211> 38
   <212> DNA
   <213> Artificial Sequence
<220>
   <223> primer
<400> 29
   tcatagatat ccagctgttg cccaggaagt cgctggag 38
<210> 30
   <211> 39
   <212> DNA
   <213> Artificial Sequence
<220>
   <223> primer
<400> 30
   gataatgccc gggccatttt catgcacaat gacctcggt 39
<210> 31
   <211> 6
   <212> PRT
   <213> Homo sapiens
<400> 31
   Val Ile Val His Glu Asn
   1 5
<210> 32
   <211> 10
   <212> PRT
   <213> Artificial Sequence
<220>
   <223> modified Fltl receptor
<400> 32
   Lys Asn Lys Arg Ala Ser Val Arg Arg Arg
   1 5 10
<210> 33
   <211> 8
   <212> PRT
   <213> Artificial Sequence
<220>
   <223> modified Flt1 receptor
<400> 33
   Asn Ala Ser Val Asn Gly Ser Arg
   1 5
<210> 34
   <211> 10
   <212> PRT
   <213> Artificial Sequence
<220>
   <223> modified Flt1 receptor
<400> 34
   Lys Asn Lys Cys Ala Ser Val Arg Arg Arg
   1 5 10
<210> 35
   <211> 4
   <212> PRT
   <213> Homo sapiens
<400> 35
   Ser Lys Leu Lys
   1
<210> 36
   <211> 9
   <212> PRT
   <213> Homo sapiens
<400> 36
   Lys Asn Lys Arg Ala Ser Val Arg Arg
    1               5

## Description

### INTRODUCTION

The field of this invention is modified polypeptides with improved pharmacokinetics. Specifically, the field of this invention relates to Flt1 receptor polypeptides that have been modified in such a way as to improve their pharmacokinetic profile. The field of this invention also relates to methods of making the modified polypeptides and using the modified polypeptides to treat eye disorders.

### BACKGROUND

The ability of polypeptide ligands to bind to cells and thereby elicit a phenotypic response such as cell growth, survival, cell product secretion, or differentiation is often mediated through transmembrane receptors on the cells. The extracellular domain of such receptors (i.e. that portion of the receptor that is displayed on the surface of the cell) is generally the most distinctive portion of the molecule, as it provides the protein with its ligand binding characteristic. Binding of a ligand to the extracellular domain generally results in signal transduction which transmits a biological signal to intracellular targets. Often, this signal transduction acts via a catalytic intracellular domain. The particular array of sequence motifs of this catalytic intracellular domain determines its access to potential kinase substrates (Mohammadi, et al.,1990, Mol. Cell. Biol. 11:5068-5078; Fantl, et al., 1992, Cell 69:413-413). Examples of receptors that transduce signals via catalytic intracellular domains include the receptor tyrosine kinases (RTKs) such as the Trk family of receptors which are generally limited to cells of the nervous system, the cytokine family of receptors including the tripartate CNTF receptor complex (Stahl & Yancopoulos, 1994, J. Neurobio. 25:1454-1466) which is also generally limited to the cells of the nervous system, G-protein coupled receptors such as the β₂-adrenergic receptor found on, for instance, cardiac muscle cells, and the multimeric IgE high affinity receptor FcεRI which is localized, for the most part, on mast cells and basophils (Sutton & Gould, 1993, Nature 366:421-428).

All receptors identified so far appear to undergo dimerization, multimerization, or some related conformational change following ligand binding (Schlessinger, J., 1988, Trend Biochem. Sci. 13:443-447; Ullrich & Schlessinger, 1990, Cell 61:203-212; Schlessinger & Ullrich, 1992, Neuron 9:383-391) and molecular interactions between dimerizing intracellular domains lead to activation of catalytic function. In some instances, such as platelet-derived growth factor (PDGF), the ligand is a dimer that binds two receptor molecules (Hart, et al., 1988, Science, 240:1529-1531; Heldin, 1989, J. Biol. Chem. 264:8905-8912) while, for example, in the case of epidermal growth factor (EGF), the ligand is a monomer (Weber, et al., 1984, J. Biol. Chem. 259:14631-14636). In the case of the FcεRI receptor, the ligand, IgE, exists bound to FcεRI in a monomeric fashion and only becomes activated when antigen binds to the IgE/FcεRI complex and cross-links adjacent IgE molecules (Sutton & Gould, 1993, Nature 366:421-428).

Often, the tissue distribution of a particular receptor within higher organisms provides insight into the biological function of the receptor. The RTKs for some growth and differentiation factors, such as fibroblast growth factor (FGF), are widely expressed and therefore appear to play some general role in tissue growth and maintenance. Members of the Trk RTK family (Glass & Yancopoulos, 1993, Trends in Cell Biol. 3:262-268) of receptors are more generally limited to cells of the nervous system, and the Nerve Growth Factor family consisting of nerve growth factor (NGF), brain-derived neurotrophic factor (BDNF), neurotrophin-3 (NT-3) and neurotrophin-4/5 (NT-4/5), which bind the Trk RTK family receptors, promote the differentiation of diverse groups of neurons in the brain and periphery (Lindsay, R. M, 1993, in Neurotrophic Factors, S.E. Loughlin & J.H. Fallon, eds., pp. 257-284, San Diego, CA, Academic Press). FcεRI is localized to a very limited number of types of cells such as mast cells and basophils. Mast cells derive from bone marrow pluripotent hematopoietic stem cell lineage, but complete their maturation in the tissue following migration from the blood stream (See Janeway & Travers, 1996, in Immunobiology, 2d. Edition, M. Robertson & E. Lawrence, eds., pp. 1:3-1:4, Current Biology Ltd., London, UK, Publisher) and are involved in the allergic response.

Many studies have demonstrated that the extracellular domain of a receptor provides the specific ligand binding characteristic. Furthermore, the cellular environment in which a receptor is expressed may influence the biological response exhibited upon binding of a ligand to the receptor. For example, when a neuronal cell expressing a Trk receptor is exposed to a neurotrophin which binds to that receptor, neuronal survival and differentiation results. When the same receptor is expressed by a fibroblast, exposure to the neurotrophin results in proliferation of the fibroblast (Glass, et al., 1991, Cell 66:405-413).

A class of cell-derived dimeric mitogens with selectivity for vascular endothelial cells has been identified and designated vascular endothelial cell growth factor (VEGF). VEGF has been purified from conditioned growth media of rat glioma cells [Conn et al., (1990), Proc. Natl. Acad. Sci. U.S.A., 87. pp 2628-2632]; and conditioned growth media of bovine pituitary follicle stellate cells [Ferrara and Henzel, (1989), Biochem. Biophys. Res. Comm., 161, pp. 851-858; Gozpadorowicz et al., (1989), Proc. Natl. Acad. Sci. U.S.A., 86, pp. 7311-7315] and conditioned growth medium from human U937 cells [Connolly, D. T. et al. (1989), Science, 246, pp. 1309-1312]. VEGF is a dimer with an apparent molecular mass of about 46 kDa with each subunit having an apparent molecular mass of about 23 kDa. VEGF has some structural similarities to platelet derived growth factor (PDGF), which is a mitogen for connective tissue cells but not mitogenic for vascular endothelial cells from large vessels.

The membrane-bound tyrosine kinase receptor, known as Flt, was shown to be a VEGF receptor [DeVries, C. et al., (1992), Science, 255, pp.989-991]. The Flt receptor specifically binds VEGF which induces mitogenesis. Another form of the VEGF receptor, designated KDR, is also known to bind VEGF and induce mitogenesis. The partial cDNA sequence and nearly full length protein sequence of KDR is known as well [Terman, B. I. et al., (1991) Oncogene 6, pp. 1677-1683; Terman, B. I. et al., (1992) Biochem. Biophys. Res. Comm. 187, pp. 1579-1586].

Persistent angiogenesis may cause or exacerbate certain diseases such as psoriasis, rheumatoid arthritis, hemangiomas, angiofibromas, diabetic retinopathy and neovascular glaucoma. An inhibitor of VEGF activity would be useful as a treatment for such diseases and other VEGF-induced pathological angiogenesis and vascular permeability conditions, such as tumor vascularization. The present invention relates to a VEGF inhibitor that is based on the VEGF receptor Flt1.

Plasma leakage, a key component of inflammation, occurs in a distinct subset of microvessels. In particular, in most organs plasma leakage occurs specifically in the venules. Unlike arterioles and capillaries, venules become leaky in response to numerous inflammatory mediators including histamine, bradykinin, and serotonin. One characteristic of inflammation is the plasma leakage that results from intercellular gaps that form in the endothelium of venules. Most experimental models of inflammation indicate that these intercellular gaps occur between the endothelial cells of postcapillary and collecting venules (Baluk, P., et al., Am. J. Pathol. 1998 152:1463-76). It has been shown that certain lectins may be used to reveal features of focal sites of plasma leakage, endothelial gaps, and finger-like processes at endothelial cell borders in inflamed venules (Thurston, G., et al., Am. J. Physiol, 1996, 271: H2547-62). In particular, plant lectins have been used to visualize morphological changes at endothelial cell borders in inflamed venules of, for example, the rat trachea. Lectins, such as conconavalin A and ricin, that bind focally to inflamed venules reveal regions of the subendothelial vessel wall exposed by gaps that correspond to sites of plasma leakage (Thurston, G., et al., Am J Physiol, 1996, 271: H2547-62).

The properties of the microvessels are dynamic. Chronic inflammatory diseases, for example, are associated with microvascular remodeling, including angiogenesis and microvessel enlargement. Microvessels can also remodel by acquiring abnormal phenotypic properties. In a murine model of chronic airway inflammation, airway capillaries acquire properties of venules, including widened vessel diameter, increased immunoreactivity for von Willebrand factor, and increased immunoreactivity for P-selectin. In addition, these remodeled vessels leak in response to inflammatory mediators, whereas vessels in the same position in the airways of normal mice do not.

Certain substances have been shown to decrease or inhibit vascular permeability and/or plasma leakage. For example, mystixins are synthetic polypeptides that have been reported to inhibit plasma leakage without blocking endothelial gap formation (Baluk, P., et al., J. Pharmacol. Exp. Ther., 1998, 284: 693-9). Also, the beta 2-adrenergic receptor agonist formoterol reduces microvascular leakage by inhibiting endothelial gap formation (Baluk, P. and McDonald, D.M., Am. J. Physiol., 1994, 266:L461-8).

The angiopoietins and members of the vascular endothelial growth factor (VEGF) family are the only growth factors thought to be largely specific for vascular endothelial cells. Targeted gene inactivation studies in mice have shown that VEGF is necessary for the early stages of vascular development and that Ang-1 is required for later stages of vascular remodeling.

US Patent No. 6,011,003, issued January 4, 2000, in the name of Metris Therapeutics Limited, discloses an altered, soluble form of FLT polypeptide being capable of binding to VEGF and thereby exerting an inhibitory effect thereon, the polypeptide comprising five or fewer complete immunoglobulin domains.

US Patent No. 5,712,380, issued January 27, 1998 and assigned to Merck & Co., discloses vascular endothelial cell growth factor (VEGF) inhibitors that are naturally occurring or recombinantly engineered soluble forms with or without a C-terminal transmembrane region of the receptor for VEGF.

Also assigned to Merck & Co. is PCT Publication No. WO 98/13071, published April 2, 1998, which discloses gene therapy methodology for inhibition of primary tumor growth and metastasis by gene transfer of a nucleotide sequence encoding a soluble receptor protein which binds to VEGF.

PCT Publication No. WO 97/44453, published November 27, 1997, in the name of Genentech, Inc., discloses novel chimeric VEGF receptor proteins comprising amino acid sequences derived from the vascular endothelial growth factor (VEGF) receptors Flt1 and KDR, including the murine homologue to the human KDR receptor FLK1, wherein said chimeric VEGF receptor proteins bind to VEGF and antagonize the endothelial cell proliferative and angiogenic activity thereof.

PCT Publication No. WO 97/13787, published April 17, 1997, in the name of Toa Gosei Co., LTD., discloses a low molecular weight VEGF inhibitor usable in the treatment of diseases accompanied by neovascularization such as solid tumors. A polypeptide containing the first immunoglobulin-like domain and the second immunoglobulin-like domain in the extracellular region of a VEGF receptor FLT but not containing the sixth immunoglobulin-like domain and the seventh immunoglobulin-like domain thereof shows a VEGF inhibitory activity.

Sharifi, J. et al., 1998, The Quarterly Jour. of Nucl. Med. 42:242-249, disclose that because monoclonal antibodies (MAbs) are basic, positively charged proteins, and mammalian cells are negatively charged, the electrostatic interactions between the two can create higher levels of background binding resulting in low tumor to normal organ ratios. To overcome this effect, the investigators attempted to improve MAb clearance by using various methods such as secondary agents as well as chemical and charge modifications of the MAb itself.

Jensen-Pippo, et al., 1996, Pharmaceutical Research 13:102-107, disclose that pegylation of a therapeutic protein, recombinant human granulocyte colony stimulating factor (PEG-G-CSF), results in an increase in stability and in retention of *in vivo* bioactivity when administered by the intraduodenal route.

Tsutsumi, et al., 1997, Thromb Haemost. 77:168-73, disclose experiments wherein the *in vivo* thrombopoietic activity of polyethylene glycol-modified interleukin-6 (MPEG-IL-6), in which 54% of the 14 lysine amino groups of IL-6 were coupled with PEG, was compared to that of native IL-6.

Yang, et al., 1995, Cancer 76:687-94, disclose that conjugation of polyethylene glycol to recombinant human interleukin-2 (IL-2) results in a compound, polyethylene glycol-modified IL-2 (PEG-IL-2) that retains the in vitro and in vivo activity of IL-2, but exhibits a markedly prolonged circulating half-life.

R. Duncan and F. Spreafico, Clin. Pharmacokinet. 27: 290-306, 296 (1994) review efforts to improve the plasma half-life of asparaginase by conjugating polyethylene glycol.

PCT International Publication No. WO 99/03996 published January 28, 1999 in the name of Regeneron Pharmaceuticals, Inc. and The Regents of The University of California describes modified human noggin polypeptides having deletions of regions of basic amino acids. The modified human noggin polypeptides are described as retaining biological activity while having reduced affinity for heparin and superior pharmacokinetics in animal sera as compared to the unmodified human noggin.

### SUMMARY OF THE INVENTION

The present invention is directed to: use of a fusion polypeptide capable of binding vascular endothelial growth factor (VEGF) in the manufacture of a medicament for treating an eye disorder characterized by vascular permeability, wherein the fusion polypeptide comprises:
(a) a VEGF receptor component consisting essentially of an immunoglobulin-like (Ig) domain 2 of a first VEGF receptor; and Ig domain 3 of a second VEGF receptor; and
(b) a multimerizing component;
and wherein the first VEGF receptor is Flt1, the second VEGF receptor is Flk1 or Flt4, and the VEGF receptor component is the only VEGF receptor component of the fusion polypeptide.

In a preferred embodiment, the nucleotide sequence encoding Ig domain 2 of the extracellular domain of the first VEGF receptor is upstream of the nucleotide sequence encoding Ig domain 3 of the extracellular domain of the second VEGF receptor.

In another preferred embodiment, the nucleotide sequence encoding lg domain 2 of the extracellular domain of the first VEGF receptor is downstream of the nucleotide sequence encoding lg domain 3 of the extracellular domain of the second VEGF receptor.

In a preferred embodiment of the invention, the multimerizing component comprises an immunoglobulin domain.

In another embodiment, the immunoglobulin domain is selected from the group consisting of the Fc domain of IgG, the heavy chain of IgG, and the light chain of IgG.

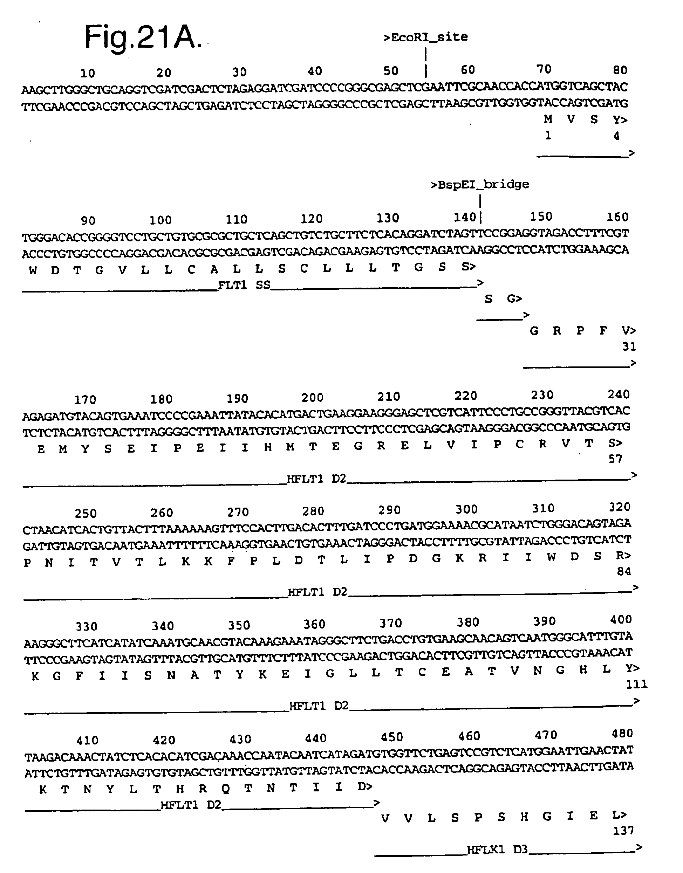

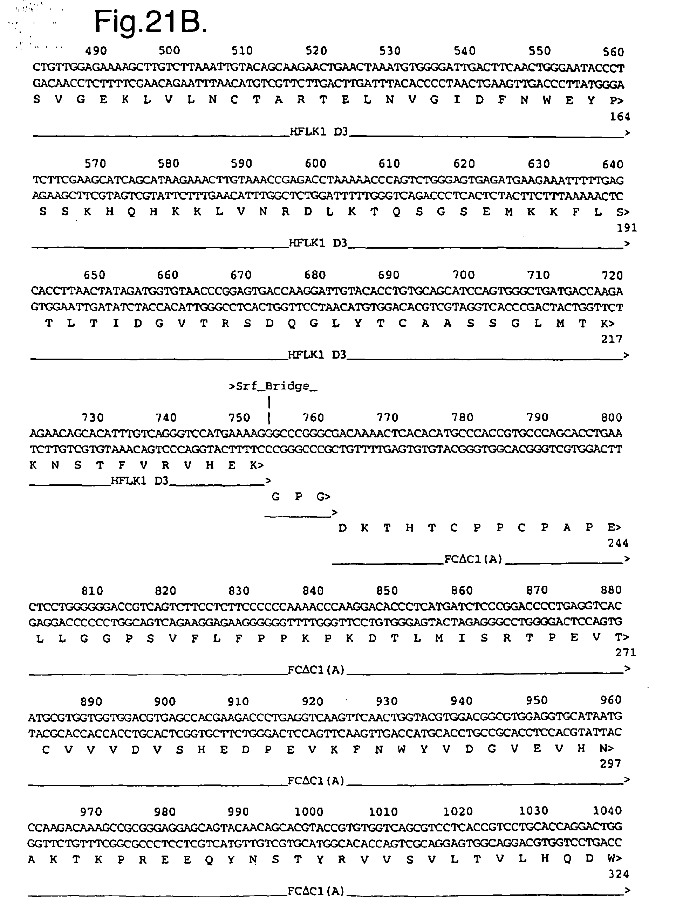

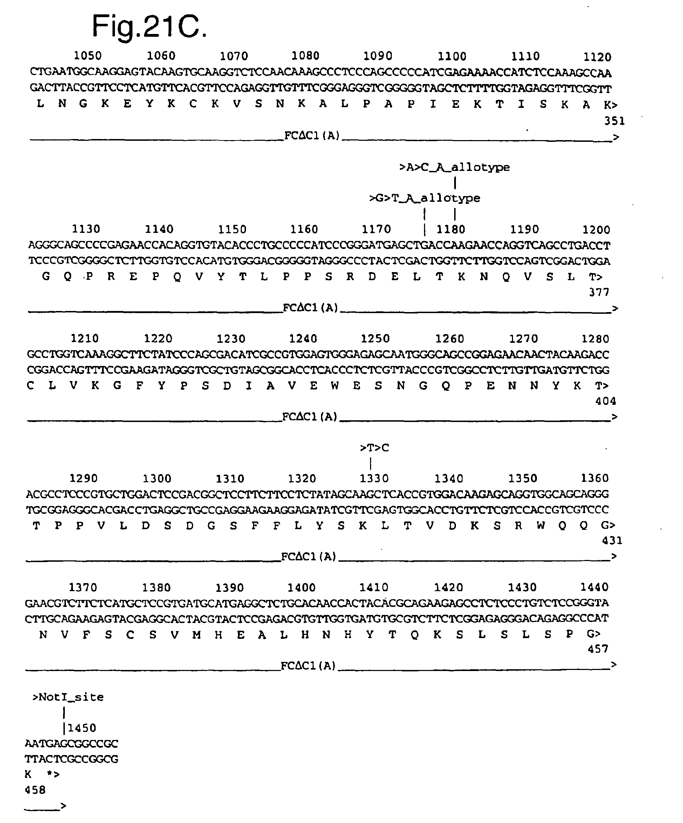

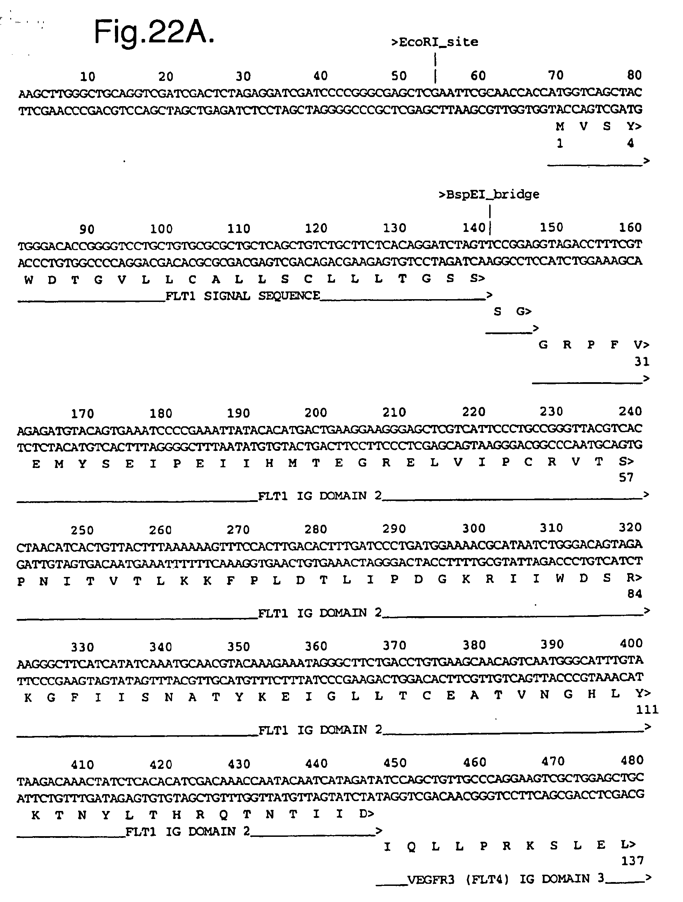

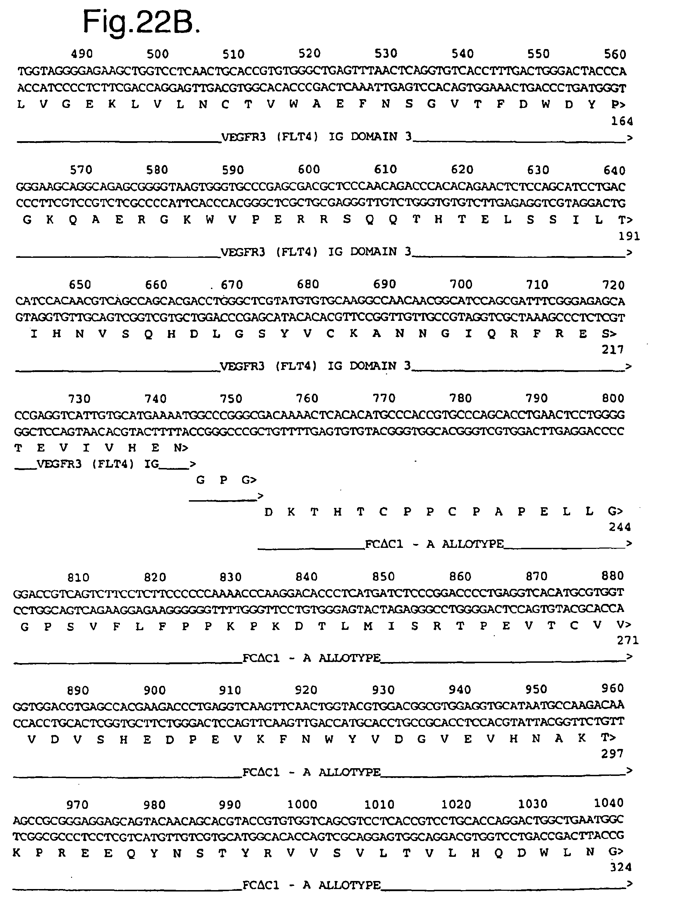

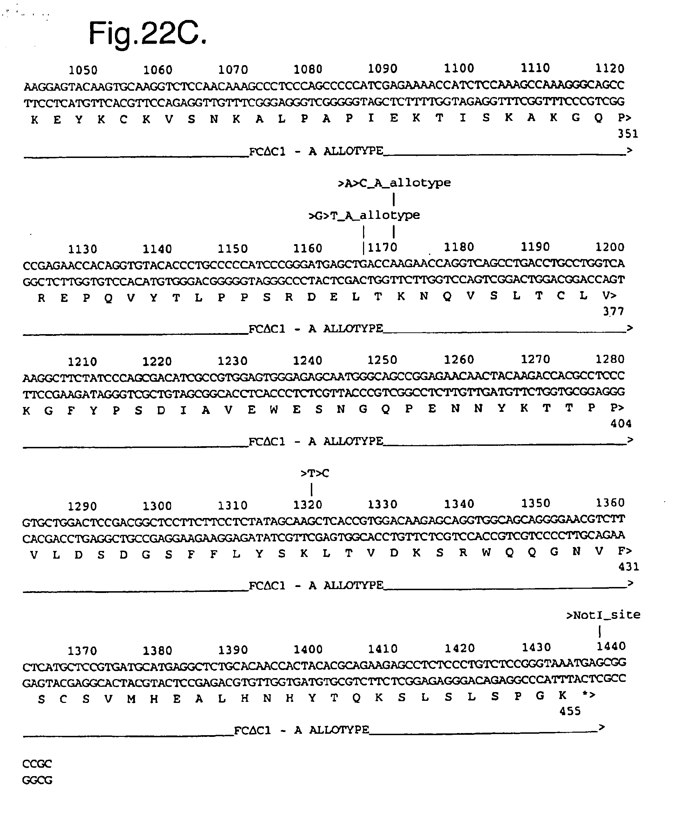

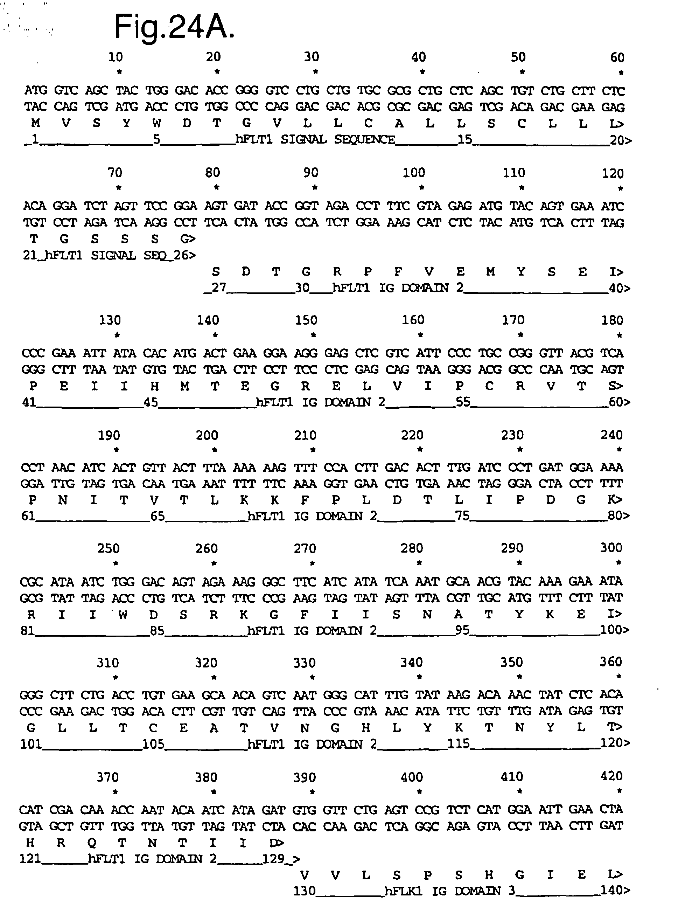

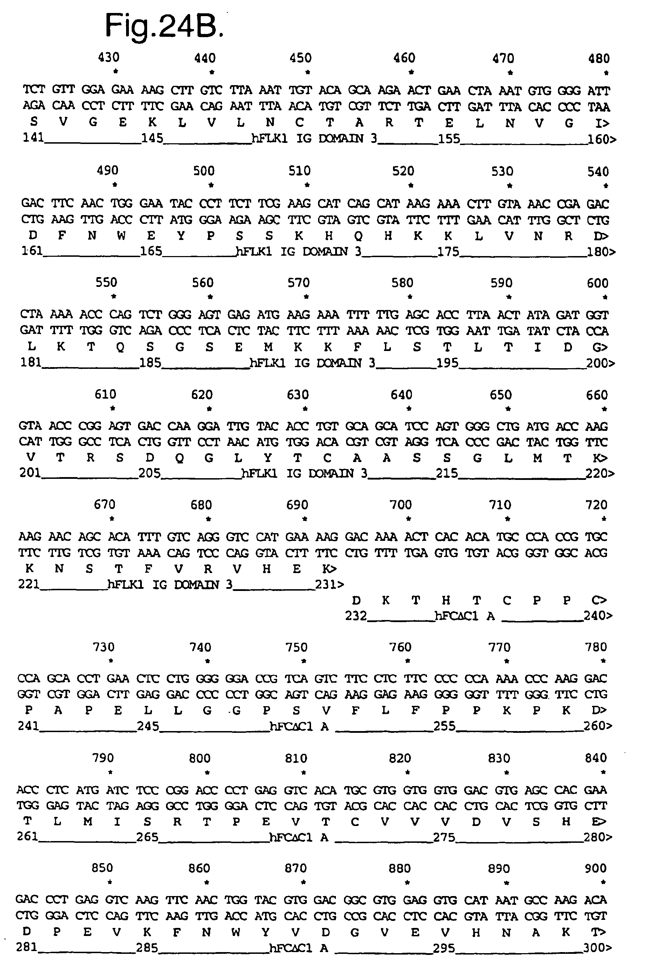

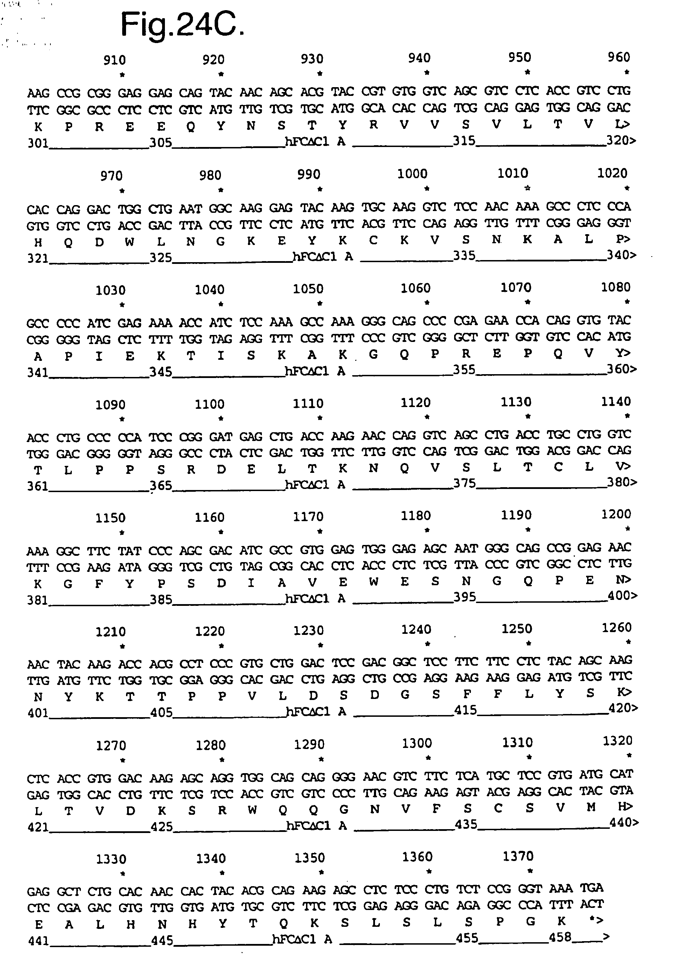

Preferred embodiments include the use of an isolated nucleic acid molecule comprising a nucleotide sequence encoding a modified Flt1 receptor fusion polypeptide, wherein the coding region of the nucleic acid molecule consists of a nucleotide sequence selected from the group consisting of
(a) the nucleotide sequence set forth in Figure 21A-21C;
(b) the nucleotide sequence set forth in Figure 22A-22C;
(c) the nucleotide sequence set forth in Figure 24A-24C; and
(d) a nucleotide sequence which, as a result of the degeneracy of the genetic code, differs from the nucleotide sequence of (a), (b) or (c), and which encodes a fusion polypeptide molecule having the biological activity of the modified Flt1 receptor fusion polypeptide.

In a further embodiment of the invention, a fusion polypeptide for use according to the invention is encoded by the isolated nucleic acid molecules described above. These may be provided within a vector which comprises the nucleic acid molecules described above, including an expression vector comprising the nucleic acid molecules described wherein the nucleic acid molecule is operatively linked to an expression control sequence.

In a preferred embodiment, the invention relates to the use of a composition comprising a multimer of the fusion polypeptide. In such a composition, the multimer may be a dimer. The composition may be in a carrier.

For the production of fusion polypeptides of the invention, a host-vector system which comprises the expression vector, in a suitable host cell, may be used, for example a host-vector system wherein the suitable host cell is a bacterial cell, yeast cell, insect cell, or mammalian cell; the host-vector system wherein the suitable host cell is E. Coli; the host-vector system wherein the suitable host cell is a COS cell; the host-vector system wherein the suitable host cell is a CHO cell.

Fusion polypeptides for use according to may be produced by a method which comprises growing cells of the host-vector system under conditions permitting production of the fusion polypeptide and recovering the fusion polypeptide so produced.

Fusion polypeptides for use according to the invention may have been modified by acetylation or pegylation wherein the acetylation is accomplished with at least about a 100 fold molar excess of acetylation reagent or wherein acetylation is accomplished with a molar excess of acetylation reagent ranging from at least about a 10 fold molar excess to about a 100 fold molar excess or wherein the pegylation is 10K or 20K PEG.

Preferred embodiments of the invention relate to the treatment of an eye disorder in a human, wherein the eye disorder is characterized by vascular permeability.

Preferred embodiments include the use of a fusion polypeptide wherein amino acid sequence of lg domain 2 of the extracellular domain of the first VEGF receptor is upstream of the amino acid sequence of lg domain 3 of the extracellular domain of the second VEGF receptor and a fusion polypeptide wherein the amino acid sequence of lg domain 2 of the extracellular domain of the first VEGF receptor is downstream of the amino acid sequence of Ig domain 3 of the extracellular domain of the second VEGF receptor.

In yet another embodiment, the fusion polypeptide multimerizing component comprises an immunoglobulin domain including an embodiment wherein the immunoglobulin domain is selected from the group consisting of the Fc domain of IgG, the heavy chain of IgG, and the light chain of IgG.

### BRIEF DESCRIPTION OF THE FIGURES

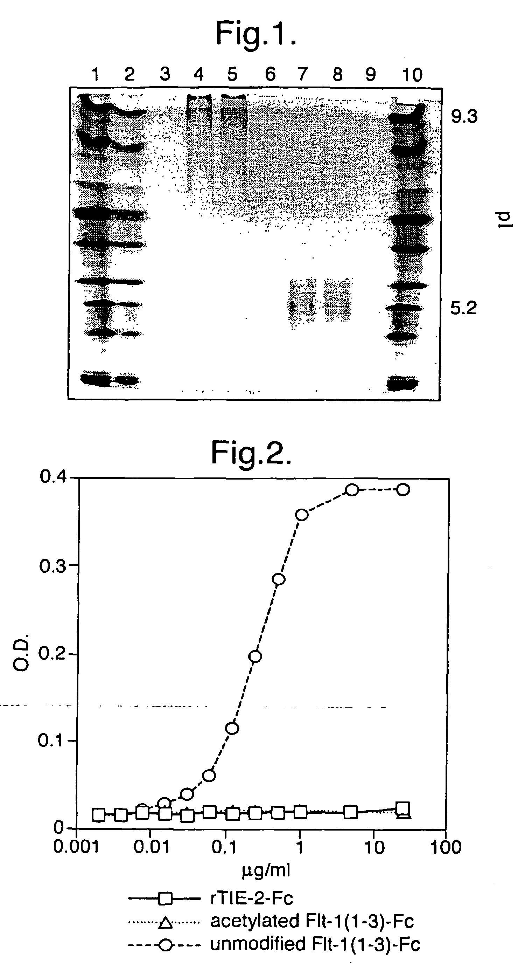

**Figure 1****.** IEF gel analysis of unmodified and acetylated Flt1(1-3)-Fc proteins. Unmodified Flt1(1-3)-Fc protein is unable to enter the gel due to its >9.3 pl, whereas acetylated Flt1(1-3)-Fc is able to enter the gel and equilibrate at pl 5.2.
**Figure 2****.** Binding of unmodified Flt1(1-3)-Fc and acetylated Flt1(1-3)-Fc proteins to Matrigel® coated plates. Unmodified Flt1(1-3)-Fc proteins binds extensive to extracellular matrix components in Matrigel®, whereas acetylated Flt1(1-3)-Fc does not bind.

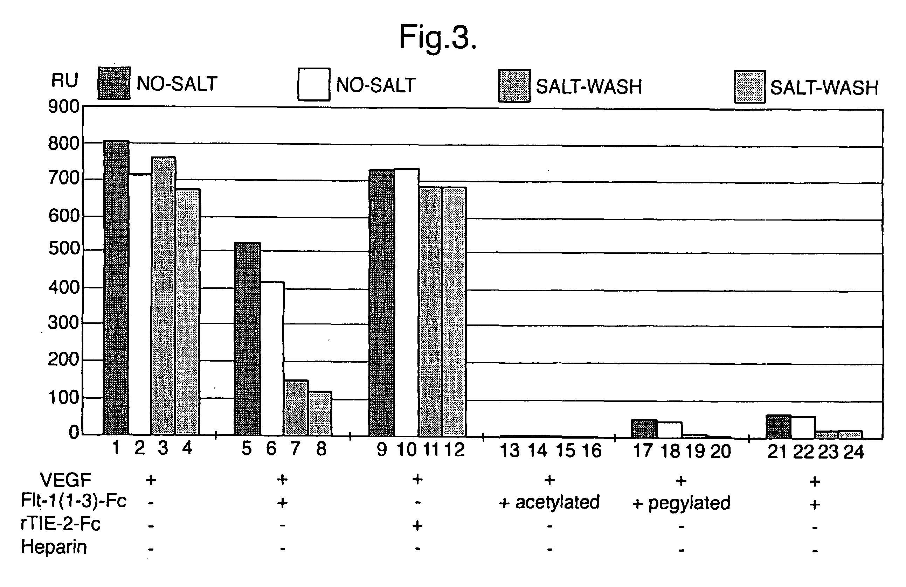

**Figure 3****.** Binding of unmodified Flt1(1-3)-Fc, acetylated Flt1(1-3)-Fc, and pegylated Flt1(1-3)-Fc in a Biacore-based assay. Acetylated (columns 13-16), pegylated (columns 17-20), and heparin-treated Flt1(1-3)-Fc (columns 21-24) are each able to completely compete with the Biacore chip-bound Flt1(1-3)-Fc for VEGF binding as compared to control (columns 1-4) and irrelevant protein (columns 5-8). Unmodified Flt1(1-3)-Fc (columns 5-6) appears to only partially compete with Biacore chip-bound Flt1(1-3)-Fc for VEGF binding. However, washing the bound samples with 0.5M NaCl (columns 7-8) results in a binding profile similar to the modified forms of Flt1(1-3)-Fc, indicating that the unmodified protein is exhibiting non-specific binding to the chip that can be eliminated by the salt wash.

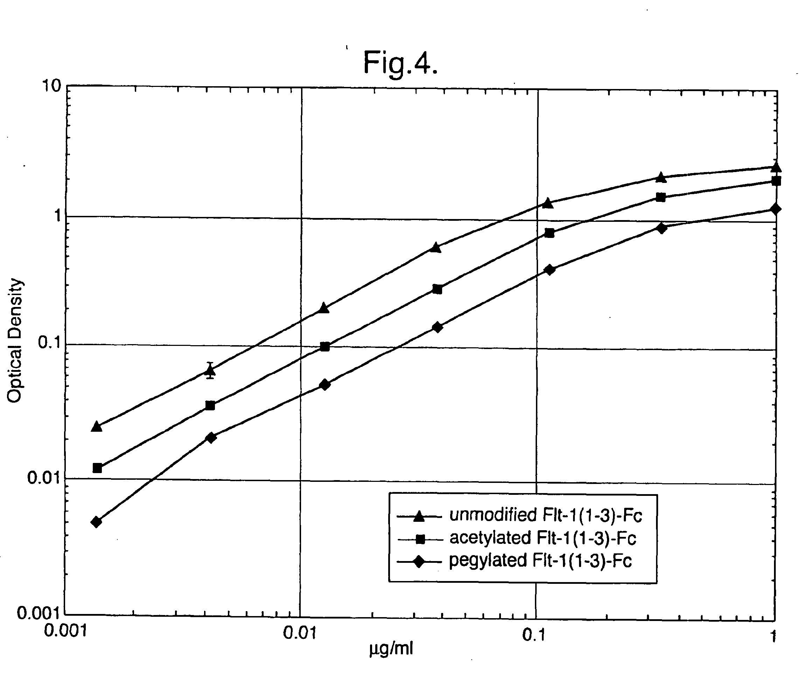

**Figure 4****.** Binding of unmodified Flt1(1-3)-Fc, acetylated Flt1(1-3)-Fc, and pegylated Flt1(1-3)-Fc to VEGF in an ELISA-based assay. Both pegylated and acetylated Flt1(1-3)-Fc proteins bind to VEGF with affinities approaching that of unmodified Flt1(1-3)-Fc.

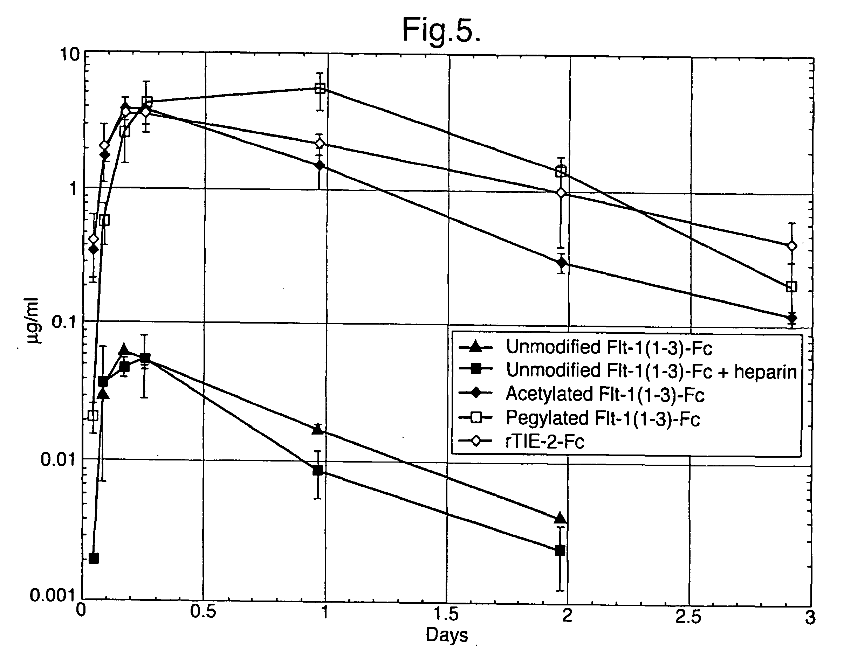

**Figure 5****.** Pharmacokinetic profiles of unmodified Flt1(1-3)-Fc, acetylated Flt1(1-3)-Fc, and pegylated Flt1(1-3)-Fc. Balb/c mice (23-28g) were injected subcutaneously with 4mg/kg of unmodified, acetylated, or pegylated Flt1(1-3)-Fc. The mice were tail bled at 1, 2, 4, 6, 24 hours, 2 days, and 3 days after injection of protein and the sera were assayed in a standard ELISA-based assay designed to detect Flt1(1-3)-Fc protein. The Tₘₐₓ for all of the Flt1(1-3)-Fc proteins was between the 6 hour and 24 hour time points. The Cₘₐₓ for the different proteins was as follows: Unmodified: 0.06 µg/ml - 0.15 µg/ml; acetylated: 1.5 µg/ml - 4.0 µg/ml; and pegylated: approximately 5 µg/ml.

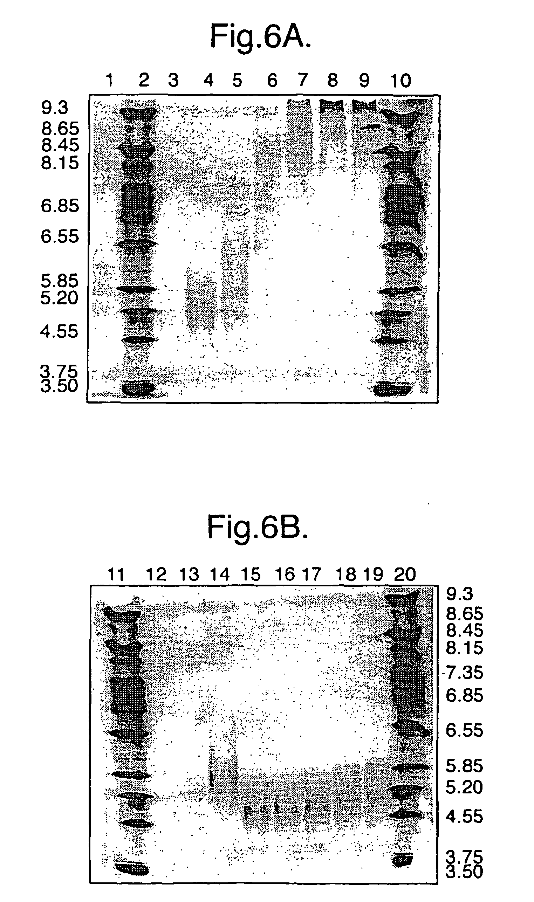

**Figure 6A-6B****.** IEF gel analysis of unmodified and step-acetylated Flt1(1-3)-Fc proteins. Unmodified Flt1(1-3)-Fc protein is unable to enter the gel due to its >9.3 pl, whereas most of the step-acetylated Flt1(1-3)-Fc samples (30-100 fold excess samples) were able to migrate into the gel and equilibrate at pls ranging between 4.55 - 8.43, depending on the degree of acetylation.

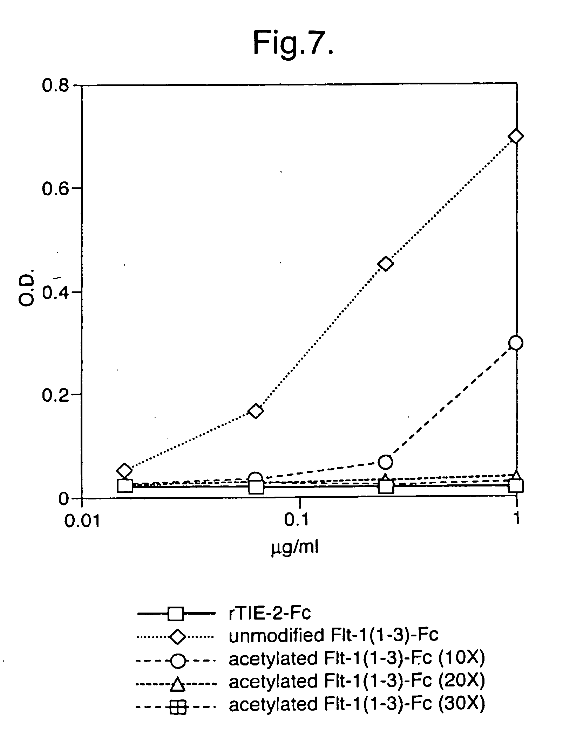

**Figure 7****.** Binding of unmodified Flt1(1-3)-Fc and step-acetylated Flt1(1-3)-Fc proteins to Matrigel® coated plates. As with the irrelevant control protein, rTie2-Fc, step-acetylated Flt1(1-3)-Fc (20 and 30 fold excess samples) does not exhibit any binding to the Matrigel coated plate, whereas the non-acetylated Flt1(1-3)-Fc protein exhibits significant binding. The 10 fold excess sample shows reduced binding, but the degree of acetylation is not enough to completely block binding to extracellular matrix components.

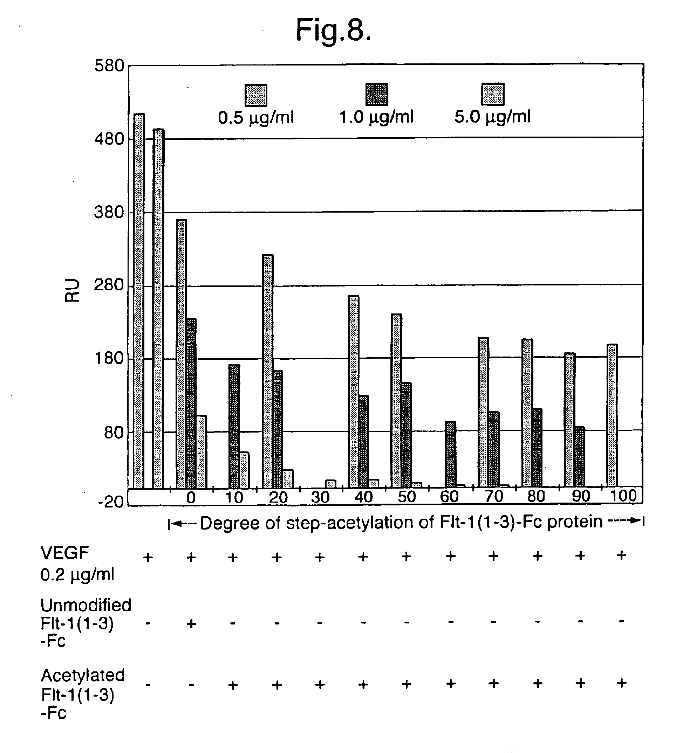

**Figure 8****.** Binding of unmodified Flt1(1-3)-Fc and step-acetylated Flt1(1-3)-Fc in a Biacore-based assay. At a sub-stoichiometric ratio (0.5 µg/ml of either unmodified Flt1(1-3) or step-acetylated Flt1(1-3)-Fc vs. 0.2 µg/ml VEGF), there is not enough Flt1(1-3)-Fc (either unmodified or step-acetylated) in the solution to completely bind the VEGF. At 1.0 µg/ml, which approximates a 1:1 stoichiometric ratio, the both unmodified and step-acetylated Flt1(1-3)-Fc are better able to compete for VEGF binding, but there is still insufficient Flt1(1-3)-Fc protein (either unmodified or step-acetylated) to completely saturate the available VEGF. However, at 5.0 µg/ml, which is several times greater than a 1:1 stoichiometric ratio, both the Flt1(1-3)-Fc and the step-acetylated Flt1(1-3)-Fc proteins are able to saturate the VEGF, regardless of the degree of acetylation.

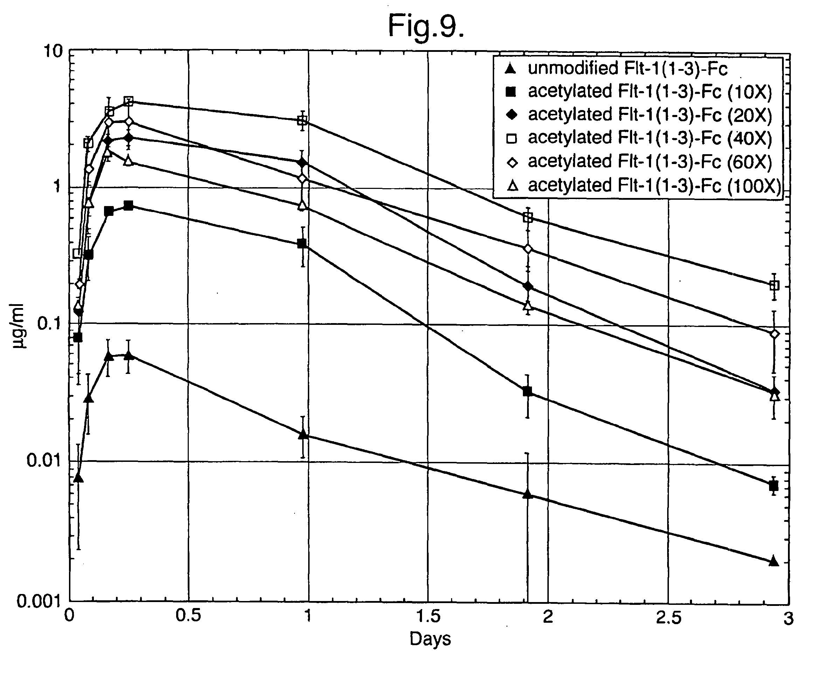

**Figure 9****.** Pharmacokinetic profiles of unmodified Flt1(1-3)-Fc and step-acetylated Flt1(1-3)-Fc. Balb/c mice (23-28g) were injected subcutaneously with 4mg/kg of unmodified or 10, 20, 40, 60 and 100 fold excess samples of step-acetylated Flt1(1-3)-Fc (3 mice for unmodified, 10, 20 and 40 fold excess samples and 2 mice for 60 and 100 fold excess samples). The mice were tail bled at 1, 2, 4, 6, 24 hours, 2 days and 3 days after injection. The sera were assayed in an ELISA-based assay designed to detect Flt1(1-3)-Fc. The Tₘₐₓ for all of the Flt1(1-3)-Fc proteins tested was at the 6 hour time point but the Cₘₐₓ was as follows: Unmodified Flt1(1-3)-Fc: 0.06µg/ml; 10 fold excess sample: - 0.7µg/ml, 20 fold excess sample - 2µg/ml, 40 fold excess sample - 4µg/ml, 60 fold excess sample - 2µg/ml, 100 fold excess sample - 1µg/ml.

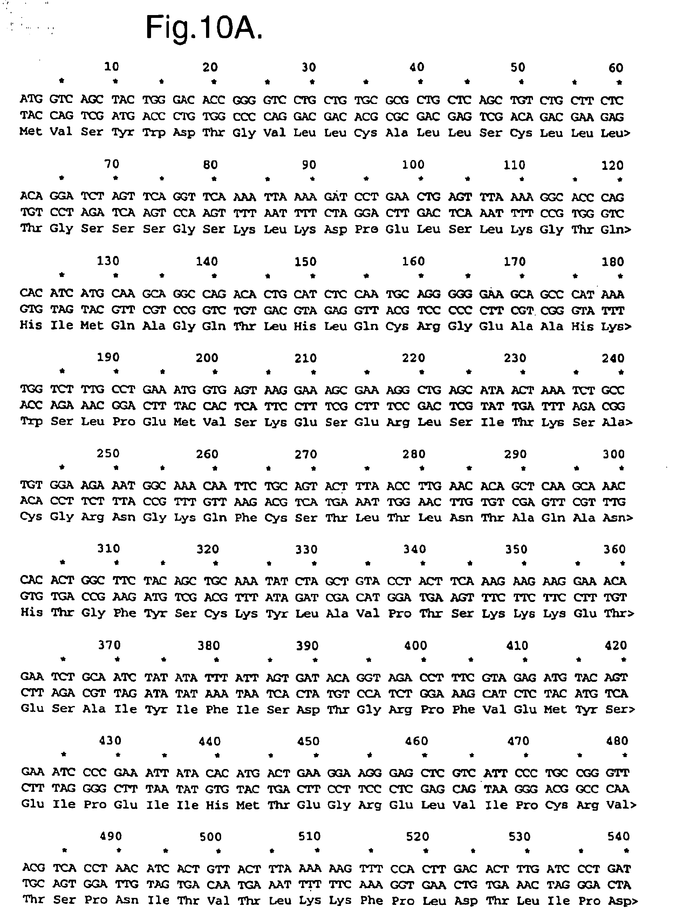

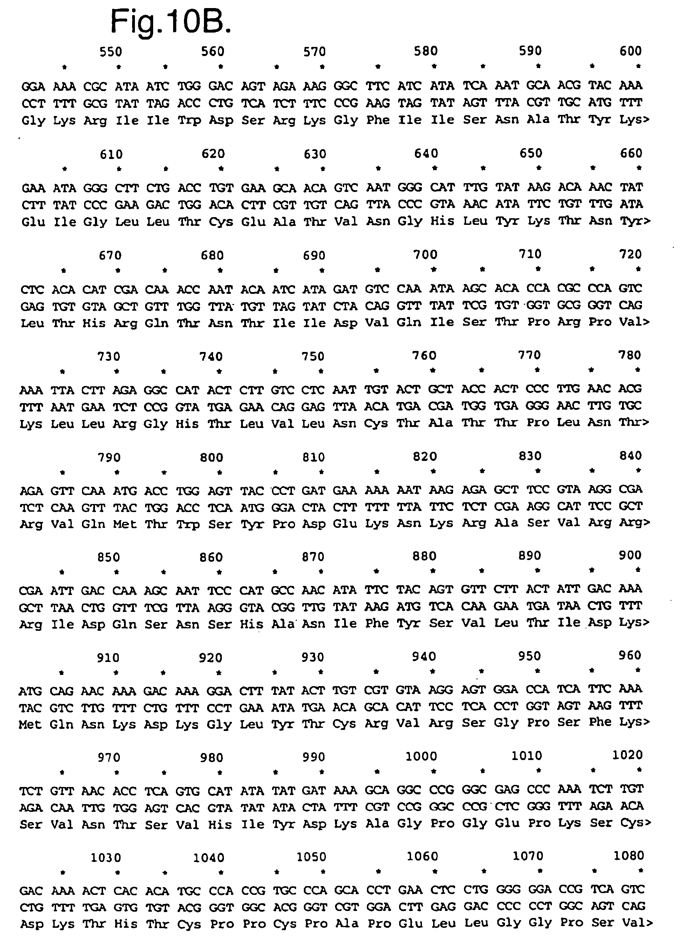

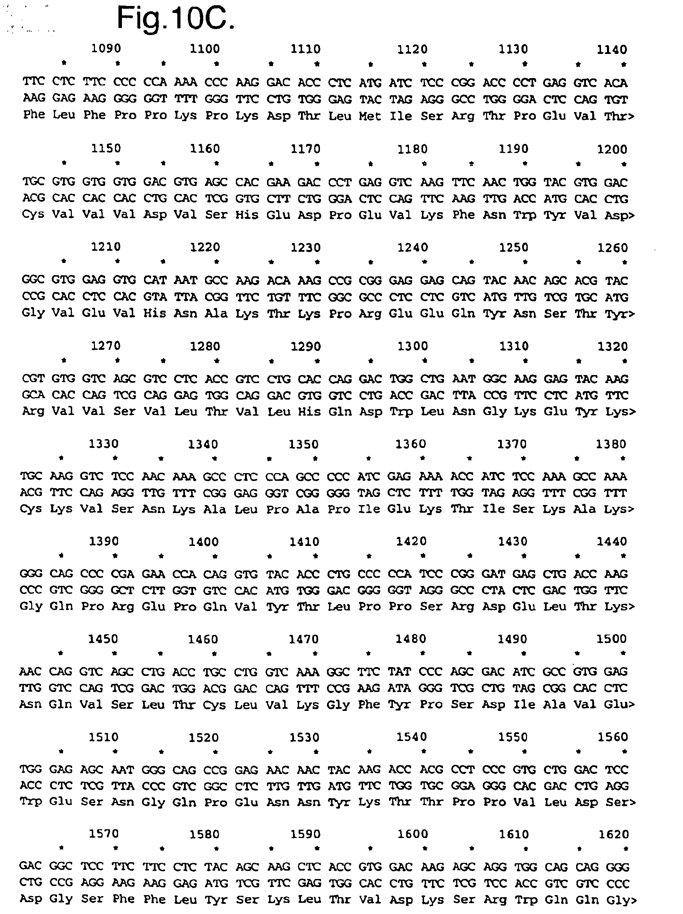

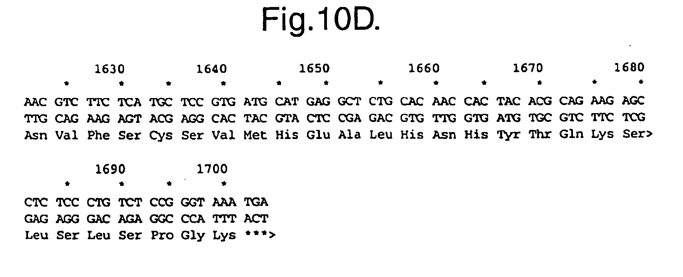

**Figure 10A-10D****.** Nucleic acid and deduced amino acid sequence of Flt1(1-3)-Fc.

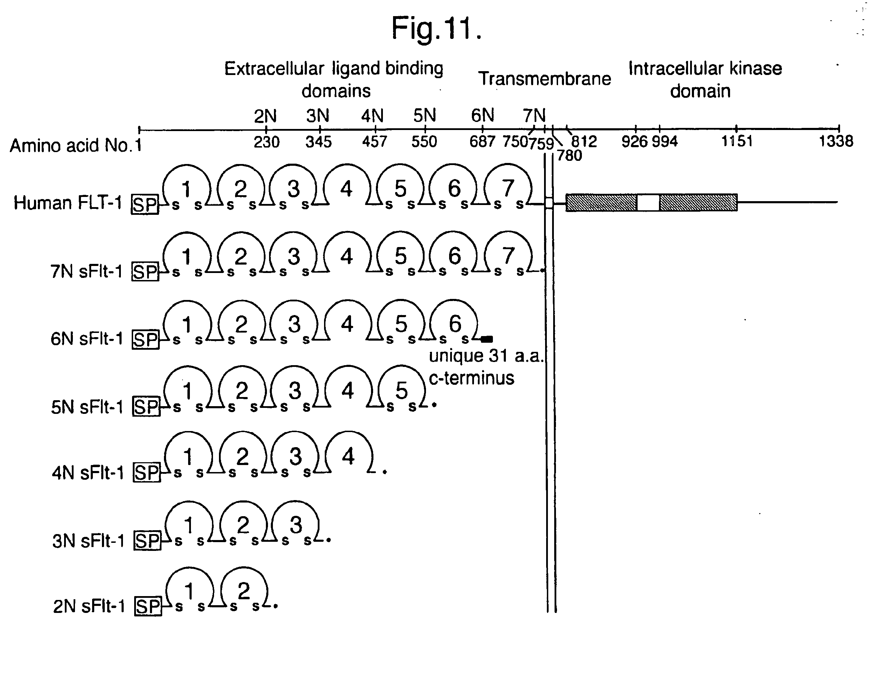

**Figure 11****.** Schematic diagram of the structure of Flt1.

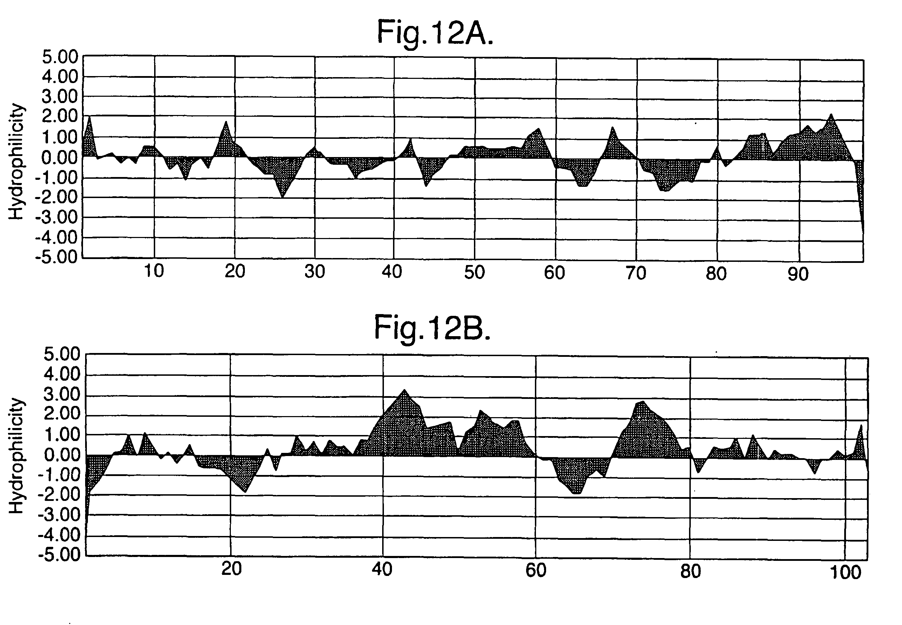

**Figure 12A and 12B****.** Hydrophilicity analysis of the amino acid sequences of Ig domain 2 and Ig domain 3 of Flt1.

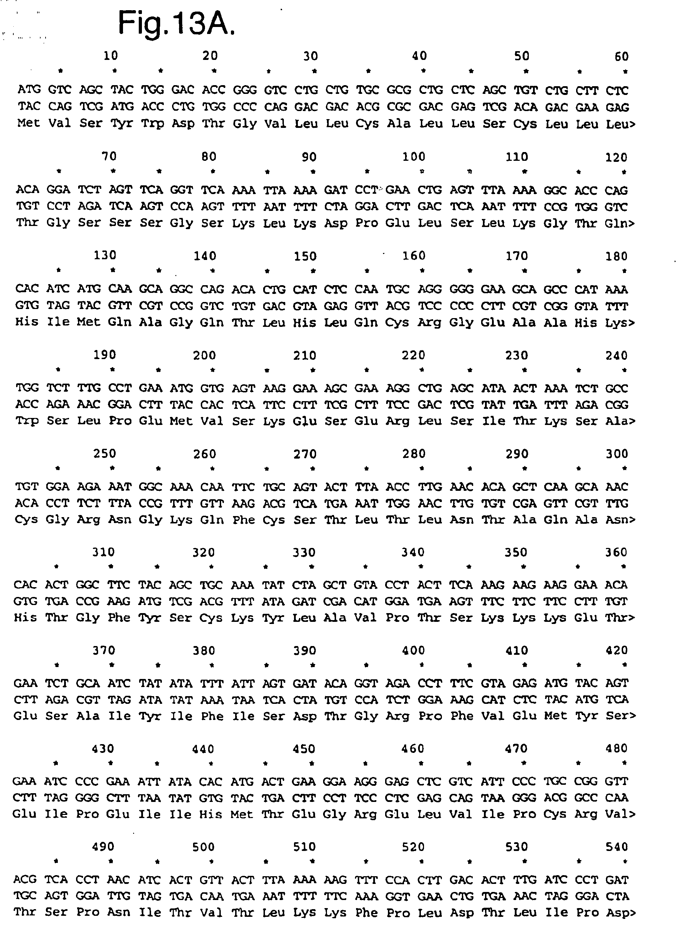

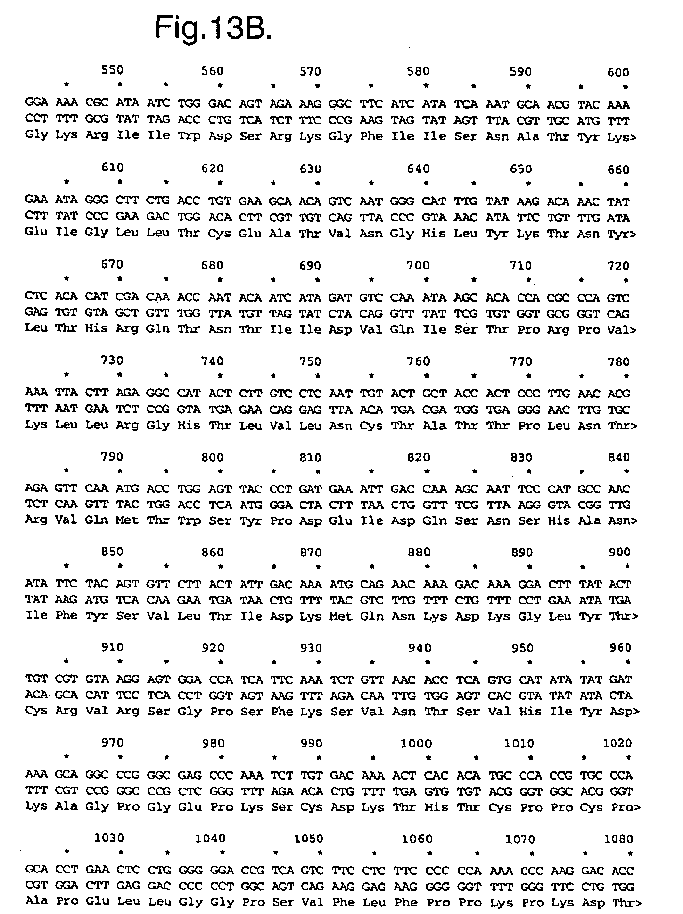

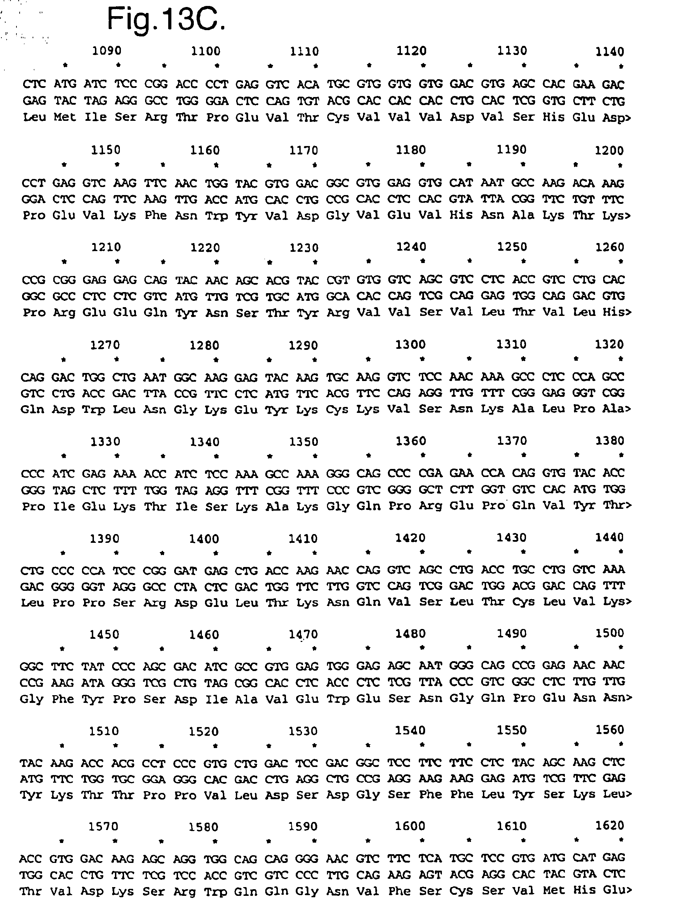

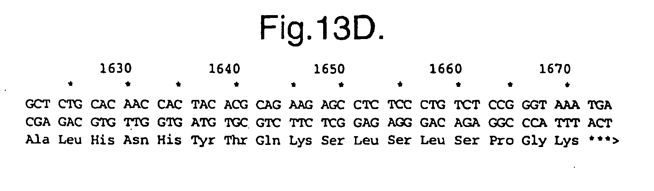

**Figure 13A-13D****.** Nucleic acid and deduced amino acid sequence of Mut1: Flt1(1-3_{ΔB})-Fc.

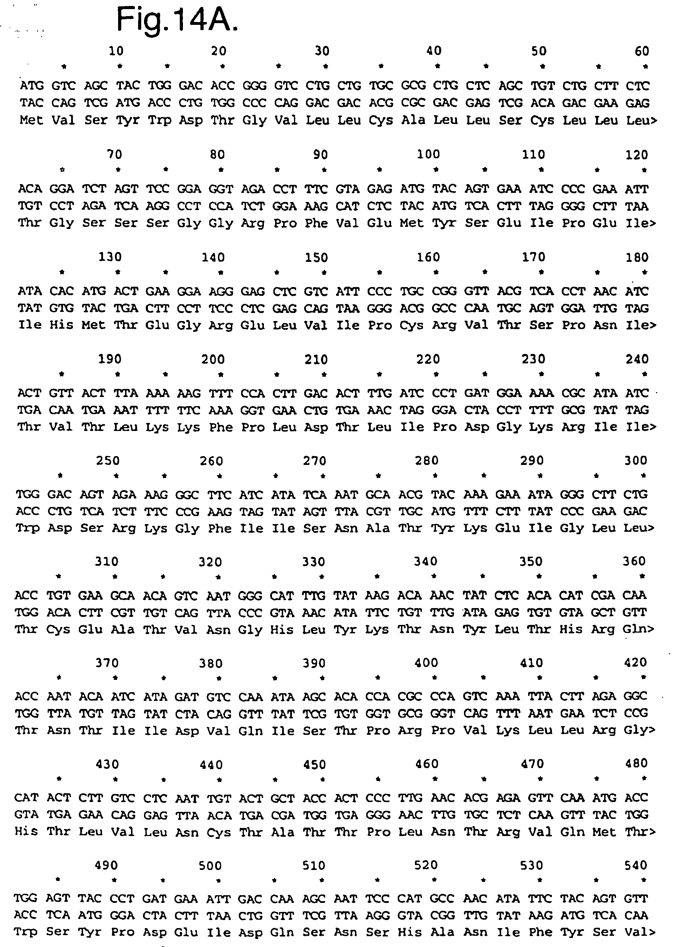

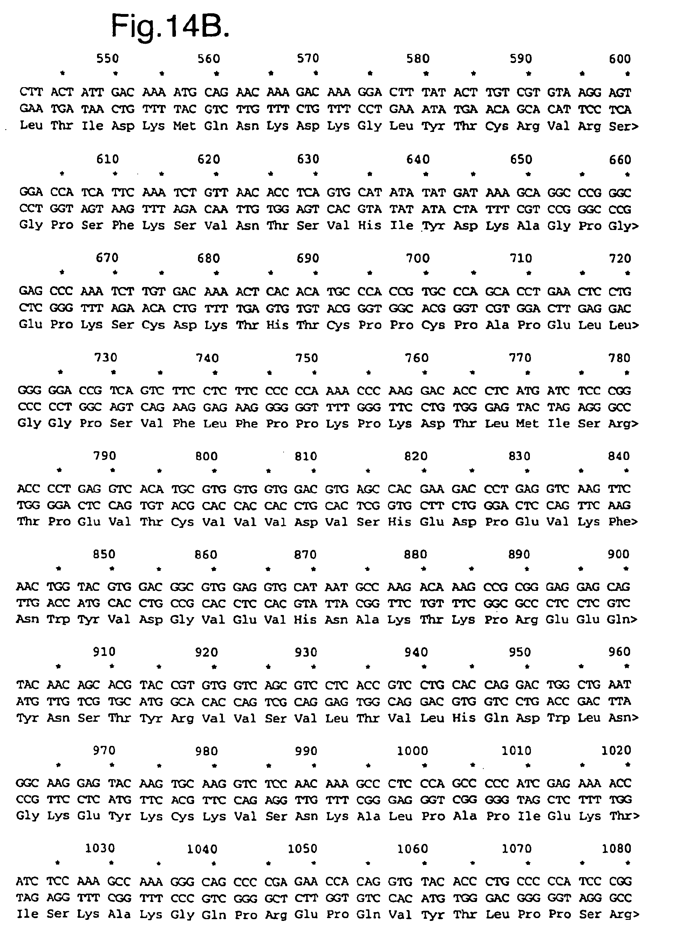

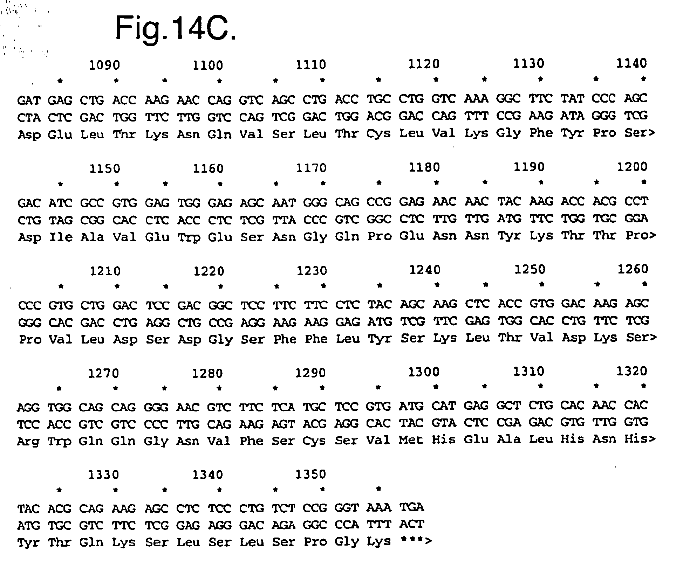

**Figure 14A-14** **C.** Nucleic acid and deduced amino acid sequence of Mut2: Flt1(2-3_{ΔB})-Fc.

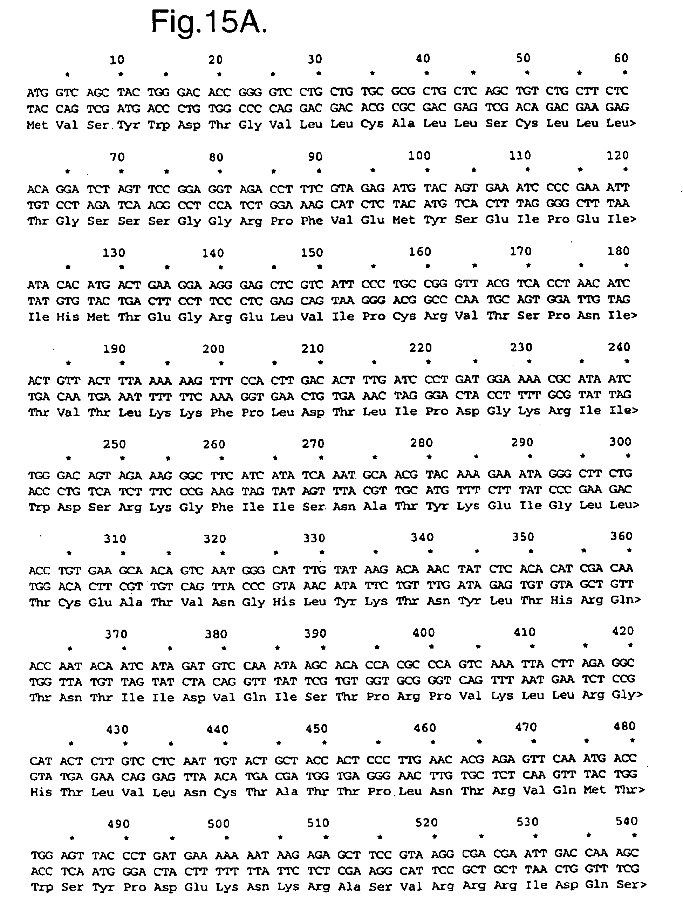

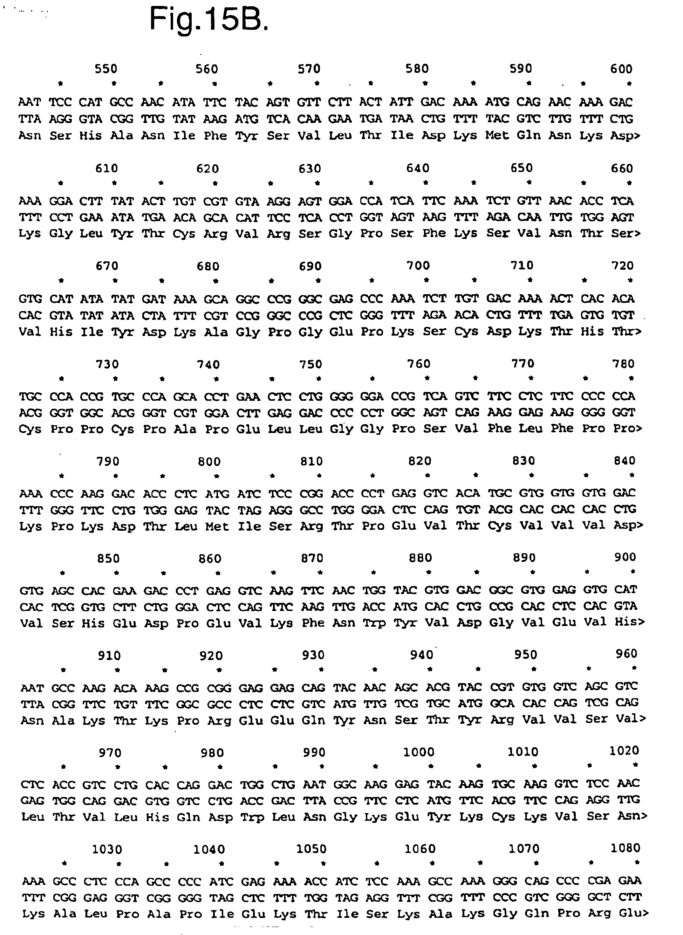

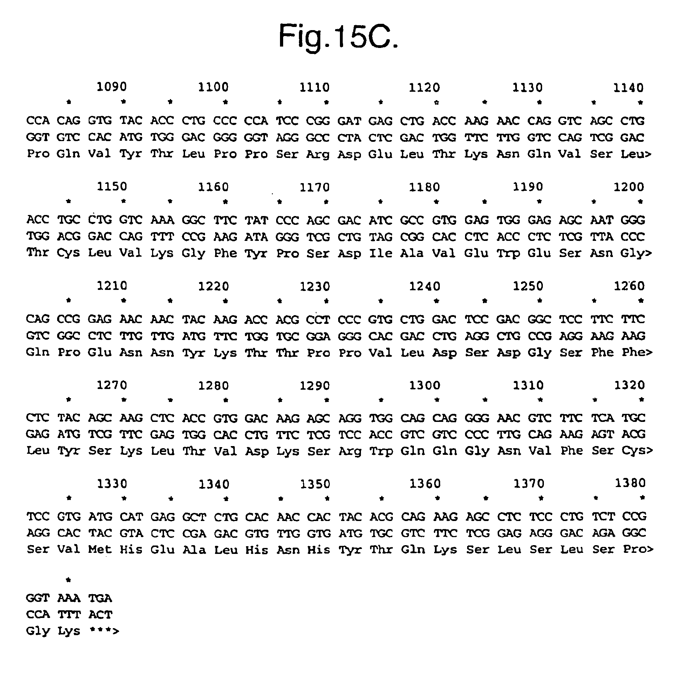

**Figure 15A-15C****.** Nucleic acid and deduced amino acid sequence of Mut3: Flt1(2-3)-Fc.

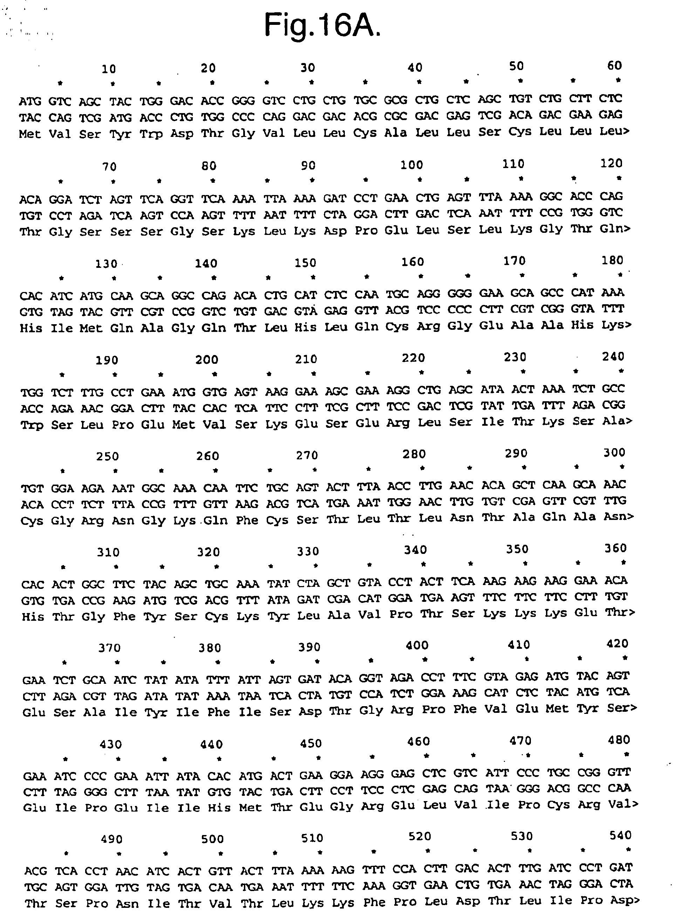

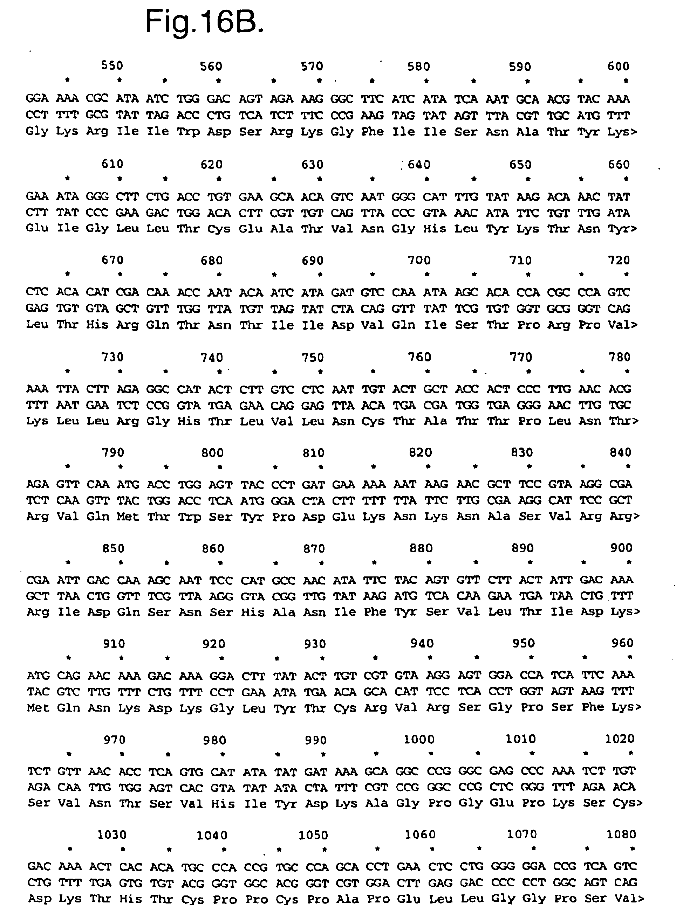

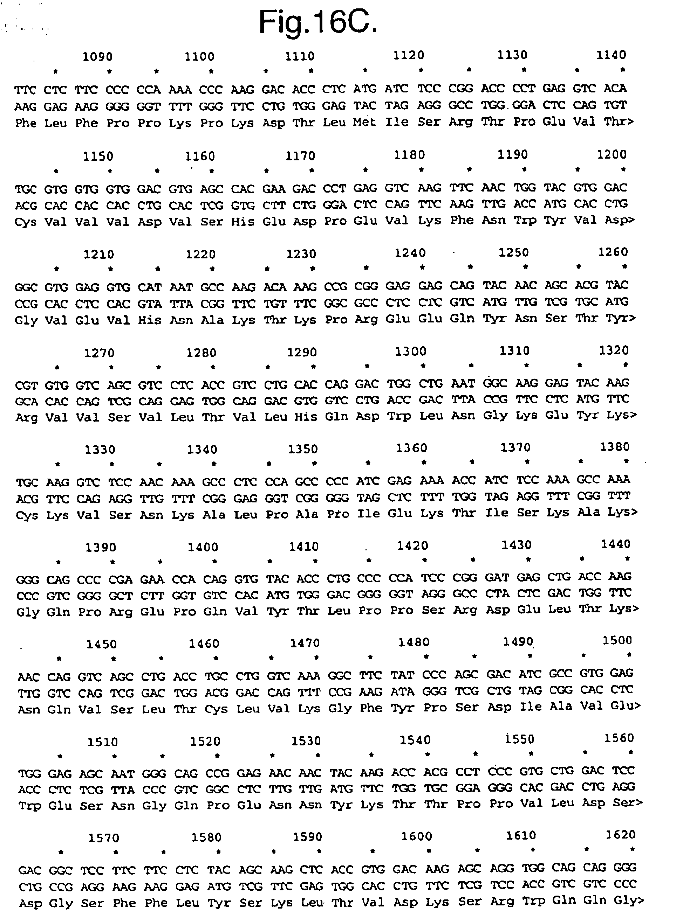

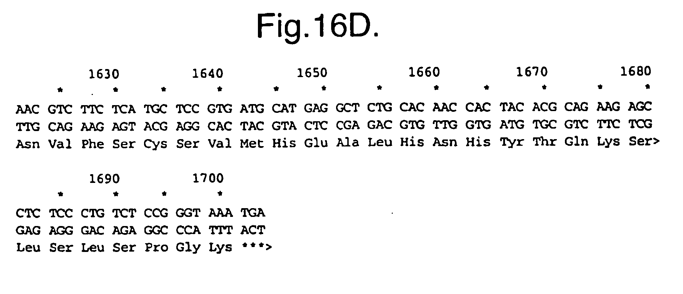

**Figure 16A-16D****.** Nucleic acid and deduced amino acid sequence of Mut4: Flt1(1-3_{R->N})-Fc.

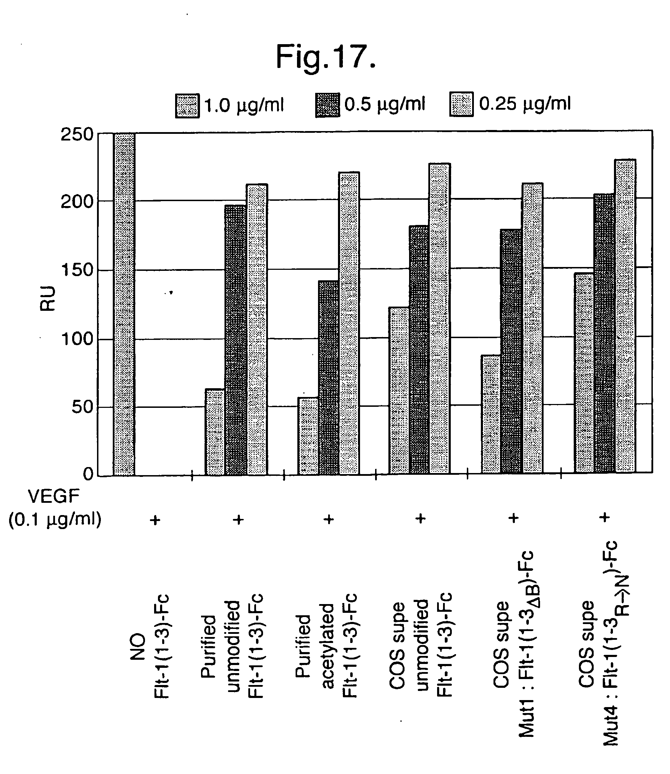

**Figure 17****.** Binding of unmodified Flt1(1-3)-Fc, basic region deletion mutant Flt1(1-3)-Fc, and Flt1(1-3)_{R->N} mutant proteins in a Biacore-based assay. At the sub-stoichiometric ratio (0.25 µg/ml Flt1(1-3)-Fc of unmodified, acetylated or genetically modified samples vs. 01. µg/ml VEGF), there is insufficient Flt1(1-3)-Fc protein to block binding of VEGF to the Flt1(1-3)-Fc immobilized on the Biacore chip. At 0.5 µg/ml of unmodified, acetylated or genetically modified Flt1(1-3)-Fc proteins, the stoichiometric ratio approximates 1:1 and there is an increased ability to block VEGF binding to the Biacore chip. At 1.0 µg/ml of unmodified, acetylated or genetically modified Flt1(1-3)-Fc proteins, which is approximately a 10:1 stoichiometric ratio, the Flt1(1-3)-Fc proteins are able to block binding of VEGF to the Biacore chip, but they are not equivalent. Unmodified, acetylated, and Mut1: Flt1(1-3_{ΔB})-Fc are essentially equal in their ability to block VEGF binding, whereas Mut4: Flt1(1-3_{R->N})-Fc is somewhat less efficient at blocking binding

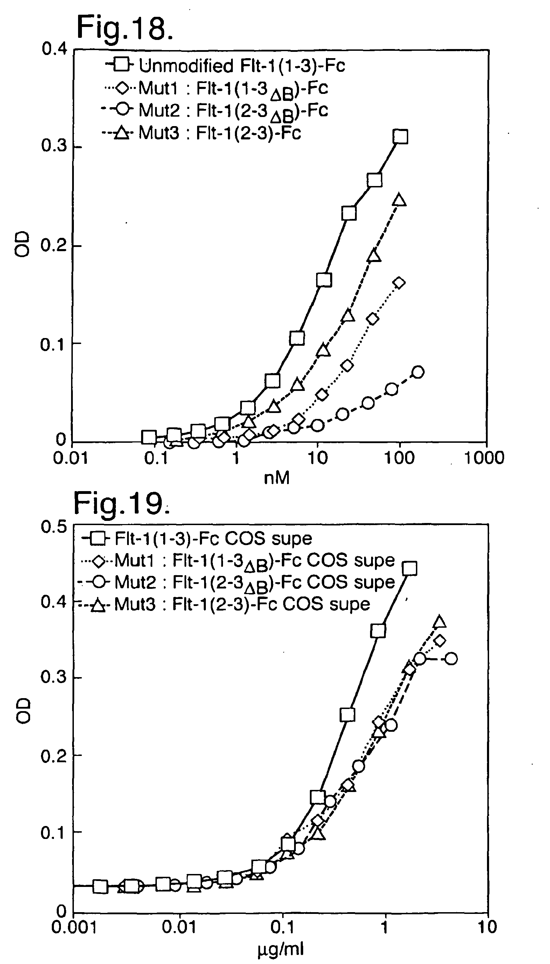

**Figure 18****.** Binding of unmodified Flt1(1-3)-Fc, Mut1: Flt1(1-3_{ΔB})-Fc, Mut2: Flt1(2-3_{ΔB})-Fc, and Flt1(2-3) mutant proteins to Matrigel® coated plates. Unmodified Flt1(1-3)-Fc protein binds avidly to these wells, the Mut3: Flt1(2-3)-Fc protein binds somewhat more weakly, the Mut1: Flt1(1-3_{ΔB})-Fc protein binds more weakly still, and the Mut2: Flt1(2-3_{ΔB})-Fc protein shows the best profile, binding more weakly than any of the other mutant proteins. The Mut4: Flt1(1-3_{R->N})-Fc glycosylation mutant protein shows only marginal benefit on the Matrigel assay.
**Figure 19****.** Binding of unmodified Flt1(1-3)-Fc, Mut1: Flt1(1-3_{ΔB})-Fc, Mut2: Flt1(2-3_{ΔB})-Fc, and Flt1(2-3) mutant proteins in an ELISA-based assay. At the concentrations tested, unmodified Flt1(1-3)-Fc, Mut1: Flt1(1-3_{ΔB})-Fc, Mut2: Flt1(2-3_{ΔB})-Fc, and Flt1(2-3) mutant proteins bind VEGF similarly.

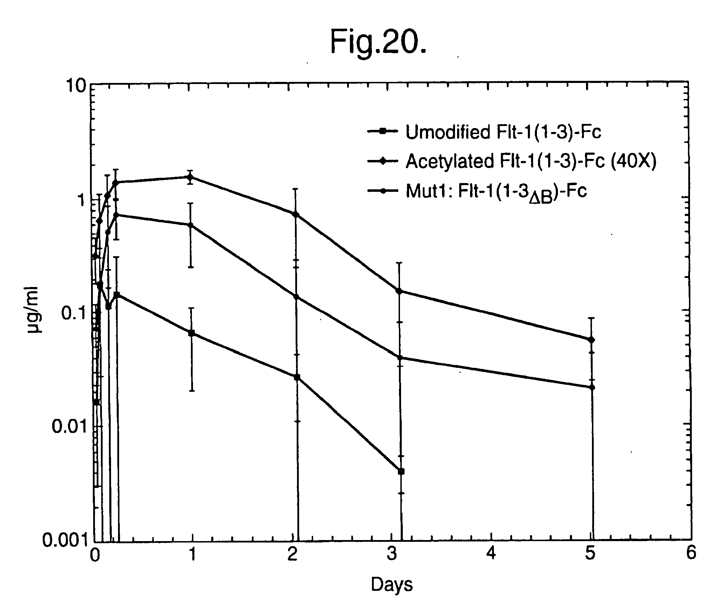

**Figure 20****.** Pharmacokinetic profiles of unmodified Flt1(1-3)-Fc, Mut1: Flt1(1-3_{ΔB})-Fc, Mut2: Flt1(2-3_{ΔB})-Fc, and Flt1(2-3) mutant proteins. the Cmax for these reagents was as follows: Unmodified Flt1(1-3)-Fc- 0.15µg/ml; 40 fold molar excess acetylated Flt1(1-3)-Fc - 1.5µg/ml; and Mut1: Flt1(1-3_{ΔB})-Fc- 0.7µg/ml.
**Figure 21A-21C****.** Nucleotide and deduced amino acid sequence of the modified Flt1 receptor termed Flt1D2.Flk1D3.FcΔC1(a).
**Figure 22A-22C****.** Nucleotide and deduced amino acid sequence of the modified Flt1 receptor termed Flt1D2.VEGFR3D3.FcΔC1(a).

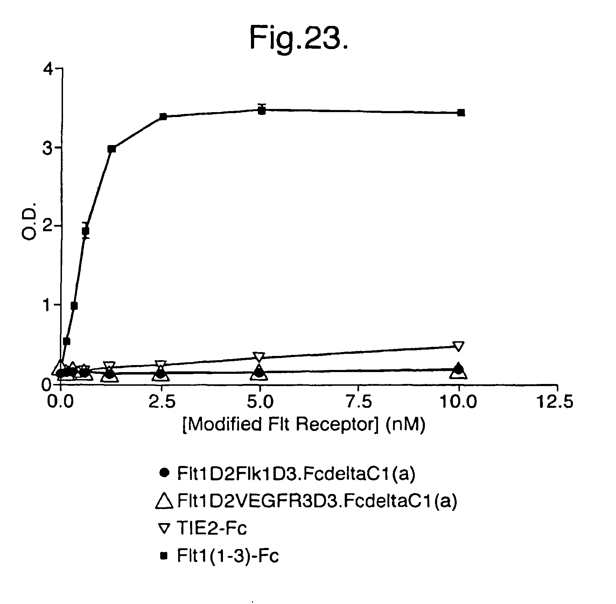

**Figure 23****.** Extracellular Matrix (ECM) Assay. The results of this assay demonstrate that the Flt1D2.Flk1D3.FcΔC1(a) and Flt1D2.VEGFR3D3.FcΔC1 (a) proteins are considerably less sticky to the ECM as compared to the Flt1(1-3)-Fc protein.
**Figure 24A-24C****.** Nucleotide and deduced amino acid sequence of the modified Flt1 receptor termed VEGFR1R2-FcΔC1 (a).

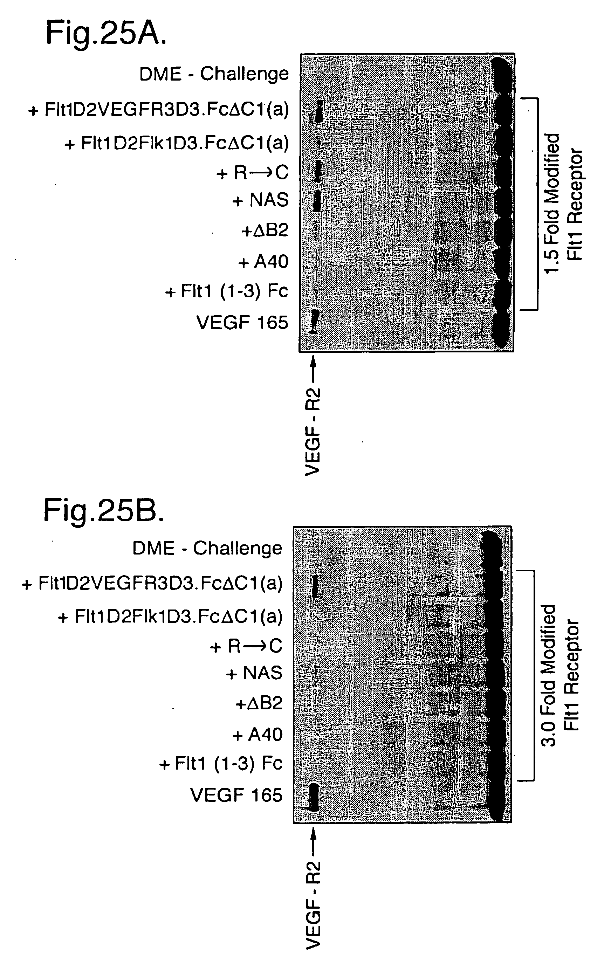

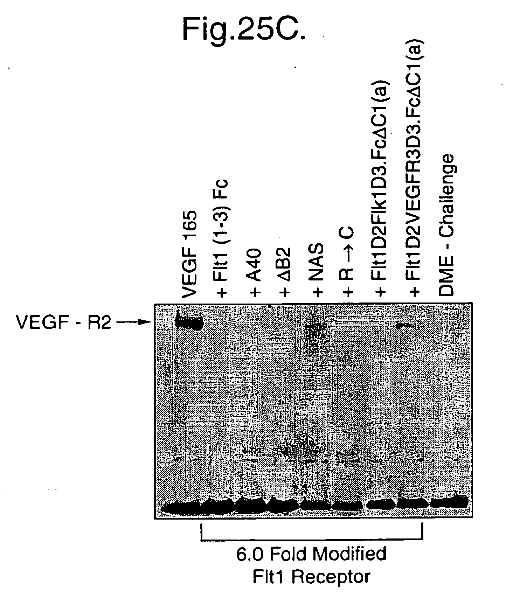

**Figure 25A-25C****.** Phosphorylation assay. At a 1.5 molar excess of either Flt1(1-3)-Fc, Flt1(1-3)-Fc (A40) or transient Flt1D2Flk1D3.FcΔC1(a) there is complete blockage of receptor stimulation by these three modified Flt1 receptors as compared to control media challenge. In contrast, transient Flt1D2VEGFR3D3.FcΔC1(a) does not show significant blockage at this molar excess, as compared with VEGF positive control challenge. Similar results are seen in Figure 25B, where the modified Flt receptors are in a 3-fold molar excess to VEGF165 ligand. In Figure 25C, where the modified Flt1 receptors are in a 6-fold molar excess to VEGF165 ligand, transient Flt1D2VEGFR3D3.FcΔC1(a) can now be shown to be partially blocking VEGF165-induced stimulation of cell-surface receptors.

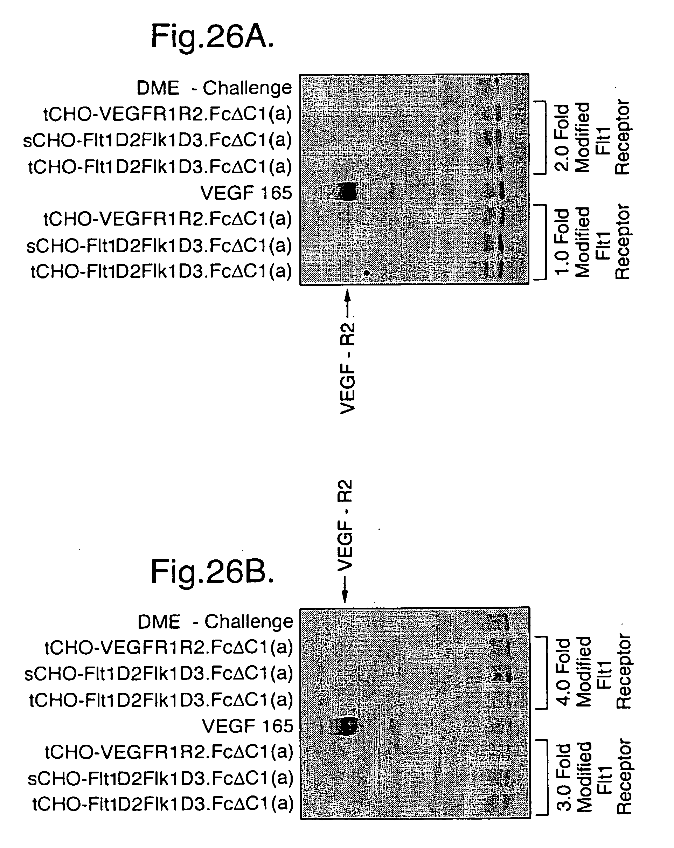

**Figure 26A-26B****.** Phosphorylation assay. Detection by Western blot of tyrosine phosphorylated VEGFR2(Flk1) by VEGF165 ligand stimulation shows that cell-surface receptors are not phosphorylated by challenge samples which have VEGF165 preincubated with 1 and 2 fold molar excess (Figure 26A) or 3 and 4 fold molar excess (Figure 26B) of either transient Flt1D2Flk1D3.FcΔC1(a), stable Flt1D2Flk1D3.FcΔC1(a), or transient VEGFR1R2-FcΔC1(a). At all modified Flt1 receptor concentrations tested there is complete binding of VEGF165 ligand during the preincubation, resulting in no detectable stimulation of cell-surface receptors by unbound VEGF165 as compared to control media challenge.

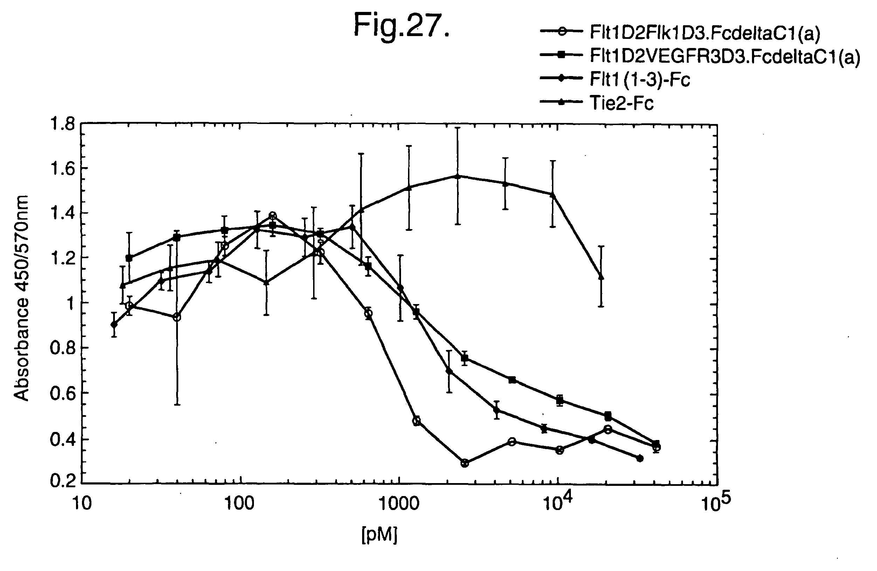

**Figure 27****.** MG/R2 Cell proliferation assay. The following modified Flt receptors Flt1(1-3)-Fc, Flt1D2.Flk1D3.FcΔC1(a) and Flt1D2.VEGFR3D3.FcΔC1(a), plus an irrelevant receptor termed Tie2-Fc as a negative control, were titrated from 40nM to 20pM and incubated on the cells for 1hr at 37°C. Human recombinant VEGF165 in defined media was then added to all the wells at a concentration of 1.56nM. The negative control receptor Tie2-Fc does not block VEGF165-induced cell proliferation at any concentration whereas Flt1D2.Flk1D3.FcΔC1(a) blocks 1.56nM VEGF165 with a half maximal dose of 0.8nM. Flt1(1-3)-Fc and Flt1D2.VEGFR3D3.FcΔC1(a) are less effective in blocking VEGF165 in this assay with a half maximal dose of - 2nM. VEGF165 alone gives a reading of 1.2 absorbance units and the background is 0.38 absorbance units.

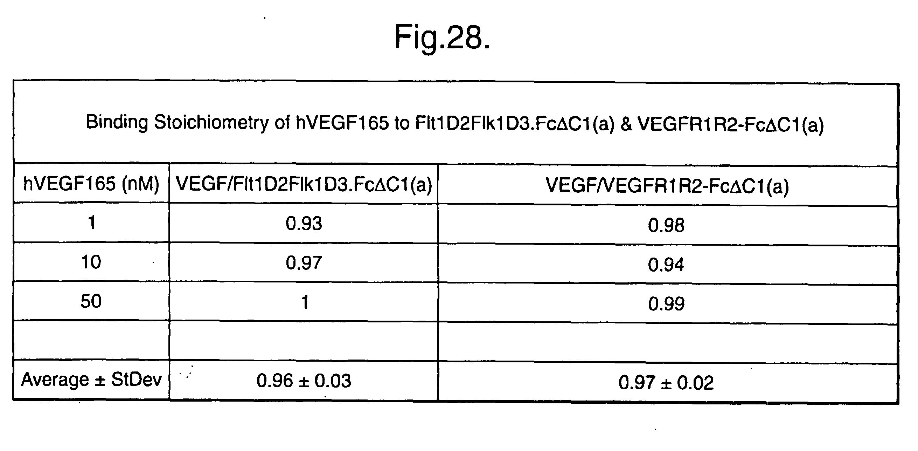

**Figure 28****.** Biacore analysis of Binding Stoichiometry. Binding stoichiometry was calculated as a molar ratio of bound VEGF165 to the immobilized Flt1D2Flk1D3.FcΔC1 (a) or VEGFR1R2-FcΔC1(a), using the conversion factor of 1000 RU equivalent to 1 ng/ml. The results indicated binding stoichiometry of one VEGF165 dimeric molecule per one Flt1D2Flk1D3.FcΔC1(a) or VEGFR1R2-FcΔC1(a) molecule.

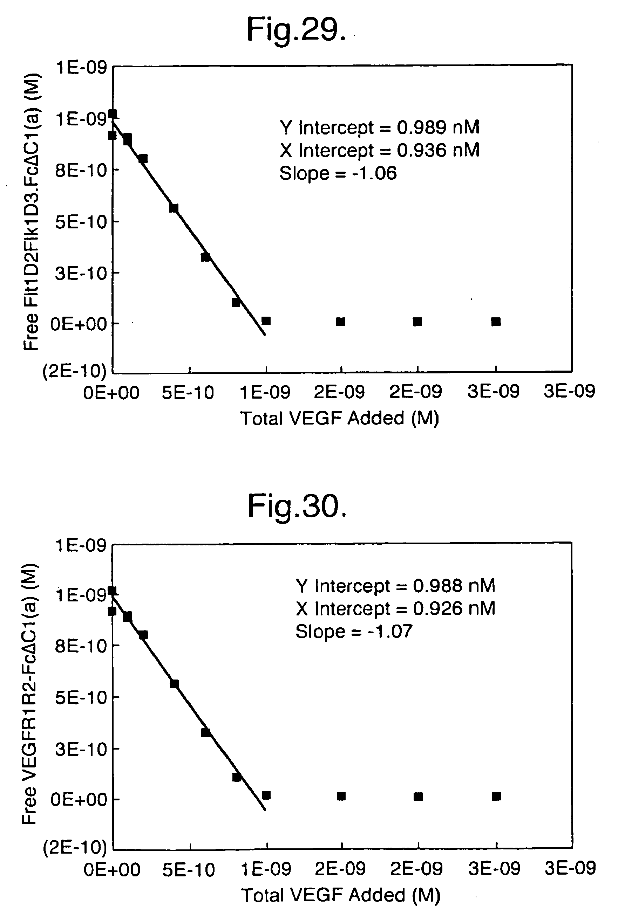

**Figure 29 and Figure 30**. Size Exclusion Chromatography Stoichiometry. Flt1D2Flk1D3.FcΔC1 (a) or VEGFR1R2-FcΔC1(a) at a concentration of 1 nM (estimated to be 1000 times higher than the KD of the Flt1D2Flk1D3.FcΔC1(a) or VEGFR1R2-FcΔC1(a)/VEGF165 interaction) were mixed with varied concentrations of VEGF165. After incubation, concentrations of the free Flt1D2Flk1D3.FcΔC1(a) in solution were measured. The data shows that the addition of 1 nM VEGF165 into the Flt1D2Flk1D3.FcΔC1(a) solution completely blocks Flt1D2Flk1D3.FcΔC1(a) binding to the VEGF165 surface. This result suggested the binding stoichiometry of one VEGF165 molecule per one Flt1D2Flk1D3.FcΔC1(a) molecule.

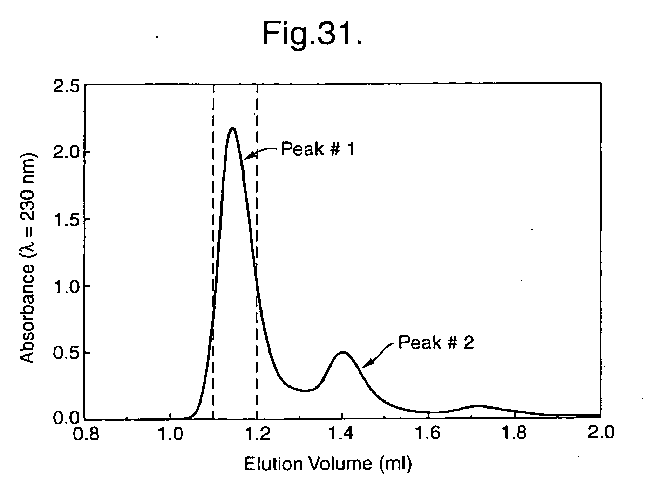

**Figure 31****.** Size Exclusion Chromatography (SEC) under native conditions. Peak #1 represents the Flt1D2Flk1D3.FcΔC1(a)/ VEGF165 complex and peak #2 represents unbound VEGF165. Fractions eluted between 1.1 and 1.2 ml were combined and guanidinium hydrochloride (GuHCl)was added to a final concentration 4.5M to dissociate the complex.

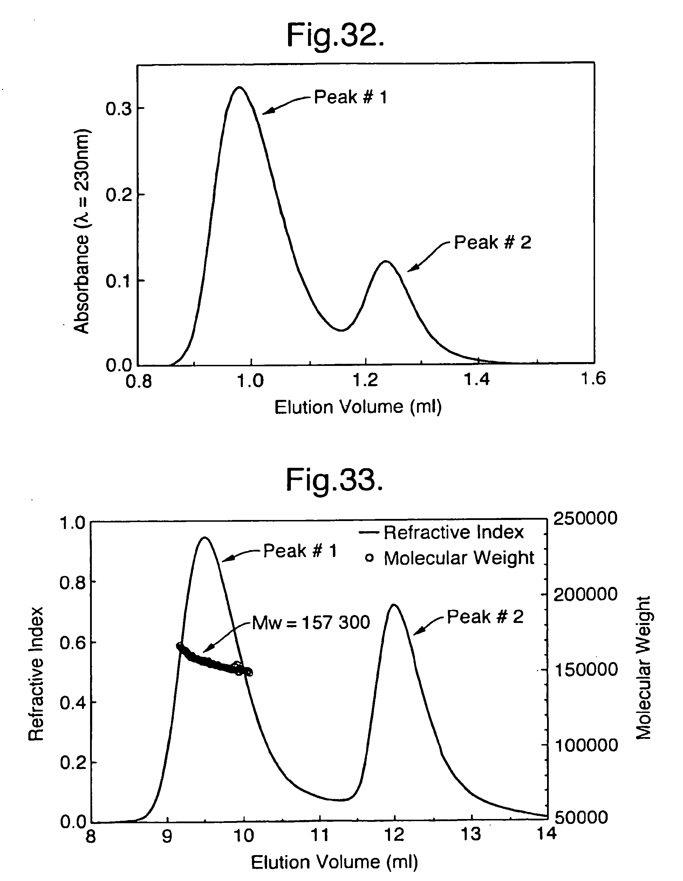

**Figure 32****.** Size Exclusion Chromatography (SEC) under dissociative conditions. To separate the components of the receptor-ligand complex and to determine their molar ratio, 50µl of dissociated complex was loaded onto a Superose 12 PC 3.2/30 equilibrated in 6M GuHCl and eluted. Peak #1 represents Flt1D2Flk1D3.FcΔC1(a) and peak #2 represents VEGF165.

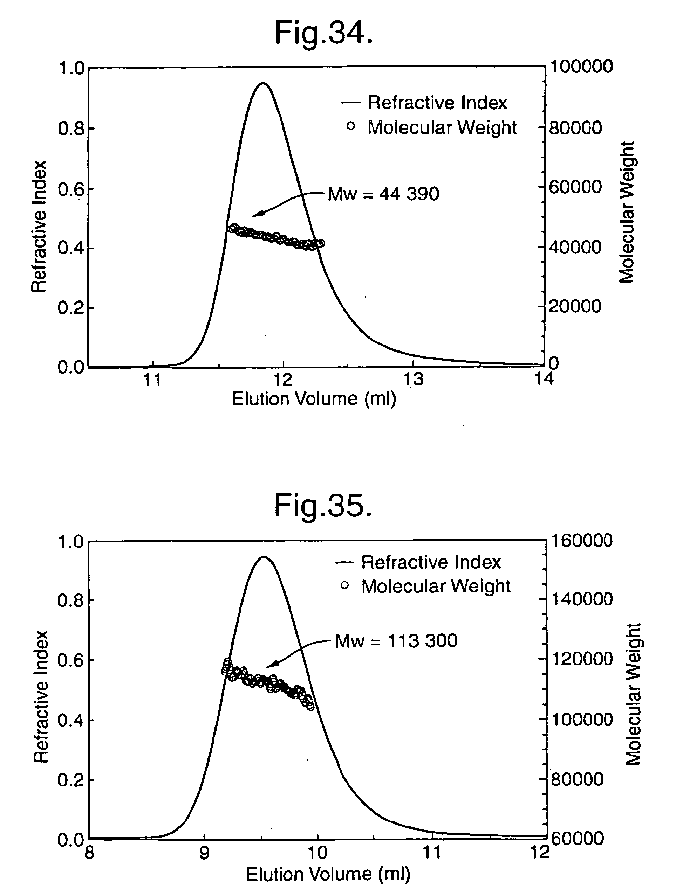

**Figure 33****,** **Figure 34 and Figure 35****.** Size Exclusion Chromatography (SEC) with On-Line Light Scattering. Size exclusion chromatography column with a MiniDawn on-line light scattering detector (Wyatt Technology, Santa Barbara, California) and refractive index (RI) detectors (Shimadzu, Kyoto, Japan) was used to determine the molecular weight (MW) of the receptor-ligand complex. As shown in Figure 33, the elution profile shows two peaks. Peak #1 represents the receptor-ligand complex and peak #2 represents the unbound VEGF165. MW was calculated from LS and RI signals. The same procedure was used to determine MW of the individual components of the receptor-ligand complex. The results of these determinations are as follows: MW of the Flt1D2Flk1D3.FcΔC1(a)/VEGF165 complex at the peak position is 157 300 (Figure 33), the MW of VEGF165 at the peak position is 44 390 (Figure 34) and the MW of R1 R2 at the peak is 113 300 (Figure 35).

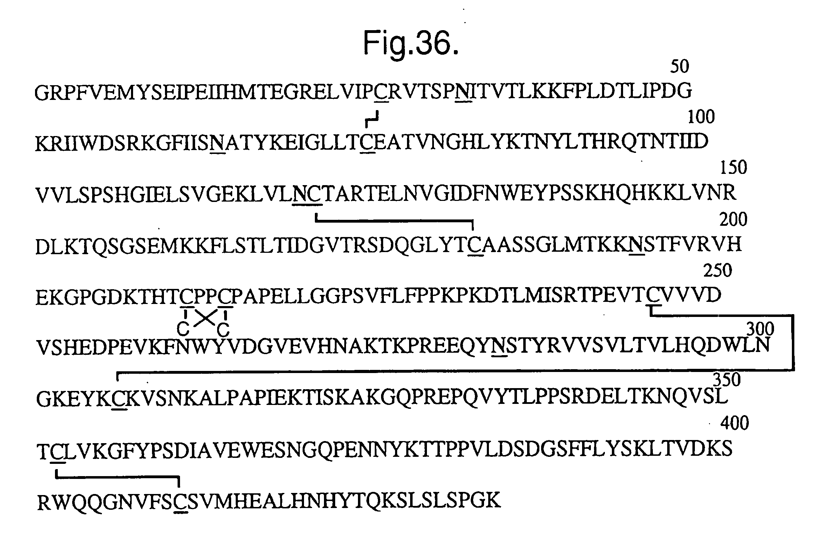

**Figure 36****.** Peptide mapping and glycosylation analysis. The disulfide structures and glycosylation sites in Flt1D2.Flk1D3.FcΔC1(a) were determined by a peptide mapping method. There are a total of ten cysteines in Flt1D2.Flk1D3.FcΔC1(a); six of them belong to the Fc region. Cys27 is disulfide bonded to Cys76. Cys121 is disulfide bonded to Cys 182. The first two cysteines in the Fc region (Cys211 and Cys214) form an intermolecular disulfide bond with the same two cysteines in another Fc chain. However, it can not be determined whether disulfide bonding is occurring between same cysteines (Cys211 to Cys211, for example) or between Cys211 and Cys214. Cys216 is disulfide bonded to Cys306. Cys 352 is disulfide bonded to Cys410.
   There are five possible N-linked glycosylation sites in Flt1D2.Flk1D3.FcΔC1(a) and are found to be glycosylated to varying degrees. Complete glycosylation is observed at Asn33, Asn193, and Asn282. Partial glycosylation is observed on Asn65 and Asn120. Sites of glycosylation are highlighted by underline in the Figure.

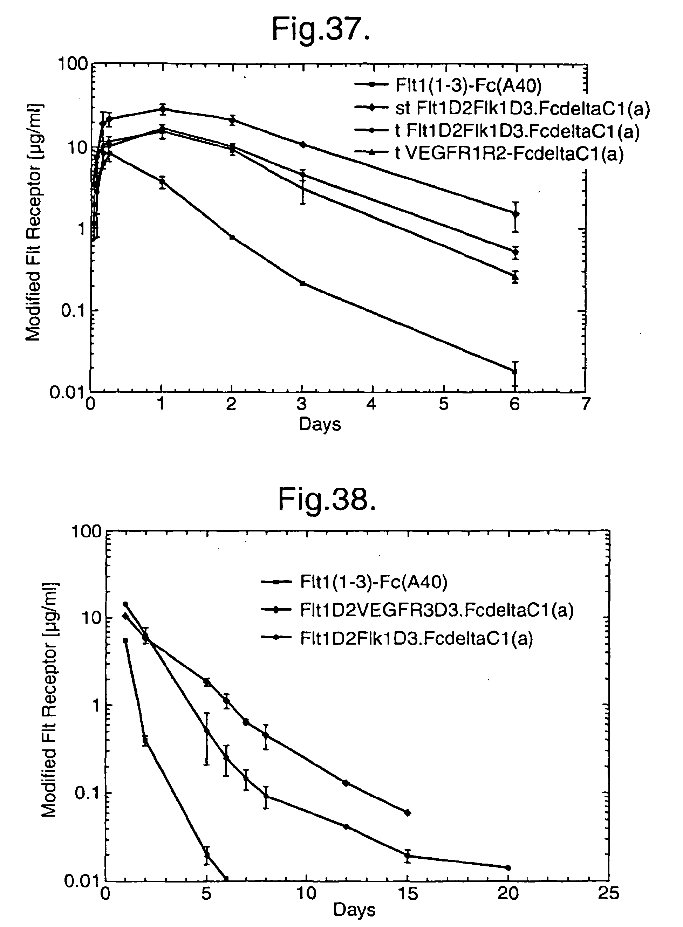

**Figure 37****.** Pharmacokinetics of Flt1(1-3)-Fc (A40), Flt1D2.Flk1D3.FcΔC1(a) and VEGFR1R2-FcΔC1(a). Balb/c mice were injected subcutaneously with 4mg/kg of Flt1(1-3)-Fc (A40), CHO transiently expressed Flt1D2.Flk1D3.FcΔC1(a), CHO stably expressed Flt1D2.Flk1D3.FcΔC1(a), and CHO transiently expressed VEGFR1 R2-FcΔC1(a). The mice were tail bled at 1, 2, 4, 6, 24 hrs, 2 days, 3 days and 6 days after injection. The sera were assayed in an ELISA designed to detect Flt1(1-3)-Fc (A40), Flt1D2.Flk1D3.FcΔC1(a) or VEGFR1 R2-FcΔC1(a). The Tmax for Flt1(1-3)-Fc (A40) was at 6 hrs while the Tmax for the transient and stable Flt1D2.Flk1D3.FcΔC1(a) and the transient VEGFR1R2-FcΔC1(a) was 24hrs. The Cmax for Flt1(1-3)-Fc (A40) was 8µg/ml, For both transients (Flt1D2.Flk1D3.FcΔC1(a) and VEGFR1R2-FcΔC1(a)) the Cmax was 18µg/ml and the Cmax for the stable VEGFR1R2-FcΔC1(a) was 30µg/ml.
**Figure 38****.** Pharmacokinetics of Flt1(1-3)-Fc (A40), Flt1D2.Flk1D3.FcΔC1(a) and Flt1D2.VEGFR3D3.FcΔC1(a). Balb/c mice were injected subcutaneously with 4mg/kg of Flt1(1-3)-Fc (A40), CHO transiently expressed Flt1D2.Flk1D3.FcΔC1(a) and CHO transiently expressed Flt1D2.VEGFR3D3.FcΔC1(a). The mice were tail bled at 1, 2, 5, 6, 7, 8, 12, 15 and 20 days after injection. The sera were assayed in an ELISA designed to detect Flt1(1-3)-Fc,Flt1D2.Flk1D3.FcΔC1(a) and Flt1D2.VEGFR3D3.FcΔC1(a). Flt1(1-3)-Fc (A40) could no longer be detected in the serum after day 5 whereas Flt1D2.Flk1D3.FcΔC1(a) and Flt1D2.VEGFR3D3.FcΔC1(a) were detectable for 15 days or more.

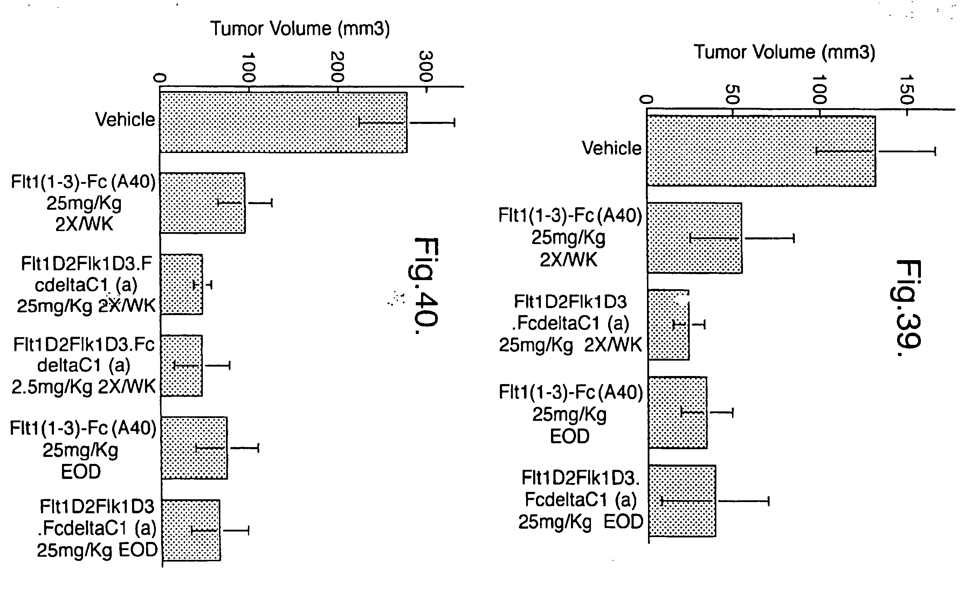

**Figure 39****.** The Ability of Flt1D2.Flk1D3.FcΔC1(a) to Inhibit HT-1080 Fibrosarcoma Tumor Growth In Vivo. Every other day or 2 times per week treatment of SCID mice with Flt1D2.Flk1D3.FcΔC1(a) at 25mg/Kg significantly decreases the growth of subcutaneous HT-1080 fibrosarcoma tumors.
**Figure 40****.** The Ability of Flt1D2.Flk1D3.FcΔC1(a) to Inhibit C6 Glioma Tumor Growth In Vivo. Every other day or 2 times a week treatment of SCID mice with Flt1D2.Flk1D3.FcΔC1(a) significantly decreases the growth of subcutaneous C6 glioma tumors at doses as low as 2.5mg/Kg.

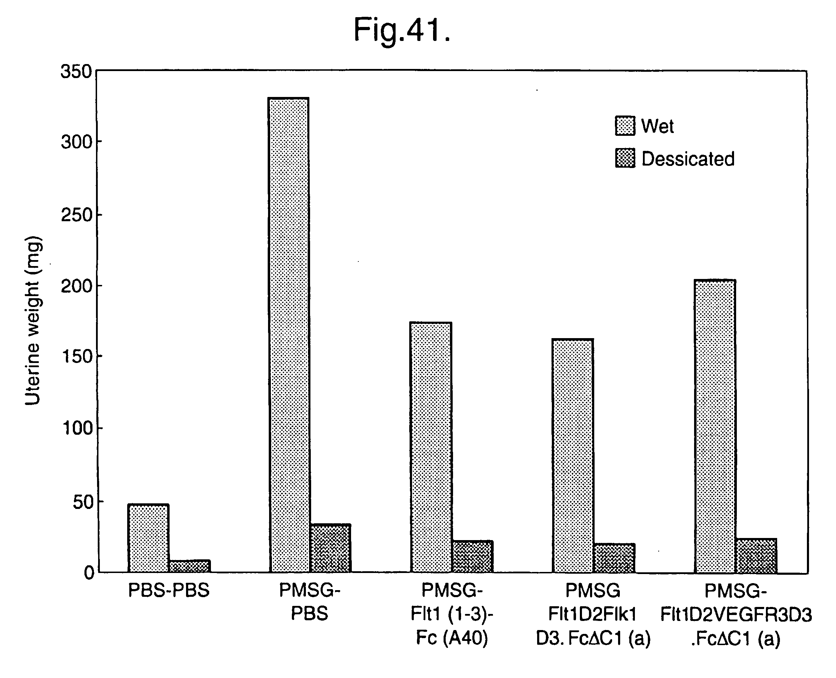

**Figure 41****.** VEGF-Induced Uterine Hyperpermeability. PMSG injected subcutaneously (5 IU) to induce ovulation in prepubertal female rats results in a surge of estradiol after 2 days which in turn causes an induction of VEGF in the uterus. This induction results in hyperpermeability of the uterus and an increase in uterine wet. Subcutaneous injection of Flt1(1-3)-Fc (A40), Flt1D2.Flk1D3.FcΔC1(a) and Flt1D2.VEGFR3D3.FcΔC1(a) at 25mg/kg at 1 hr after PMSG injection results in about a 50% inhibition of the increase in uterine wet weight.

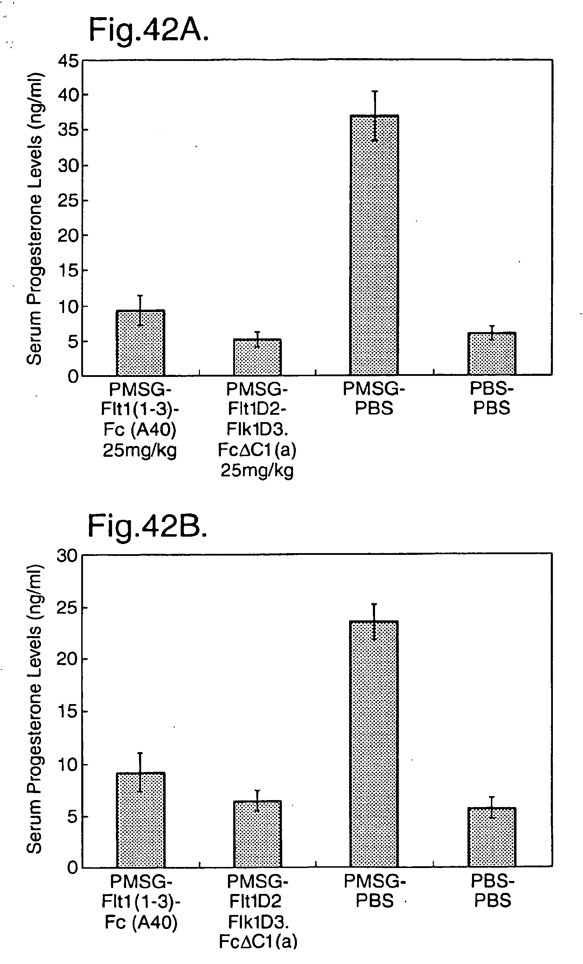

**Figure 42A-42B****.** Assessment of Corpus Luteum Angiogenesis Using Progesterone as a Readout. PMSG was injected subcutaneously (5 IU) to induce ovulation in prepubertal female rats, resulting in a fully functioning corpus luteum containing a dense network of blood vessels that secretes progesterone into the blood stream to prepare the uterus for implantation. The induction of angiogenesis in the corpus luteum requires VEGF. Resting levels of progesterone are about 5ng/ml and can be induced to 25-40ng/ml after PMSG. Subcutaneous injection of Flt1(1-3)-Fc (A40) or Flt1D2.Flk1D3.FcΔC1(a) at 25mg/kg or 5mg/kg at 1 hr. after PMSG injection resulted in a complete inhibition of the progesterone induction on day 4.

### DETAILED DESCRIPTION OF THE INVENTION

It has been a long standing problem in the art to produce a receptor based VEGF antagonist that has a pharmacokinetic profile that is appropriate for consideration of the antagonist as a therapeutic candidate. Applicants describe herein, for the first time, a chimeric polypeptide molecule, capable of antagonizing VEGF activity, that exhibits improved pharmacokinetic properties as compared to other known receptor-based VEGF antagonists. The chimeric polypeptide molecules described herein thus provide for the first time appropriate molecules for use in therapies in which antagonism of VEGF is a desired result, specifically the treatment of eye disorders characterized by vascular permeability.

The present invention involves the use of novel chimeric polypeptide molecules formed by fusing a modified extracellular ligand binding domain of the Flt1 receptor to the Fc region of IgG.

The extracellular ligand binding domain is defined as the portion of a receptor that, in its native conformation in the cell membrane, is oriented extracellularly where it can contact with its cognate ligand. The extracellular ligand binding domain does not include the hydrophobic amino acids associated with the receptor's transmembrane domain or any amino acids associated with the receptor's intracellular domain. Generally, the intracellular or cytoplasmic domain of a receptor is usually composed of positively charged or polar amino acids (i.e. lysine, arginine, histidine, glutamic acid, aspartic acid). The preceding 15-30, predominantly hydrophobic or apolar amino acids (i.e. leucine, valine, isoleucine, and phenylalanine) comprise the transmembrane domain. The extracellular domain comprises the amino acids that precede the hydrophobic transmembrane stretch of amino acids. Usually the transmembrane domain is flanked by positively charged or polar amino acids such as lysine or arginine. von Heijne has published detailed rules that are commonly referred to by skilled artisans when determining which amino acids of a given receptor belong to the extracellular, transmembrane, or intracellular domains (See von Heijne, 1995, BioEssays 17:25-30). Alternatively, websites on the Internet, such as http://ulrec3.unil.ch/software/TMPRED_form.html. have become available to provide protein chemists with information about making predictions about protein domains.

In order to produce fusion polypeptides for the uses of the invention, the present invention provides for the construction of nucleic acid molecules encoding chimeric polypeptide molecules that are inserted into a vector that is able to express the chimeric polypeptide molecules when introduced into an appropriate host cell. Appropriate host cells include, but are not limited to, bacterial cells, yeast cells, insect cells, and mammalian cells. Any of the methods known to one skilled in the art for the insertion of DNA fragments into a vector may be used to construct expression vectors encoding the chimeric polypeptide molecules under control of transcriptional/translational control signals. These methods may include in vitro recombinant DNA and synthetic techniques and in vivo recombinations (genetic recombination) (See Sambrook, et al., Molecular Cloning, A Laboratory Manual, Cold Spring Harbor Laboratory; Current Protocols in Molecular Biology, Eds. Ausubel, et al., Greene Publ. Assoc., Wiley-Interscience, NY).

Expression of nucleic acid molecules encoding the chimeric polypeptide molecules may be regulated by a second nucleic acid sequence so that the chimeric polypeptide molecule is expressed in a host transformed with the recombinant DNA molecule. For example, expression of the chimeric polypeptide molecules described herein may be controlled by any promoter/enhancer element known in the art. Promoters which may be used to control expression of the chimeric polypeptide molecules include, but are not limited to, the long terminal repeat as described in Squinto et al., (1991, Cell 65:1-20); the SV40 early promoter region (Bernoist and Chambon, 1981, Nature 290:304-310), the CMV promoter, the M-MuLV 5' terminal repeat the promoter contained in the 3' long terminal repeat of Rous sarcoma virus (Yamamoto, et al., 1980, Cell 22:787-797), the herpes thymidine kinase promoter (Wagner et al., 1981, Proc. Natl. Acad. Sci. U.S.A. 78:144-1445), the regulatory sequences of the metallothionine gene (Brinster et al., 1982, Nature 296:39-42); prokaryotic expression vectors such as the β-lactamase promoter (Villa-Kamaroff, et al., 1978, Proc. Natl. Acad. Sci. U.S.A. 75:3727-3731), or the tac promoter (DeBoer, et al., 1983, Proc. Natl. Acad. Sci. U.S.A. 80:21-25, see also "Useful proteins from recombinant bacteria" in Scientific American, 1980, 242:74-94); promoter elements from yeast or other fungi such as the Gal 4 promoter, the ADH (alcohol dehydrogenase) promoter, PGK (phosphoglycerol kinase) promoter, alkaline phosphatase promoter, and the following animal transcriptional control regions, which exhibit tissue specificity and have been utilized in transgenic animals: elastase I gene control region which is active in pancreatic acinar cells (Swift et al., 1984, Cell 38:639-646; Ornitz et al., 1986, Cold Spring Harbor Symp. Quant. Biol. 50:399-409; MacDonald, 1987, Hepatology 7:425-515); insulin gene control region which is active in pancreatic beta cells (Hanahan, 1985, Nature 315:115-122), immunoglobulin gene control region which is active in lymphoid cells (Grosschedl et al., 1984, Cell 38:647-658; Adames et al., 1985, Nature 318:533-538; Alexander et al., 1987, Mol. Cell. Biol. 7:1436-1444), mouse mammary tumor virus control region which is active in testicular, breast, lymphoid and mast cells (Leder et al., 1986, Cell 45:485-495), albumin gene control region which is active in liver (Pinkert et al., 1987, Genes and Devel. 1:268-276), alpha-fetoprotein gene control region which is active in liver (Krumlauf et al., 1985, Mol. Cell. Biol. 5:1639-1648; Hammer et al., 1987, Science 235:53-58); alpha 1-antitrypsin gene control region which is active in the liver (Kelsey et al, 1987, Genes and Devel. 1:161-171), beta-globin gene control region which is active in myeloid cells (Mogram et al., 1985, Nature 315:338-340; Kollias et al., 1986, Cell 46:89-94); myelin basic protein gene control region which is active in oligodendrocyte cells in the brain (Readhead et al., 1987, Cell 48:703-712); myosin light chain-2 gene control region which is active in skeletal muscle (Shani, 1985, Nature 314:283-286), and gonadotropic releasing hormone gene control region which is active in the hypothalamus (Mason et al., 1986, Science 234:1372-1378).

Thus, expression vectors capable of being replicated in a bacterial or eukaryotic host comprising chimeric polypeptide molecule-encoding nucleic acid as described herein, are used to transfect the host and thereby direct expression of such nucleic acids to produce the chimeric polypeptide molecules, which may then be recovered in a biologically active form. As used herein, a biologically active form includes a form capable of binding to VEGF.

Expression vectors containing the chimeric nucleic acid molecules described herein can be identified by three general approaches: (a) DNA-DNA hybridization, (b) presence or absence of "marker" gene functions, and (c) expression of inserted sequences. In the first approach, the presence of a foreign gene inserted in an expression vector can be detected by DNA-DNA hybridization using probes comprising sequences that are homologous to the inserted chimeric polypeptide molecule sequences. In the second approach, the recombinant vector/host system can be identified and selected based upon the presence or absence of certain "marker" gene functions (e.g., thymidine kinase activity, resistance to antibiotics, transformation phenotype, occlusion body formation in baculovirus, etc.) caused by the insertion of foreign genes in the vector. For example, if the chimeric polypeptide molecule DNA sequence is inserted within the marker gene sequence of the vector, recombinants containing the insert can be identified by the absence of the marker gene function. In the third approach, recombinant expression vectors can be identified by assaying the foreign gene product expressed by the recombinant. Such assays can be based, for example, on the physical or functional properties of the chimeric polypeptide molecules.

Cells may transiently or, preferably, constitutively and permanently express the chimeric polypeptide molecules.

The chimeric polypeptide molecules may be purified by any technique which allows for the subsequent formation of a stable, biologically active chimeric polypeptide molecule. For example, and not by way of limitation, the factors may be recovered from cells either as soluble proteins or as inclusion bodies, from which they may be extracted quantitatively by 8M guanidinium hydrochloride and dialysis (see, for example, Builder, et al., US Patent No. 5,663,304). In order to further purify the factors, conventional ion exchange chromatography, hydrophobic interaction chromatography, reverse phase chromatography or gel filtration may be used.

In one embodiment of the invention, the nucleotide sequence encoding the first component is upstream of the nucleotide sequence encoding the second component. In another embodiment of the invention, the nucleotide sequence encoding the first component is downstream of the nucleotide sequence encoding the second component. Further embodiments of the invention may be prepared in which the order of the first, second and third fusion polypeptide components are rearranged. For example, if the nucleotide sequence encoding the first component is designated 1, the nucleotide sequence encoding the second component is designated 2, and the nucleotide sequence of the third component is designated 3, then the order of the components in the isolated nucleic acid of the invention as read from 5' to 3' may be any of the following six combinations: 1,2,3; 1,3,2; 2,1,3; 2,3,1; 3,1,2; or 3,2,1.

The present invention also has diagnostic and therapeutic utilities. Methods of detecting aberrancies in the function or expression of the chimeric polypeptide molecules described herein may be used in the diagnosis of disorders. Manipulation of the chimeric polypeptide molecules or agonists or antagonists which bind the chimeric polypeptide molecules may be used in the treatment of diseases. In further embodiments, the chimeric polypeptide molecule is utilized as an agent to block the binding of a binding agent to its target.

The invention may be useful in treating eye disorders characterized by vascular permeability such as age related macular degeneration and diabetic retinopathy.

An amino acid sequence analysis of Flt1(1-3)-Fc revealed the presence of an unusually high number (46) of the basic amino acid residue lysine. An IEF analysis of Flt1(1-3)-Fc showed that this protein has pl greater than 9.3, confirming the prediction that the protein is very basic. It was hypothesized that the basic nature of Fltl(1-3)-Fc protein was causing it to bind to extracellular matrix components and that this interaction might be the cause of the extremely short detectable circulating serum half-life exhibited by Flt1(1-3)-Fc when injected into mice. In order to test this hypothesis, Fltl(1-3)-Fc protein was acetylated at the lysine residues to reduce the basic charge. Acetylated Flt1(1-3)-Fc was then tested in the assays described *infra.*

The following examples are offered by way of illustration and not by way of limitation.

### EXAMPLES

### Example 1: Expression of Flt1(1-3)-Fc protein in CHO K1 cells.

Using standard molecular biology techniques (see e.g., Molecular Cloning, A Laboratory Manual (Sambrook, et al., Cold Spring Harbor Laboratory), Current Protocols in Molecular Biology (Eds. Ausubel, et al., Greene Publ. Assoc., Wiley-Interscience, NY), the gene encoding Flt1(1-3)-Fc was inserted into the expression vector pEE14.1 (Lonza Biologics, plc) at a multiple cloning site downstream of the CMV promoter. CHO K1 cells were transfected with the pEE14.1/Flt1(1-3)-Fc DNA construct using lipofectamine (Gaithersburg, MD). The transfected CHO K1 cells were grown in glutamine-free DMEM (JRH, Kansas City, MO) containing 25µM methionine sulfoximine (MSX) from Sigma Inc., St. Louis, MO, and high recombinant protein expressors were obtained by screening the CHO K1 cell supernatants from over 100 hand-picked colony isolates using a standard immunoassay which captures and detects human Fc. The selected hand-picked clone was amplified in the presence of 100 µM MSX followed by a second round of screening of the amplified clones. The highest producing clone had a specific productivity of recombinant Flt1(1-3)-Fc protein of 55 pg/cell/day.

The selected clone was expanded in 225cm² T-flasks (Corning, Acton, MA) and then into 8.5L roller bottles (Corning, Acton, MA) using the cell culture media described *supra*. Cells were removed from the roller bottles by standard trypsinization and put into 3.5L of suspension medium. The suspension medium is comprised of glutamine-free ISCHO medium (Irvine Scientific, Santa Ana, CA) containing 5% fetal bovine serum (FBS from Hyclone Labs, Logan, UT), 100µM MSX and GS supplement (JRH Scientific, Kansas City, MO) in a 5L Celligen bioreactor (New Brunswick Scientific, New Brunswick, NJ) at a density of 0.3 x 10⁶ cells/mL. After the cells reached a density of 3.6 x 10⁶/mL and were adapted to suspension they were transferred to a 60L bioreactor (ABEC, Allentown, PA) at a density of 0.5 x 10⁶ cells/mL in 20L of ISCHO medium with 5% fetal bovine serum. After two days an additional 20L of ISCHO + 5% fetal bovine serum was added to the bioreactor. The cells were allowed to grow for an additional two days reaching a final density of 3.1 x 10⁶ cells/mL, and a final Flt1(1-3)-Fc concentration at harvest was 95 mg/L. At harvest the cells were removed by tangential flow filtration using 0.45µm Prostak Filters (Millipore, Inc., Bedford, MA).

### Example 2: Purification of Flt1(1-3)-Fc protein obtained from CHO K1 cells

Flt1(1-3)-Fc protein was initially purified by affinity chromatography. A Protein A column was used to bind, with high specificity, the Fc portion of the molecule. This affinity-purified protein was then concentrated and passed over a SEC column. The protein was then eluted into the formulation buffer. The following describes these procedures in detail.

### Materials and Methods

All chemicals were obtained from J.T. Baker, Phillipsburg, NJ with the exception of PBS; which was obtained as a 10X concentrate from Life Technologies, Gaithersburg, MD. Protein A Fast Flow and Superdex 200 preparation grade resins were obtained from Pharmacia, Piscataway, NJ. Equipment and membranes for protein concentration were obtained from Millipore, Bedford, MA.

Approximately 40L of 0.45µm-filtered CHO conditioned media containing Flt1(1-3)-Fc protein was applied to a 290mL Protein A Fast Flow column (10cm diameter) that had been equilibrated with PBS. The column was washed with PBS containing 350mM NaCl and 0.02% CHAPS and the bound protein was eluted with 20mM Citric Acid containing 10mM Na₂HPO₄. The single peak in the elution was collected and its pH was raised to neutrality with 1 M NaOH. The eluate fractions was concentrated to approximately 9 mg/mL using 10K regenerated cellulose membranes by both tangential flow filtration and by stirred cell concentration. To remove aggregates and other contaminants, the concentrated protein was applied to a column packed with Superdex 200 preparation grade resin (10cm x 55cm) and run in PBS containing 5 % glycerol. The main peak fractions were pooled, sterile filtered, aliquoted and stored at -80°C.

### Example 3: Acetylation of Flt1(1-3)-Fc protein.

Two milligrams of Flt1(1-3)-Fc protein were acetylated as described in the instruction manual provided with the sulfo-NHS-acetate modification kit (Pierce Chemical Co., Rockford, IL, Cat.#26777).

### Example 4: Characterization of acetylated Flt1(1-3)-Fc protein.

**(a.) IEF analysis:** Flt1(1-3)-Fc and acetylated Flt1(1-3)-Fc were analyzed by standard IEF analysis. As shown in Figure 1, Fltl(1-3)-Fc protein is not able to migrate into the gel and therefore must have a pl greater than 9.3, the highest pl in the standard. However, acetylated Flt1(1-3)-Fc is able to migrate into the gel and equilibrate at a pl of approximately 5.2. This result demonstrates that acetylation reduces the net positive charge of the protein and therefore its pl considerably.

### (b.) Binding to extracellular matrix components

To test for binding to extracellular matrix components, Flt1(1-3)-Fc and acetylated Flt1(1-3)-Fc where tested in an assay designed to mimic the interaction with extracellular matrix components. In this assay, 96-well tissue culture plates are coated with Matrigel (Biocoat MATRIGEL^{®} matrix thin layer 96 well plate, Catalog #40607, Becton Dickinson Labware, Bedford, MA). The plates are incubated with varying concentrations of either Flt1(1-3)-Fc, acetylated Flt1(1-3)-Fc, or rTie2-Fc (an irrelevant control) protein are added to the wells. The plates are incubated for 1-2 hours at either room temperature or 37°C degrees and then detection of bound proteins is accomplished by adding a secondary alkaline phosphatase-conjugated anti-human Fc antibody to the wells. Finally, alkaline phosphatase substrate is added to the wells and optical density is measured. Figure 2 shows the results of this assay. Like the irrelevant control protein rTie2-Fc, acetylated Flt1(1-3)-Fc does not exhibit any binding to the Matrigel coated plate, whereas the non-acetylated Flt1(1-3)-Fc protein exhibits significant binding. This result indicates that acetylation of basic amino acid residues is an effective way to interfere with the charge interactions that exist between positively charged proteins and the negatively charged extracellular matrix components they are exposed to *in vivo.*

### Example 5: Pegylation of Flt1(1-3)-Fc protein.

Although pegylation (polyethylene glycol - PEG) of proteins has been shown to increase their *in vivo* potency by enhancing stability and bioavailability while minimizing immunogenicity (see references cited *supra*), it is counter-intuitive that pegylating molecules that are too large to be filtered by the kidney glomeruli would improve their pharmacokinetic properties. Without being bound by theory, Applicants postulated that pegylation of the Flt1(1-3)-Fc molecules could improve the pharmacokinetic properties, possibly not by altering the positive charge or by decreasing the pl of Flt1(1-3)-Fc, but rather by physically shielding the positive charges from interacting with the extracellular matrix. Applicants decided to attempt to improve the pharmacokinetic properties of Flt1(1-3)-Fc molecules by attaching strands of 20K PEGs as described *infra*.

### Materials and Methods

Purified Flt1(1-3)-Fc derived from CHO cells (see *supra*) was used in the following pegylation experiments. Functionalized PEGs were obtained from Shearwater Polymers, Huntsville, AL; Bicine from Sigma, St Louis, MO; Superose 6 column from Pharmacia, Piscataway, NJ; PBS as a 10X concentrate from Life Technologies, Gaithersburg, MD; Glycerol from J.T. Baker, Phillipsburg, NJ; and Bis-Tris precast gels from Novex, CA.

20K PEG strands functionalized with amine-specific terminal moieties were used in small-scale reaction studies that were set-up to evaluate different reaction conditions in which the PEG:protein stoichiometry was varied. Based on these reactions and the analyses of samples on standard SDS-PAGE, Flt1(1-3)-Fc at a concentration of 1.5 mg/mL was reacted at pH 8.1 with 20K SPA-PEG (PEG succinimidyl propionate) molecules at a PEG-to-Flt1(1-3)-Fc monomer molar ratio of 1:6. The reaction was allowed to proceed at 8°C overnight. For initial purification, the reaction products were applied to a 10mm x 30cm Superose 6 column equilibrated with PBS containing 5% Glycerol. The column appeared to separate pegylated Flt1(1-3)-Fc molecules based on the extent of pegylation. Fractions corresponding to what appeared to be primarily mono-pegylated and di-pegylated dimeric Flt1(1-3)-Fc, as judged by banding patterns on reducing and non-reducing SDS-PAGE gels were pooled. The protein concentration was determined by measuring absorbance at 280 nm. The pegylated Flt1(1-3)-Fc protein was sterile filtered, aliquoted and stored at -40°C.

### Example 6: Binding of unmodified, acetylated, and pegylated Flt1(1-3)-Fc in a Biacore-based assay.

Unmodified, acetylated, and pegylated Flt1(1-3)-Fc proteins were tested in a Biacore-based assay to evaluate their ability to bind to the Flt1 ligand, VEGF. In this assay, unmodified Flt1(1-3)-Fc protein was immobilized on the surface of a Biacore chip (see Biacore Instruction Manual, Pharmacia, Inc., Piscataway, NJ, for standard procedures) and a sample containing 0.2 µg/ml VEGF and either unmodified Flt1(1-3)-Fc, acetylated Flt1(1-3)-Fc or pegylated Flt1(1-3)-Fc (each at 25 µg/ml) was passed over the Flt1(1-3)-Fc-coated chip. To minimize the effects of non-specific binding, the bound samples were washed with a 0.5M NaCl wash. In one sample, unmodified Flt1(1-3)-Fc was mixed with heparin. Heparin is a negatively charged molecule and the Flt1(1-3)-Fc protein is a positively charged molecule, so when the two molecules are mixed together, they should interact through their respective charges. This essentially neutralizes Flt1(1-3)-Fc's inherent positive charge making the molecule behave as if it has been chemically or genetically modified so as to reduce its charge and its tendency to bind via charge interactions. As shown in Figure 3, acetylated (columns 13-16), pegylated (columns 17-20), and heparin-treated Flt1(1-3)-Fc (columns 21-24) are each able to completely compete with the Biacore chip-bound Flt1(1-3)-Fc for VEGF binding as compared to control (columns 1-4) and irrelevant protein (columns 5-8). Unmodified Flt1(1-3)-Fc (columns 5-6) appeared to only partially compete with Biacore chip-bound Flt1(1-3)-Fc for VEGF binding. However, washing the bound samples with 0.5M NaCl (columns 7-8) resulted in a binding profile similar to the modified forms of Flt1(1-3)-Fc, indicating that the unmodified protein was exhibiting non-specific binding to the chip that could be eliminated by the salt wash.

### Example 7: Binding of unmodified, acetylated, and pegylated Flt1(1-3)-Fc in an ELISA-based assay.

Unmodified, acetylated, and pegylated Flt1(1-3)-Fc proteins were tested in a standard ELISA-based assay to evaluate their ability to bind the Flt1 receptor ligand VEGF. As shown in Figure 4, both pegylated and acetylated Flt1(1-3)-Fc proteins are capable of binding to VEGF, demonstrating that modifying the protein either by pegylation or acetylation does not destroy its ability to bind its ligand.

### Example 8: Pharmacokinetic analysis of unmodified Flt1(1-3)-Fc, acetylated Flt1(1-3)-Fc, and pegylated Flt1(1-3)-Fc.

*In vivo* experiments were designed to assess the pharmacokinetic profiles of unmodified Flt1(1-3)-Fc, acetylated Flt1(1-3)-Fc, and pegylated Flt1(1-3)-Fc protein. Balb/c mice (23-28g; 3 mice/group) were injected subcutaneously with 4mg/kg of unmodified, acetylated, or pegylated Flt1(1-3)-Fc. The mice were tail bled at 1, 2, 4, 6, 24 hours, 2 days, and 3 days after injection of protein. The sera were assayed in a standard ELISA-based assay designed to detect Flt1(1-3)-Fc protein. Briefly, the assay involves coating an ELISA plate with VEGF, binding the unmodified, acetylated, or pegylated Flt1(1-3)-Fc-containing sera, and reporting with an anti-Fc antibody linked to alkaline phosphatase. As shown in Figure 5, the Tmax for all of the Flt1(1-3)-Fc proteins was between the 6 hour and 24 hour time points. The Cmax for the different proteins was as follows: Unmodified: 0.06 µ/ml - 0.15 µg/ml; acetylated: 1.5 µg/ml - 4.0 µg/ml; and pegylated: approximately 5 µg/ml.

### Example 9: Step-acetylation of Flt1(1-3)-Fc

To determine what minimal amount of acetylation is necessary to eliminate binding to extracellular matrix components, an experiment was designed that acetylated the Flt1(1-3)-Fc protein in a step-wise fashion by using increasing amounts of molar excess of acetylation reagent in the acetylation reaction mixture. The range of molar excess was as follows: 0, 10, 20, 30, 40, 50, 60, 70, 80 , 90, and 100 moles of acetylation reagent per 1 mole of Flt1(1-3)-Fc monomer. The reactions were performed as detailed in the instruction manual provided with the sulfo-NHS-Acetate modification kit (Pierce Chemical Co., Rockford, IL, Cat.# 26777).

### Example 10: Characterization of step-acetylated Flt1(1-3)-Fc.

**(a.) IEF analysis** Unmodified Flt1(1-3)-Fc and step-acetylated Flt1(1-3)-Fc proteins were analyzed by standard IEF analysis. As shown in Figure 6A-6B, unmodified Fltl(1-3)-Fc protein was not able to migrate into the gel due to its extremely high pl (greater than 9.3). However, most of the step-acetylated Flt1(1-3)-Fc samples (30-100 fold molar excess samples) were able to migrate into the gel and equilibrate at pls ranging between 4.55 - 8.43, depending on the degree of acetylation of the protein. This result demonstrates that acetylation can change the positive charge of the protein in a dose-dependent manner and that reduction of the pl can be controlled by controlling the degree of acetylation.

### (b.) Binding of step-acetylated Flt1(1-3)-Fc to extracellular matrix components

To test for binding to extracellular matrix components, Flt1(1-3)-Fc and step-acetylated Flt1(1-3)-Fc where tested in the above-described assay designed to mimic the interaction with extracellular matrix components. Varying concentrations of either unmodified Flt1(1-3)-Fc, step-acetylated Flt1(1-3)-Fc (10, 20, and 30 fold molar excess samples), or rTie2-Fc (an irrelevant control) protein were added to the wells. The plates were incubated for 1-2 hours at room temperature or 37°C and then detection of bound proteins was accomplished by adding a secondary alkaline phosphatase-conjugated anti-human Fc antibody to the wells. Alkaline phosphatase substrate was subsequently added to the wells and optical density measured. Figure 7 shows the results of this assay. Like the irrelevant control protein rTie2-Fc, step-acetylated Flt1(1-3)-Fc (20 and 30 fold molar excess samples) did not exhibit any significant binding to the Matrigel coated plate, whereas the non-acetylated Flt1(1-3)-Fc protein exhibited significant binding. The binding is saturable, indicating that the Flt1(1-3)-Fc protein may be binding to specific sites, rather than a more general charge-mediated interaction that might not be saturable. The 10 fold molar excess sample showed reduced binding, but the degree of acetylation was not enough to completely block binding to extracellular matrix components. The 20 fold molar excess and higher samples displayed no detectable binding, despite the fact that by IEF analysis (Figure 6A and 6B) the lower molar excess samples still had a large net positive charge. This result demonstrates that it is not necessary to completely acetylate all available basic amino acids in order to eliminate binding to extracellular matrix components.

### (c.) Binding of step-acetylated Flt1(1-3)-Fc in a Biacore-based assay.

Unmodified and step-acetylated Flt1(1-3)-Fc proteins where tested in a Biacore-based assay to evaluate their ability to bind to the Flt1 ligand, VEGF. In this assay, unmodified Flt1(1-3)-Fc protein (0.5, 1.0, or 5.0 µg/ml) was immobilized on the surface of a Biacore chip (see Biacore Instruction Manual, Pharmacia, Inc., Piscataway, NJ, for standard procedures) and a solution containing 0.2 µg/ml VEGF and either unmodified Flt1(1-3)-Fc (at either 0.5, 1.0, or 5.0 µg/ml) or 10 different step-acetylated Flt1(1-3)-Fc samples (at 0.5, 1.0, or 5.0 µg/ml each) were passed over the Flt1(1-3)-Fc-coated chip. As shown in Figure 8, at a sub-stoichiometric ratio (0.5 µg/ml of either unmodified Flt1(1-3) or step-acetylated Flt1(1-3)-Fc vs. 0.2 µg/ml VEGF), there is not enough Flt1(1-3)-Fc (either unmodified or step-acetylated) in the solution to completely bind the VEGF. At 1.0 µg/ml, which approximates a 1:1 stoichiometric ratio, both unmodified and step-acetylated Flt1(1-3)-Fc are better able to compete for VEGF binding, but there is still insufficient Flt1(1-3)-Fc protein (either unmodified or step-acetylated) to completely bind the available VEGF. However, at 5.0 µg/ml, which is several times greater than a 1:1 stoichiometric ratio, both the Flt1(1-3)-Fc and the step-acetylated Flt1(1-3)-Fc proteins are able to bind the VEGF, regardless of the degree of acetylation. This clearly demonstrates that acetylation does not alter Flt1(1-3)-Fc's ability to bind VEGF.

### (d.) Pharmacokinetic analysis of step-acetylated Flt1(1-3)-Fc

*In vivo* experiments were designed to assess the pharmacokinetic profiles of unmodified Flt1(1-3)-Fc and step-acetylated Flt1(1-3)-Fc protein. Balb/c mice (23-28g) were injected subcutaneously with 4mg/kg of unmodified or 10, 20, 40, 60 and 100 fold molar excess samples of step-acetylated Flt1(1-3)-Fc (3 mice for unmodified, 10, 20 and 40 fold molar excess samples and 2 mice for 60 and 100 fold molar excess samples). The mice were tail bled at 1, 2, 4, 6, 24 hours, 2 days and 3 days after injection. The sera were assayed in an ELISA-based assay designed to detect Flt1(1-3)-Fc (described *supra*). Figure 9 details the results of this study. The Tmax for all of the Flt1(1-3)-Fc proteins tested was at the 6 hour time point but the Cmax was as follows: Unmodified Flt1(1-3)-Fc: 0.06µg/ml; 10 fold molar excess sample: - 0.7µg/ml, 20 fold molar excess sample - 2µg/ml, 40 fold molar excess sample - 4µg/ml, 60 fold molar excess sample - 2µg/ml, 100 fold molar excess sample - 1µg/ml. This results demonstrates that acetylation or pegylation of Flt1(1-3)-Fc significantly improves its pharmacokinetic profile.

### Example 11: Construction of Flt1(1-3)-Fc basic region deletion mutant designated Mut1: Flt1(1-3_{ΔB})-Fc.

Based on the observation that acetylated Flt1(1-3)-Fc, which has a pl below 6, has much better pharmacokinetics than the highly positive unmodified Flt1(1-3)-Fc (pl > 9.3), it was asked whether the difference in pharmacokinetics could be attributed to the net charge of the protein, which made it stick to negatively charged extracellular matrix components, or whether there were perhaps specific locations on the surface of the Flt1(1-3)-Fc protein that constituted specific binding sites for extracellular matrix components. For example, many proteins are known to have heparin binding sites, often consisting of a cluster of basic residues. Sometimes these residues are found in a cluster on the primary sequence of the protein; some of the literature has identified "consensus sequences" for such heparin binding sites (see for example Hileman, et al., 1998, Bioessays 20(2):156-67). In other cases, the known crystal structure of a protein reveals a cluster of positively charged residues on the surface of a protein, but the residues come from different regions of the primary sequence and are only brought together when the protein folds into its tertiary structure. Thus it is difficult to deduce whether an isolated amino acid residue forms part of a cluster of basic residues on the surface of the protein. However, if there is a cluster of positively charged amino acid residues in the primary sequence, it is not unreasonable to surmise that the residues are spatially close to one another and might therefore be part of an extracellular matrix component binding site. Flt1 receptor has been studied extensively and various domains have been described (see for example Tanaka et al., 1997, Jpn. J. Cancer Res 88:867-876). Referring to the nucleic acid and amino acid sequence set forth in Figure 10A-10D of this application, one can identify the signal sequence for secretion which is located at the beginning of the sequence and extends to the glycine coded for by nucleotides 76-78. The mature protein begins with Ser-Lys-Leu-Lys, starting at nucleotide 79 of the nucleic acid sequence. Flt1 Ig domain 1 extends from nucleotide 79 to 393, ending with the amino acids Ser-Asp-Thr. Flt1 Ig domain 2 extends from nucleotide 394 to 687 (encoding Gly-Arg-Pro to Asn-Thr-Ile), and Flt1 Ig domain 3 extends from nucleotides 688 to 996 (encoding Ile-Asp-Val to Asp-Lys-Ala). There is a bridging amino acid sequence, Gly-Pro-Gly, encoded by nucleotides 997-1005, followed by the nucleotide sequence encoding human Fc (nucleotides 1006-1701 or amino acids Glu-Pro-Lys to Pro-Gly-Lys-stop).

A more detailed analysis of the Flt1 amino acid sequence reveals that there is a cluster, namely, amino acid residues 272-281 (KNKRASVRR) of Figure 10A-10D, in which 6 out of 10 amino acid residues are basic. This sequence is located in Flt1 Ig domain 3 of the receptor (see Figure 11), which is not itself essential for binding of VEGF ligand, but which confers a higher affinity binding to ligand. An alignment of the sequence of Ig domain 3 with that of Ig domain 2 reveals that in this region, there is very poor alignment between the two Ig domains, and that there are about 10 additional amino acids in Ig domain 3. An analysis of the hydrophilicity profiles (MacVector computer software) of these two domains clearly indicates the presence of a hydrophilic region in the protein (Figure 12A-12B). These observations raised the possibility that the actual three dimensional conformation of Flt1 Ig domain 3 allowed for some type of protrusion that is not in Flt1 Ig domain 2. To test this hypothesis, the 10 additional amino acids were deleted and the resulting protein was tested to see whether the deletion would affect the pharmacokinetics favorably without seriously compromising the affinity of the receptor for VEGF. This DNA construct, which was constructed using standard molecular biology techniques (see e.g., Molecular Cloning, A Laboratory Manual (Sambrook, et al., Cold Spring Harbor Laboratory), Current Protocols in Molecular Biology (Eds. Ausubel, et al., Greene Publ. Assoc., Wiley-Interscience, NY) in the mammalian expression vector pMT21 (Genetics Institute, Inc., Cambridge, MA), is referred to as Mut1: Flt1(1-3_{ΔB})-Fc. The Mut1: Flt1(1-3_{ΔB})-Fc construct was derived from Flt1(1-3)-Fc by deletion of nucleotides 814-843 (set forth in Figure 10A-10D), which deletes the highly basic 10-amino acid residue sequence Lys-Asn-Lys-Arg-Ala-Ser-Val-Arg-Arg-Arg from Flt1 Ig domain 3.

The final DNA construct was sequence-verified using an ABI 373A DNA sequencer and Taq Dideoxy Terminator Cycle Sequencing Kit (Applied Biosystems, Inc., Foster City, CA). The sequence of Mut1: Flt1(1-3_{ΔB})-Fc is set forth in Figure 13A-13D.

### Example 12: Construction of Flt1(1-3)-Fc basic region deletion mutant designated Mut2: Flt1(2-3 _{ΔB})-Fc.

A second deletion mutant construct, designated Mut2: Flt1(2-3_{ΔB})-Fc, was derived from the Mut1: Flt1(1-3_{ΔB})-Fc construct by deletion of Flt1 Ig domain 1 encoded by nucleotides 79-393 (see Figure 10A-10D); for convenience, nucleotides 73-78 (TCA GGT) were changed to TCC GGA. This introduced a restriction site (BspE1) without altering the associated amino acid sequence, Ser-Gly. This DNA construct, which was constructed using standard molecular biology techniques (see e.g., Molecular Cloning, A Laboratory Manual (Sambrook, et al., Cold Spring Harbor Laboratory), Current Protocols in Molecular Biology (Eds. Ausubel, et al., Greene Publ. Assoc., Wiley-Interscience, NY) in the mammalian expression vector pMT21 (Genetics Institute, Inc., Cambridge, MA), was also sequence-verified using an ABI 373A DNA sequencer and Taq Dideoxy Terminator Cycle Sequencing Kit (Applied Biosystems, Inc., Foster City, CA). The sequence of Mut2: Flt1(2-3_{ΔB})-Fc is set forth in Figure 14A-14C.

### Example 13: Construction of Flt1(1-3)-Fc deletion mutant designated Mut3: Flt1(2-3)-Fc.

A third deletion mutate construct, designated Mut3: Flt1(2-3)-Fc, was constructed the same way as the Mut2: Flt1(2-3_{ΔB})-Fc construct, except that Flt1 Ig domain 3 was left intact (the basic region amino acids were not deleted). The construct was constructed using standard molecular biology techniques and the final construct was sequence-verified as described *supra.* The sequence of Mut3: Flt1(2-3)-Fc is set forth in Figure 15A-15C.

### Example 14: Construction of Flt(1-3)-Fc basic region N-glycosylation mutant designated Mut4: Flt1(1- 3 _{R->N})-Fc.

A final construct was made in which a N-glycosylation site was introduced into the middle of the basic region of Flt1 Ig domain 3. This construct was designated Mut4: Flt1(1-3_{R**->**N})-Fc and was made by changing nucleotides 824-825 from GA to AC, consequently changing the coded Arg residue (AGA) into an Asn residue (AAC) (see Figure 10A-10D). The resulting amino acid sequence is therefore changed from Arg-Ala-Ser to Asn-Ala-Ser, which matches the canonical signal (Asn-Xxx-Ser/Thr) for the addition of a N-glycosylation site at the Asn residue. The sequence of Mut4: Flt1(1-3_{R->N})-Fc is set forth in Figure 16A-16D.

### Example 15: Characterization of acetylated Flt1(1-3)-Fc. Mut1 : Flt1(1-3_{ΔB})-Fc, and Mut4: Flt1(1-3_{R->N})-Fc mutants.

### (a.) Binding to extracellular matrix components

To determine whether the three modified proteins were more or less likely to have improved pharmacokinetic properties, Matrigel coated 96-well dishes (as described *supra*) were incubated with varying concentrations of the mutant proteins and detected with anti-human Fc/alkaline-phosphatase conjugated antibodies. As shown in Figure 18, this experiment showed that while the unmodified Flt1(1-3)-Fc protein could bind avidly to these wells, the Mut3: Flt1(2-3)-Fc protein bound somewhat more weakly, the Mut1: Flt1(1-3_{ΔB})-Fc protein bound more weakly still, and the Mut2: Flt1(2-3_{ΔB})-Fc protein showed the best profile, binding more weakly than any of the other mutant proteins. The Mut4: Flt1(1-3_{R->N})-Fc glycosylation mutant protein showed only marginal benefit on the Matrigel assay. These results confirm the hypothesis that a linear sequence of positive amino acids can be deleted from the primary sequence resulting in a decrease in charge interaction with extracellular matrix components.

### (b.) Binding of Mut1 : Flt1(1-3_{ΔB})-Fc and Mut4: Flt1 (1-3_{R->N})-Fc in a Biacore-based assay.

Unmodified and acetylated Flt1(1-3)-Fc and genetically modified Mut1: Flt1(1-3_{ΔB})-Fc and Mut4: Flt1(1-3_{R->N})-Fc proteins where tested in a Biacore-based assay to evaluate their ability to bind to the Flt1 ligand, VEGF. In this assay, unmodified Flt1(1-3)-Fc protein (0.25, 0.5, or 1.0 µg/ml) was immobilized on the surface of a Biacore chip (see Biacore Instruction Manual, Pharmacia, Inc., Piscataway, NJ, for standard procedures) and a solution containing 0.1 µg/ml VEGF and either purified or COS cell supernatant containing unmodified Flt1(1-3)-Fc (at approximately (0.25, 0.5, or 1.0 µg/ml), purified acetylated Flt1(1-3)-Fc (at (0.25, 0.5, or 1.0 µg/ml), COS cell supernatant containing Mut1: Flt1(1-3_{ΔB})-Fc (at approximately (0.25, 0.5, or 1.0 µg/ml), or COS cell supernatant containing Mut4: Flt1(1-3_{R->N})-Fc (at approximately (0.25, 0.5, or 1.0 µg/ml) were passed over the Flt1(1-3)-Fc-coated chip. As shown in Figure 17, at the sub-stoichiometric ratio (0.25 µg/ml Flt1(1-3)-Fc of unmodified, acetylated or genetically modified samples vs. 01. µg/ml VEGF), there is insufficient Flt1(1-3)-Fc protein to block binding of VEGF to the Flt1(1-3)-Fc immobilized on the Biacore chip. At 0.5 µg/ml of unmodified, acetylated or genetically modified Flt1(1-3)-Fc proteins, the stoichiometric ratio approximates 1:1 and there is an increased ability to block VEGF binding to the Biacore chip. At 1.0 µg/ml of unmodified, acetylated or genetically modified Flt1(1-3)-Fc proteins, which is approximately a 10:1 stoichiometric ratio, the Flt1(1-3)-Fc proteins are able to block binding of VEGF to the Biacore chip, but they are not equivalent. Unmodified, acetylated, and Mut1: Flt1(1-3_{ΔB})-Fc are essentially equal in their ability to block VEGF binding, whereas Mut4: Flt1(1-3_{R->N})-Fc is somewhat less efficient at blocking binding. These results confirm the hypothesis that it is possible to reduce the non-specific binding of a positively charged molecule by genetically removing a linear sequence of predominantly negatively charged amino acids.

### (c.) Binding of Mut1: Flt1(1-3_{ΔB})-Fc, Mut2: Flt1(2-3_{ΔB})-Fc. Mut3: Flt1(2-3)-Fc, and in an ELISA-based assay.

To determine whether the three mutant proteins could bind the Flt1 ligand VEGF, binding experiments were done in which 96-well plates coated with VEGF were incubated with varying concentrations of the respective mutant protein, and after washing, the amount bound was detected by incubating with an alkaline phosphatase conjugated anti-human Fc antibody and quantitated colorimetrically by the addition of an appropriate alkaline phosphatase substrate. As shown in Figure 19, this experiment showed that all the mutant proteins could bind VEGF similarly, at the concentrations tested.

### Example 16: Pharmacokinetic analysis of acetylated Flt1 (1-3)-Fc, Mut1: Flt1(1-3_{ΔB}-Fc, and unmodified Flt1(1-3)-Fc.

*In vivo* experiments were designed to assess the pharmacokinetic profiles of unmodified Flt1(1-3)-Fc, Mut1: Flt1(1-3_{ΔB})-Fc, and 40 fold molar excess acetylated Flt1(1-3)-Fc protein. Balb/c mice (25-30g) were injected subcutaneously with 4mg/kg of unmodified Flt1(1-3)-Fc, 40 fold molar excess acetylated Flt1(1-3)-Fc, and Mut1: Flt1(1-3_{ΔB})-Fc proteins (4 mice each). These mice were tail bled at 1, 2, 4, 6, 24 hours, 2 days, 3 days, and 5 days after injection. The sera were assayed in an ELISA designed to detect Flt1(1-3)-Fc protein which involves coating an ELISA plate with VEGF, binding the Flt1(1-3)-Fc and reporting with an anti-Fc antibody linked to alkaline phosphatase. As shown in Figure 20, the Cmax for these reagents was as follows: Unmodified Flt1(1-3)-Fc - 0.15µg/ml; 40 fold molar excess acetylated Flt1(1-3)-Fc - 1.5µg/ml; and Mut1: Flt1(1-3_{ΔB})-Fc - 0.7µg/ml.

### Example 17: Modified Flt1 receptor vector construction

The rationale for constructing modified versions of the Flt1 receptor (also known as VEGFR1) was based on the observation that the protein sequence of Flt1 was highly basic, and was therefore likely to stick to extracellular matrix (ECM). The highly basic nature of Flt1 probably explains why unmodified Flt1(1-3)-Fc (described *supra*) has poor pharmacokinetics that make it difficult to use as a therapeutic agent. As described *supra,* the chemically modified form of 40 fold molar excess acetylated Flt1(1-3)-Fc, hereinafter termed A40, exhibited a greatly improved pharmacokinetic (PK) profile over the non-acetylated Flt1(1-3)-Fc. Therefore, attempts were made to engineer DNA molecules that could be used to recombinantly express modified forms of a Flt1 receptor molecule that would possess the improved PK profile exhibited by A40 and still maintain the ability to bind tightly to VEGF.

It is known in the literature that the first Ig domain of Flt1 (which has a net charge of +5 at neutral pH) is not essential for tight binding to VEGF, so this domain was deleted. The third Ig domain (having a net charge of +11) is not essential for binding, but confers higher affinity for VEGF than the second Ig domain, so instead of deleting it entirely, it was replaced with the equivalent domains of the Flt1 receptor relatives Flk1 (also known as VEGFR2) and Flt4 (also known as VEGFR3). These chimeric molecules (denoted R1 R2 (Flt1.D2.Flk1D3.FcΔC1(a) and VEGFR1R2-FcΔC1(a) and R1 R3 (Flt1D2.VEGFR3D3-FcΔC1(a) and VEGFR1R3-FcΔC1(a) respectively, wherein R1 and Flt1D2 = Ig domain 2 of Flt1 (VEGFR1); R2 and Flk1D3 = Ig domain 3 of Flk1 (VEGFR2); and R3 and VEGFR3D3 = Ig domain 3 of Flt4 (VEGFR3)) were much less sticky to ECM, as judged by an *in vitro* ECM binding assay as described *infra ,* had greatly improved PK as described *infra.* In addition, these molecules were able to bind VEGF tightly as described *infra* and block phosphorylation of the native Flk1 receptor expressed in endothelial cells as described *infra.*

### (a) Construction of the expression plasmid pFlt1 D2.Flk1D3.FcΔC1(a)

Expression plasmids pMT21.Flt1(1-3).Fc (6519bp) and pMT21.Flk-1(1-3).Fc (5230bp) are plasmids that encode ampicillin resistance and Fc-tagged versions of Ig domains 1-3 of human Flt1 and human Flk1, respectively. These plasmids were used to construct a DNA fragment consisting of a fusion of Ig domain 2 of Flt1 with Ig domain 3 of Flk1, using PCR amplification of the respective Ig domains followed by further rounds of PCR to achieve fusion of the two domains into a single fragment. For Ig domain 2 of Flt1, the 5' and 3' amplification primers were as follows:
5': bsp/flt1D2 (5'-GACTAGCAGTCCGGAGGTAGACCTTTCGTAGAGATG-3')
3': Flt1D2-Flk1D3.as (5'-CGGACTCAGAACCACATCTATGATTGTATTGGT-3')

The 5' amplification primer encodes a BspE1 restriction enzyme site upstream of Ig domain 2 of Flt1, defined by the amino acid sequence GRPFVEM (corresponding to amino acids 27-33 of Figure 21A-21C). The 3' primer encodes the reverse complement of the 3' end of FIt1 Ig domain 2 fused directly to the 5' beginning of Flk1 Ig domain 3, with the fusion point defined as TIID of FIt1 (corresponding to amino acids 123-126 of Figure 21A-21C) and continuing into VVLS (corresponding to amino acids 127-130 of Figure 21A-21C) of Flk1.

For Ig domain 3 of Flk1, the 5' and 3' amplification primers were as follows:

The 5' amplification primer encodes the end of Flt1 Ig domain 2 fused directly to the beginning of Flk1 Ig domain 3, as described above. The 3' amplification primer encodes the end of Flk1 Ig domain 3, defined by the amino acids VRVHEK (corresponding to amino acids 223-228 of Figure 21A-21C), followed by a bridging sequence that includes a recognition sequence for the restriction enzyme Srf1, and encodes the amino acids GPG. The bridging sequence corresponds to amino acids 229-231 of Figure 21A-21C.

After a round of PCR amplification to produce the individual domains, the products were combined in a tube and subjected to a further round of PCR with the primers bsp/flt1D2 and Flk1D3/apa/srf.as (described *supra*) to produce the fusion product. This PCR product was subsequently digested with the restriction enzymes BspEI and Smal and the resulting 614bp fragment was subcloned into the BspEI to Srfl restriction sites of the vector pMT21/ΔB2.Fc, to create the plasmid pMT21/Flt1D2.Flk1D3.Fc. The nucleotide sequence of the Flt1D2-Flk1D3 gene fusion insert was verified by standard sequence analysis. This plasmid was then digested with the restriction enzymes EcoRI and Srfl and the resulting 702bp fragment was transferred into the EcoRI to Srfl restriction sites of the plasmid pFlt1(1-3)B2-FcΔC1(a) to produce the plasmid pFlt1D2.Flk1D3.FcΔC1(a). The complete DNA and deduced amino acid sequences of the Flt1D2.Flk1D3.FcΔC1(a) chimeric molecule is set forth in Figure 21A-21C.

### (b) Construction of the expression plasmid

### pFlt1 D2VEGFR3D3FcΔC1(a)

The expression plasmid pMT21.Flt1(1-3).Fc (6519bp) encodes ampicillin resistance and an Fc-tagged version of Ig domains 1-3 of human Flt1 receptor. This plasmid was used to produce a DNA fragment containing Ig domain 2 of Flt1 by PCR. RNA from the cell line HEL921.7 was used to produce Ig domain 3 of Flk1, using standard RT-PCR methodology. A further round of PCR amplification was used to achieve fusion of the two Ig domains into a single fused fragment. For Ig domain 2 of Flt1, the 5' and 3' amplification primers were as follows:
5': bsp/flt1D2 (5'-GACTAGCAGTCCGGAGGTAGACCTTTCGTAGAGATG-3')

The 5' amplification primer encodes a BspE1 restriction site upstream of Ig domain 2 of Flt1, defined by the amino acid sequence GRPFVEM (corresponding to amino acids 27-33 of Figure 22A-22C). The 3' amplification primer encodes the reverse complement of the end of Flt1 Ig domain 2 fused directly to the beginning of VEGFR3 Ig domain 3, with the fusion point defined as TIID of Flt1 (corresponding to amino acids 123-126 of Figure 22A-22C) and continuing into IQLL of VEGFR3 (corresponding to amino acids 127-130 of Figure 22A-22C).

For Ig domain 3 of VEGFR3, the 5' and 3' primers used for RT-PCR were as follows:
5': R3D3.s (ATCCAGCTGTTGCCCAGGAAGTCGCTGGAGCTGCTGGTA)
3': R3D3.as (ATTTTCATGCACAATGACCTCGGTGCTCTCCCGAAATCG)

Both the 5' and 3' amplification primers match the sequence of VEGFR3. The 296bp amplification product of this RT-PCR reaction was isolated by standard techniques and subjected to a second round of PCR to add suitable sequences to allow for fusion of the Flt1D2 with the Flk1D3 domains and fusion of the Flk1D3 and Fc domains via a GPG bridge (see below). The amplification primers were as follows:
5':Flt1D2.VEGFR3D3.s
   (TCATAGATATCCAGCTGTTGCCCAGGAAGTCGCTGGAG)
3': VEGFR3D3/srf.as
   (GATAATGCCCGGGCCATTTTCATGCACAATGACCTCGGT)

The 5' amplification primer encodes the 3' end of Flt1 Ig domain 2 fused directly to the beginning (5' end) of VEGFR3 lg domain 3, as described above. The 3' amplification primer encodes the 3' end of VEGFR3 Ig domain 3, defined by the amino acids VIVHEN (corresponding to amino acids 221-226 of Figure 22A-22C), followed by a bridging sequence that includes a recognition sequence for Srf1, and encodes the amino acids GPG. The bridging sequence corresponds to amino acids 227-229 of Figure 22A-22C.

After one round (for Flt1 Ig domain 2) or two rounds (for Flt4 Ig domain 3) of PCR to produce the individual Ig domains, the PCR products were combined in a tube and subjected to a further round of PCR amplification with the amplification primers bsp/flt1D2 and VEGFR3D3/srf.as described *supra,* to produce the fusion product. This PCR product was subsequently digested with the restriction enzymes BspEI and Smal and the resulting 625bp fragment was subcloned into the BspEI to Srfl restriction sites of the vector pMT21/Flt1ΔB2.Fc (described *supra*), to create the plasmid pMT21/Flt1D2.VEGFR3D3.Fc. The sequence of the Flt1D2-VEGFR3D3 gene fusion insert was verified by standard sequence analysis. This plasmid was then digested with the restriction enzymes EcoRI and Srfl and the resulting 693bp fragment was subcloned into the EcoRI to Srfl restriction sites of the plasmid pFlt1(1-3)ΔB2-FcΔC1(a) to produce the plasmid designated pFlt1D2.VEGFR3D3.FcΔC1(a). The complete DNA deduced amino acid sequence of the Flt1D2.VEGFR3D3.FcΔC1(a) chimeric molecule is set forth in Figure 22A-22C.

### Example 18: Extracellular Matrix Binding (ECM) Binding Assay

ECM-coated plates (Becton Dickinson catalog # 35-4607) were rehydrated with warm DME supplemented with glutamine (2mM), 100U penicillin, 100U streptomycin, and 10% BCS for at least 1 hr. before adding samples. The plates were then incubated for 1 hr. at room temperature with varying concentrations of Flt1D2.Flk1D3.FcΔC1(a) and Flt1D2.VEGFR3D3.FcΔC1(a) starting at 10nM with subsequent 2-fold dilutions in PBS plus 10% BCS. The plates were then washed 3 times with PBS plus 0.1% Triton-X and incubated with alkaline phosphatase-conjugated anti-human Fc antibody (Promega, 1:4000 in PBS plus 10% BCS) for 1 hr. at room temperature. The plates were then washed 4 times with PBS 0.1% Triton-X and alkaline phosphatase buffer/pNPP solution (Sigma) was added for color development. Plates were read at I = 405-570nm. The results of this experiment are shown in Figure 23 and demonstrate that the Flt1D2.Flk1D3.FcΔC1(a) and Flt1D2.VEGFR3D3.FcΔC1(a) proteins are considerably less sticky to the ECM as compared to the Flt1(1-3)-Fc protein.

### Example 19: Transient expression of pFlt1D2.Flk1D3.FcΔC1(a) in CHO-K1 (E1A) cells

A large scale (2L) culture of E. coli DH10B cells carrying the pFlt1D2.Flk1D3.FcΔC1(a) plasmid described *supra* in Example 17(a) was grown overnight in Terrific Broth (TB) plus 100µg/ml ampicillin. The next day, the plasmid DNA was extracted using a QIAgen Endofree Megaprep kit following the manufacturer's protocol. The concentration of the purified plasmid DNA was determined by standard techniques using a UV spectrophotometer and fluorometer. The plasmid DNA was verified by standard restriction enzyme digestion of aliquots using the restriction enzymes EcoRI plus Notl and Asel. All restriction enzyme digest fragments corresponded to the predicted sizes when analyzed on a 1% agarose gel.

Forty 15 cm petri plates were seeded with CHO-K1/E1A cells at a density of 4 x 106 cells/plate. Plating media was Gibco Ham's F-12 supplemented with 10% Hyclone Fetal Bovine Serum (FBS), 100U penicillin/100U streptomycin and glutamine (2mM). The following day each plate of cells was transfected with 6 µg of the pFlt1 D2.Flk1D3.FcΔC1(a) plasmid DNA using Gibco Optimem and Gibco Lipofectamine in 12 ml volume, following the manufacturer's protocol. Four hours after adding the transfection mix to the cells, 12 ml/plate of Optimem supplemented with 10% FBS was added. Plates were incubated at 37°C in a 5% CO₂ incubator overnight. The following day the media was removed from each plate and 25 ml expression media (Gibco CHO-S-SFM II supplemented with glutamine (2mM) and 1 mM sodium butyrate) was added. The plates were incubated at 37°C for 3 days. After 3 days of incubation, the media was aspirated from each plate and centrifuged at 400 rpm in a swinging bucket rotor to pellet cells. The supernatant was decanted into sterile 1 L bottles and purification of the expressed protein was performed as described *infra.*

### Example 20: Construction pVEGFR1R2-FcΔC1(a) expression vector

The pVEGFR1R2.FcΔC1(a) expression plasmid was constructed by insertion of DNA encoding amino acids SDT (corresponding to amino acids 27-29 of Figure 24A-24C) between Flt1d2-Flk1d3-FcΔC1(a) amino acids 26 and 27 of Figure 21A-21C (GG) and removal of DNA encoding amino acids GPG corresponding to amino acids 229-231 of Figure. The SDT amino acid sequence is native to the Flt1 receptor and was added back in to decrease the likelihood of heterogeneous N-terminal processing. The GPG (bridging sequence) was removed so that the Flt1 and Flk1 Ig domains were fused directly to one another. The complete DNA and deduced amino acid sequences of the pVEGFR1R2.FcΔC1(a) chimeric molecule is set forth in Figure 24A-24C.

### Example 21: Cell Culture Process Used to Produce Modified Flt1 Receptors

### (a) Cell Culture Process Used to Produce Flt1 D2.Flk1 D3.FcΔC1 (a)

The process for production of Flt1D2.Flk1D3.FcΔC1(a) protein using the expression plasmid pFlt1D2.Flk1D3.FcΔC1(a) described *supra* in Example 1 involves suspension culture of recombinant Chinese hamster ovary (CHO K1/E1A) cells which constitutively express the protein product. The cells are grown in bioreactors and the protein product is isolated and purified by affinity and size exclusion chromatography. The process is provided in greater detail below.

### Cell Expansion

Two confluent T-225 cm² flasks containing the Flt1D2.Flk1D3.FcΔC1(a) expressing cell line were expanded by passaging cells into eight T-225 cm² flasks in medium (GMEM + 10% serum, GIBCO) and incubated at 37°C and 5% CO₂. When the flasks approached confluence (approximately 3 to 4 days) the cells were detached using trypsin. Fresh medium was added to protect the cells from further exposure to the trypsin. The cells were centrifuged and resuspended in fresh medium then transferred to eight 850 cm² roller bottles and incubated at 37°C and 5% CO₂ until confluent.

### Suspension Culture in Bioreactors

Cells grown in roller bottles were trypsinized to detach them from the surface and washed with suspension culture medium. The cells are aseptically transferred to a 5L bioreactor (New Brunswick Celligen Plus) where the cells are grown in 3.5L of suspension culture. The suspension culture medium was a glutamine-free low glucose modification of IS-CHO (Irvine Scientific) to which 5% fetal bovine serum (Hyclone), GS supplement (Life Technologies) and 25 µM methionine sulfoximine (Sigma) was added. The pH was controlled at 7.2 by addition of carbon dioxide to the inlet gas or by addition of a liquid solution of sodium carbonate to the bioreactor. Dissolved oxygen level was maintained at 30% of saturation by addition of oxygen or nitrogen to the inlet gas and temperature controlled at 37°C. When a density of 4 x10⁶ cells/mL was reached the cells were transferred to a 40L bioreactor containing the same medium and setpoints for controlling the bioreactor. The temperature setpoint was reduced to 34°C to slow cell growth and increase the relative rate of protein expression.

### (b) Cell Culture Process Used to Produce Flt1D2.VEGFR3D3.FcΔC1(a)

The same methodologies as described *supra* for Flt1D2.Flk1D3.FcΔC1(a) were used to produce Flt1D2.VEGFR3D3.FcΔC1(a).

### Example 22: Harvest and Purification of Modified Flt1 Receptors

### (a) Harvest and Purification of Flt1D2.Flk1D3.FcΔC1(a)

The product protein was aseptically harvested from the bioreactor while retaining cells using Millipore Prostak tangential-flow filtration modules and a low-shear mechanical pump (Fristam). Fresh medium was added to the bioreactor to replace that removed during the harvest filtration. Approximately 40L of harvest filtrate was then loaded onto a 400 mL column containing Protein A Sepharose resin (Amersham Pharmacia). After loading the resin was washed with buffer containing 10 mM sodium phosphate, 500 mM sodium chloride, pH 7.2 to remove any unbound contaminating proteins. Flt1D2.Flk1D3.FcΔC1(a) protein was eluted with a pH 3.0 citrate buffer. The eluted protein was neutralized by addition of Tris base and frozen at -20°C.

Several frozen lots of Flt1D2.Flk1D3.FcΔC1(a) protein from the Protein A step above were thawed, pooled and concentrated using a Millipore 30kD nominal molecular weight cutoff (NMWCO) tangential flow filtration membrane. The protein was transferred to a stirred cell concentrator (Millipore) and further concentrated to 30 mg/mL using a 30kD NMWCO membrane. The concentrated protein was loaded onto a size exclusion column packed with Superdex 200 resin (Amersham Pharmacia) that was equilibrated with phosphate buffered saline plus 5% glycerol. The same buffer was used to run the column. The fractions corresponding to Flt1D2.Flk1D3.FcΔC1(a) dimer were pooled, sterile filtered through a 0.22 micron filter, aliquoted and frozen.

### (b) Harvest and Purification of Flt1D2.VEGFR3D3.FcAC1(a)

The same methodologies as described *supra* for Flt1D2.Flk1D3.FcΔC1(a) were used to harvest and purify Flt1D2.VEGFR3D3.FcΔC1(a).

### Example 23: Phosphorylation Assay for Transiently Expressed VEGFR2

Primary human umbilical vein endothelial cells (HUVECs), passage 4-6, were starved for 2 hrs in serum-free DME high glucose media. Samples containing 40 ng/ml (1 nM) human VEGF165, which is a ligand for the VEGF receptors Flt1, Flk1 and Flt4(VEGFR3) were prepared and were preincubated for 1 hr. at room temperature with varying amounts of the modified Flt1 receptors Flt1(1-3)-Fc, Flt1(1-3)-Fc (A40), Flt1D2Flk1D3.FcΔC1(a) and Flt1D2VEGFR3D3.FcΔC1(a) in serum-free DME-high glucose media containing 0.1% BSA. Cells were challenged for 5 minutes with the samples prepared above +/- VEGF165, followed by whole cell lysis using complete lysis buffer. Cell lysates were immunoprecipitated with an antibody directed against the C-terminus of VEGFR2 receptor. The immunoprecipitated lysates were loaded onto 4-12% SDS-PAGE Novex gel and then transferred to PVDF membrane using standard transfer methodologies. Detection of phosphorylated VEGFR2 was done by immunoblotting with the anti-phospho Tyrosine mAb called 4G10 (UBI) and developed using ECL-reagent (Amersham).

Figures 25A-25C and 26A-26B show the results of this experiment. Figure 25A-25C reveals that detection by Western blot of tyrosine phosphorylated VEGFR2(Flk1) by VEGF165 ligand stimulation shows that cell-surface receptors are phosphorylated to varying levels depending on which modified Flt1 receptor is used during the preincubations with VEGF. As is seen in Figure 25A, at a 1.5 molar excess of either Flt1(1-3)-Fc , Flt1(1-3)-Fc (A40) or transient Flt1D2Flk1D3.FcΔC1(a) there is complete blockage of receptor stimulation by these three modified Flt1 receptors as compared to control media challenge. In contrast, transient Flt1D2VEGFR3D3.FcΔC1(a) does not show significant blockage at this molar excess, as compared with VEGF positive control challenge. Similar results are seen in Figure 25B, where the modified Flt receptors are in a 3-fold molar excess to VEGF165 ligand. In Figure 25C, where the modified Flt1 receptors are in a 6-fold molar excess to VEGF165 ligand, transient Flt1D2VEGFR3D3.FcΔC1(a) can now be shown to be partially blocking VEGF165-induced stimulation of cell-surface receptors.

In Figure 26A-26B, detection by Western blot of tyrosine phosphorylated VEGFR2(Flk1) by VEGF165 ligand stimulation shows that cell-surface receptors are not phosphorylated by challenge samples which have VEGF165 preincubated with 1 and 2 fold molar excess (Figure 26A) or 3 and 4 fold molar excess (Figure 26B) of either transient Flt1D2Flk1D3.FcΔC1(a), stable Flt1D2Flk1D3.FcΔC1(a), or transient VEGFR1R2-FcΔC1(a). At all modified Flt1 receptor concentrations tested there is complete binding of VEGF165 ligand during the preincubation, resulting in no detectable stimulation of cell-surface receptors by unbound VEGF165 as compared to control media challenge.

### Example 24: Cell Proliferation Bioassay

The test cell population is MG87 cells that have been stably transfected with a expression plasmid that contains a DNA insert encoding the VEGFR2(Flk1) extracellular domain fused to the TrkB intracellular kinase domain, thus producing a chimeric molecule. The reason the TrkB intracellular kinase domain was used rather than the native VEGFR2(Flk1) intracellular kinase domain is that the intracellular kinase domain of VEGFR2(Flk1) does not cause a strong proliferative response when stimulated by VEGF165 in these cells. It is known that MG87 cells containing full length TrkB receptor give a robust proliferative response when stimulated with BDNF, so the TrkB intracellular kinase domain was engineered to replace the intracellular kinase domain of VEGFR2(Flk1) to take advantage of this proliferative response capability.

5 x 10³ cells/well were plated in a 96 well plate and allowed to settle for 2 hrs at 37°C. The following modified Flt receptors Flt1(1-3)-Fc, Flt1D2.Flk1D3.FcΔC1(a) and Flt1D2.VEGFR3D3.FcΔC1(a), plus an irrelevant receptor termed Tie2-Fc as a negative control, were titrated from 40nM to 20pM and incubated on the cells for 1hr at 37°C. Human recombinant VEGF165 in defined media was then added to all the wells at a concentration of 1.56nM. The plates were incubated for 72 hrs at 37°C and then MTS (Owen's reagent, Promega) added and the plates were incubated for an additional for 4 hrs. Finally, the plates were read on a spectrophotometer at 450/570nm. The results of this experiment are shown in Figure 27. The control receptor Tie2-Fc does not block VEGF165-induced cell proliferation at any concentration whereas Flt1D2.Flk1D3.FcΔC1(a) blocks 1.56nM VEGF165 with a half maximal dose of 0.8nM. Flt1(1-3)-Fc and Flt1D2.VEGFR3D3.FcΔC1(a) are less effective in blocking VEGF165 in this assay with a half maximal dose of - 2nM. VEGF165 alone gives a reading of 1.2 absorbance units and the background is 0.38 absorbance units.

### Example 25: Binding Stoichiometry of Modified Flt Receptors to VEGF165

### (a) BIAcore Analysis

The stoichiometry of Flt1D2Flk1D3.FcΔC1(a) and VEGFR1R2-FcΔC1(a) interaction with human VEGF165 was determined by measuring either the level of VEGF saturation binding to the Flt1D2Flk1D3.FcΔC1(a) or VEGFR1R2-FcΔC1(a) surfaces or measuring concentration of VEGF165 needed to completely prevent binding of Flt1D2Flk1D3.FcΔC1(a) or VEGFR1R2-FcΔC1(a) to VEGF BIAcore chip surface.

Modified Flt receptors Flt1D2Flk1D3.FcΔC1(a) and VEGFR1R2-FcΔC1(a). were captured with an anti-Fc specific antibody that was first immobilized on a Biacore chip (BIACORE) using amine-coupling chemistry. A blank antibody surface was used as a negative control. VEGF165 was injected at a concentration of 1 nM, 10 nM, and 50 nM over the Flt1D2Flk1D3.FcΔC1(a) and VEGFR1R2-FcΔC1(a) surfaces at 10 µl/min for one hour. A real-time binding signal was recorded and saturation binding was achieved at the end of each injection. Binding stoichiometry was calculated as a molar ratio of bound VEGF165 to the immobilized Flt1D2Flk1D3.FcΔC1(a) or VEGFR1R2-FcΔC1(a), using the conversion factor of 1000 RU equivalent to 1 ng/ml. The results indicated binding stoichiometry of one VEGF165 dimeric molecule per one Flt1D2Flk1D3.FcΔC1(a) or VEGFR1R2-FcΔC1(a) molecule (Figure 28).

In solution, Flt1D2Flk1D3.FcΔC1(a) or VEGFR1R2-FcΔC1(a) at a concentration of 1nM (estimated to be 1000 times higher than the KD of the Flt1D2Flk1D3.FcΔC1(a) or VEGFR1R2-FcΔC1(a)/VEGF165 interaction) were mixed with varied concentrations of VEGF165. After one hour incubation, concentrations of the free Flt1D2Flk1D3.FcΔC1(a) in solution were measured as a binding signal to an amine-coupled VEGF165 surface. A calibration curve was used to convert the Flt1D2Flk1D3.FcΔC1(a) BIAcore binding signal to its molar concentration. The data showed that the addition of 1 nM VEGF165 into the Flt1D2Flk1D3.FcΔC1(a) solution completely blocked Flt1D2Flk1D3.FcΔC1(a) binding to the VEGF165 surface. This result suggested the binding stoichiometry of one VEGF165 molecule per one Flt1D2Flk1D3.FcΔC1(a) molecule (Figure 29 and Figure 30). When the concentration of Flt1D2Flk1D3.FcΔC1(a) was plotted as a function of added concentration of VEGF165, the slope of the linear portion was - 1.06 for Flt1D2Flk1D3.FcΔC1(a) and -1,07 for VEGFR1R2-FcΔC1(a). The magnitude of the slope, very close to negative one, was indicative that one molecule of VEGF165 bound to one molecule of either Flt1D2Flk1D3.FcΔC1(a) or VEGFR1R2-FcΔC1(a).

### (b) Size Exclusion Chromatography

Flt1D2Flk1D3.FcΔC1(a) was mixed with a 3-fold excess of VEGF165 and the receptor-ligand complex was purified using a Pharmacia Superose 6 size exclusion chromatography column. The receptor-ligand complex was then incubated in a buffer containing 6M guanidine hydrochloride in order to dissociate it into its component proteins. Flt1D2Flk1D3.FcΔC1(a) was separated from VEGF165 using Superose 6 size exclusion chromatography column run in 6M guanidium chloride. In order to determine complex stoichiometry, several injections of Flt1D2Flk1D3.FcΔC1(a) and VEGF165 were made and peak height or peak integrated intensity was plotted as a function of the concentration of injected protein. The calibration was done under condition identical to one used in separating components of Flt1D2Flk1D3.FcΔC1(a)/VEGF complex. Quantification of the Flt1D2Flk1D3.FcΔC1(a)/VEGF complex composition was based on the calibration curves. The results of this experiment are set forth in Figure 28, which shows the ratio of VEGF165 to Flt1D2Flk1D3.FcΔC1(a) in a complex to be 1:1.

### Example 26: Determination of the Binding Stoichiometry of Flt1D2Flk1D3.FcΔC1(a)/VEGF165 Complex by Size Exclusion Chromatography

### Flt1D2Flk1D3.FcΔC1(a)/VEGF165 Complex Preparation

VEGF165 (concentration = 3.61 mg/ml) was mixed with CHO cell transiently expressed Flt1D2.Flk1D3.FcΔC1(a) (concentration = 0.9 mg/ml) in molar ratio of 3:1 (VEGF165:Flt1D2.Flk1D3.FcΔC1(a)) and incubated overnight at 4°C.

### (a) Size Exclusion Chromatography (SEC) under native conditions

To separate the complex from excess of unbound VEGF165, 50 µl of the complex was loaded on a Pharmacia Superose 12 PC 3.2/30 which was equilibrated in PBS buffer. The sample was eluted with the same buffer at flow rate 40µl/min, at room temperature. The results of this SEC are shown in Figure 31. Peak #1 represents the complex and peak #2 represents unbound VEGF165. Fractions eluted between 1.1 and 1.2 ml were combined and guanidinium hydrochloride (GuHCl) was added to a final concentration 4.5M to dissociate the complex.

### (b) Size Exclusion Chromatography (SEC) under dissociative conditions

To separate the components of the receptor-ligand complex and to determine their molar ratio, 50µl of dissociated complex as described *supra* was loaded onto a Superose 12 PC 3.2/30 equilibrated in 6M GuHCl and eluted with the same solution at a flow rate 40µl/min. at room temperature. The results of this SEC are shown in Figure 32. Peak #1 represents Flt1D2Flk1D3.FcΔC1(a) and peak #2 represents VEGF165.

### (c) Calculation of Flt1D2Flk1D3.FcΔC1(a):VEGF165 Complex Stoichiometry

The stoichiometry of the receptor-ligand complex was determined from the peak area or the peak height of the components. Concentrations of VEGF165 and Flt1D2Flk1D3.FcΔC1(a) corresponding to the peak height or peak area, respectively, were obtained from the standard curves for VEGF165 and Flt1D2Flk1D3.FcΔC1(a). To obtain a standard curve, four different concentrations (0.04 mg/ml -0.3mg/ml) of either component were injected onto a Pharmacia Superose 12 PC 3.2/30 column equilibrated in 6M guanidinium chloride and eluted with the same solution at flow rate 40µl/min. at room temperature. The standard curve was obtained by plotting peak area or peak height vs protein concentration. The molar ratio of VEGF165:Flt1D2Flk1D3.FcΔC1(a) determined from the peak area of the components was 1.16. The molar ratio of VEGF165:Flt1D2Flk1D3.FcΔC1(a) determined from the peak height of the components was 1.10.

### Example 27: Determination of the Stoichiometry of the Flt1D2Flk1D3.FcΔC1(a)/VEGF165 Complex by Size Exclusion Chromatography with On-Line Light Scattering

### Complex preparation

VEGF165 was mixed with CHO transiently expressed Flt1D2.Flk1D3.FcΔC1(a) protein in molar ratio of 3:1 (VEGF165:Flt1D2Flk1D3.FcΔC1(a)) and incubated overnight at 4°C.

### (a) Size Exclusion Chromatography (SEC) with On-Line Light Scattering

Size exclusion chromatography column with a MiniDawn on-line light scattering detector (Wyatt Technology, Santa Barbara, California) and refractive index (RI) detectors (Shimadzu, Kyoto, Japan) was used to determine the molecular weight (MW) of the receptor-ligand complex. Samples were injected onto a Superose 12 HR 10/30 column (Pharmacia) equilibrated in PBS buffer and eluted with the same buffer at flow rate 0.5 ml/min. at room temperature. As shown in Figure 33, the elution profile shows two peaks. Peak #1 represents the receptor-ligand complex and peak #2 represents the unbound VEGF165. MW was calculated from LS and RI signals. The same procedure was used to determine MW of the individual components of the receptor-ligand complex. The results of these determinations are as follows: MW of the Flt1D2Flk1D3.FcΔC1(a)/VEGF165 complex at the peak position is 157 300 (Figure 33), the MW of VEGF165 at the peak position is 44 390 (Figure 34) and the MW of R1 R2 at the peak is 113 300 (Figure 35).

These data indicated that the stoichiometry of the Flt1D2Flk1D3.FcΔC1(a)/VEGF complex is 1:1 as its corresponds to the sum of molecular weights for Flt1D2Flk1D3.FcΔC1(a) and VEGF165. Importantly, this method conclusively proved that the Flt1D2Flk1D3.FcΔC1(a)/VEGF165 complex was indeed composed of only one molecule of VEGF165 ligand and only one molecule of the Flt1D2Flk1D3.FcΔC1(a).

### Example 28: Peptide Mapping of Flt1D2.Flk1D3.FcΔC1(a)

The disulfide structures and glycosylation sites in Flt1D2.Flk1D3.FcΔC1(a) were determined by a peptide mapping method. In this method, the protein was first cleaved with trypsin. Tryptic fragments were analyzed and identified by HPLC coupled with mass spectrometry, in addition to an N-terminal sequencing technique. Reduction of the tryptic digest was employed to help identify disulfide-bond-containing fragments. Treatment of the tryptic digest with PNGase F (Glyko, Novato, CA) was employed to help identify fragments with N-linked glycosylation sites. The results are summarized in the accompanying Figure 36.

There are a total of ten cysteines in Flt1 D2.Flk1D3.FcΔC1(a); six of them belong to the Fc region. Cys27 has been confirmed to be disulfide bonded to Cys76. Cys121 is confirmed to be disulfide bonded to Cys 182. The first two cysteines in the Fc region (Cys211 and Cys214) form an intermolecular disulfide bond with the same two cysteines in another Fc chain. However, because these two cysteines can not be separated enzymatically from each other, it can not be determined whether disulfide bonding is occurring between same cysteines (Cys211 to Cys211, for example) or between Cys211 and Cys214. Cys216 is confirmed to be disulfide bonded to Cys306. Cys 352 is confirmed to be disulfide bonded to Cys410.

There are five possible N-linked glycosylation sites in Flt1D2.Flk1D3.FcΔC1(a). All five of them are found to be glycosylated to varying degrees. Complete glycosylation was observed at Asn33 (amino acid sequence NIT), Asn193 (amino acid sequence NST), and Asn282 (amino acid sequence NST). In addition, partial glycosylation is observed on Asn65 and Asn120. Sites of glycosylation are highlighted by underline in the Figure 36.

### Example 29: Pharmacokinetic Analysis of Modified Flt Receptors

### (a) Pharmacokinetic analysis of Flt1(1-3)-Fc (A40), Flt1D2.Flk1D3.FcΔC1(a) and VEGFR1R2-FcΔC1(a)

Balb/c mice (25-30g) were injected subcutaneously with 4mg/kg of Flt1(1-3)-Fc (A40), CHO transiently expressed Flt1D2.Flk1D3.FcΔC1(a), CHO stably expressed Flt1D2.Flk1D3.FcΔC1(a), and CHO transiently expressed VEGFR1R2-FcΔC1(a). The mice were tail bled at 1, 2, 4, 6, 24hrs, 2 days, 3 days and 6 days after injection. The sera were assayed in an ELISA designed to detect Flt1(1-3)-Fc (A40), Flt1D2.Flk1D3.FcΔC1(a) or VEGFR1R2-FcΔC1(a). The ELISA involves coating an ELISA plate with VEGF165, binding the detect Flt1(1-3)-Fc (A40), Flt1D2.Flk1D3.FcΔC1(a) or VEGFR1R2-FcΔC1(a) and reporting with an anti-Fc antibody linked to horse radish peroxidase. The results of this experiments are shown in Figure 37. The Tₘₐₓ for Flt1(1-3)-Fc (A40) was at 6 hrs while the Tₘₐₓ for the transient and stable Flt1D2.Flk1D3.FcΔC1(a) and the transient VEGFR1R2-FcΔC1(a) was 24hrs. The Cₘₐₓ for Flt1(1-3)-Fc (A40) was 8µg/ml. For both transients (Flt1D2.Flk1D3.FcΔC1 (a) and VEGFR1R2-FcΔC1(a)) the Cₘₐₓ was 18µg/ml and the Cₘₐₓ for the stable VEGFR1R2-FcΔC1(a) was 30µg/ml.

### (b) Pharmacokinetic analysis of Flt1(1-3)-Fc (A40). Flt1D2.Flk1D3.FcΔC1(a) and Flt1D2.VEGFR3D3.FcΔC1(a)

Balb/c mice (25-30g) were injected subcutaneously with 4mg/kg of Flt1(1-3)-Fc (A40), CHO transiently expressed Flt1D2.Flk1D3.FcΔC1(a) and CHO transiently expressed Flt1D2.VEGFR3D3.FcΔC1(a). The mice were tail bled at 1, 2, 5, 6, 7, 8, 12, 15 and 20 days after injection. The sera were assayed in an ELISA designed to detect Flt1(1-3)-Fc, Flt1 D2.Flk1 D3.FcΔC1(a) and Flt1D2.VEGFR3D3.FcΔC1(a). The ELISA involves coating an ELISA plate with 165, binding the Flt1(1-3)-Fc, Flt1 D2.Flk1D3.FcΔC1(a) or Flt1D2.VEGFR3D3.FcΔC1(a) and reporting with an anti-Fc antibody linked to horse radish peroxidase. Flt1(1-3)-Fc (A40) could no longer be detected in the serum after day 5 whereas , Flt1D2.Flk1D3.FcΔC1(a) and Flt1D2.VEGFR3D3.FcΔC1 (a) were detectable for 15 days or more. The results of this experiment are shown in Figure 38.

### Example 30: Evaluation of the Ability of Flt1D2.Flk1D3.FcΔC1(a) to Inhibit Tumor Growth In Vivo

To evaluate the ability of Flt1D2.Flk1D3.FcΔC1(a) to inhibit tumor growth in vivo a model in which tumor cell suspensions are implanted subcutaneously on the right flank of male severe combined immunodeficiency (SCID) mice was employed. Two cell lines, the human HT-1080 fibrosarcoma cell line (ATCC accession no. CCL-121) and the rat C6 glioma cell line (ATCC accession no. CCL-107), each of which exhibit distinctly different morphologies and growth characteristics, were used in the assay. The first dose of Flt1D2.Flk1D3.FcΔC1(a) (at 25mg/Kg or as indicated in Figures 39 and 40) was given on the day of tumor implantation. Animals subsequently received subcutaneous injections of Flt1(1-3)-Fc (A40), Flt1D2.Flk1D3.FcΔC1(a) or vehicle either every other day (EOD) or two times per week (2X/wk) for a period of 2 weeks. After 2 weeks, animals were perfused with fixative, tumors were removed and samples were blinded. Tumor volume was determined by measuring the length and width of visible subcutaneous tumors. Both of Flt1(1-3)-Fc (A40) and Flt1D2.Flk1D3.FcΔC1(a) significantly reduced the growth of tumors formed by HT-1080 and C6 cells. The results of these experiments are shown in Figure 39 and Figure 40.

### Example 31: The Effect of VEGF165 and Modified Flt Receptors in Female Reproductive System

The stereotypic pattern of vascular remodeling which occur in the uterus and ovary over the course of the reproductive cycle has been well characterized, making these tissues particularly well suited to the study of mechanisms which regulate angiogenesis, vascular remodeling and vascular regression. Indeed, *in situ* hybridization studies in the reproductive tissues provided the first clear evidence that VEGF acts as a mediator of physiological angiogenesis in mature rodents, as well as humans and non-human primates (Phillips et al, 1990; Ravindranath et al, 1992; Shweiki et al, 1993; Kamat et al, 1995). As cyclic angiogenesis and vascular remodeling are prominent features of the normal ovary and uterus, it is not surprising that abnormal blood vessel growth and/or vascular dysfunction have been found to characterize many pathological conditions which affect these organs. Furthermore, these pathogenic vascular abnormalities are thought to be caused or perpetuated by the dysregulated expression of one or more angiogenic or anti-angiogenic factors, most prominently VEGF.

For example, abnormal angiogenesis is characteristic of polycystic ovary disease, endometriosis and endometrial carcinoma, and in each case VEGF is over expressed in the affected tissue (Kamat et al, 1995; Shifren et al, 1996; Guidi et al, 1996; Donnez et al, 1998). Overexpression of VEGF is also thought to play a pathogenic role in the establishment of systemic vascular hyperpermeability in ovarian hyperstimulation syndrome (McClure et al, 1994; Levin et al, 1998) and preeclampsia (Baker et al, 1995; Sharkey et al, 1996). In addition, VEGF has been implicated as the permeability factor responsible for the production of ascites associated with ovarian carcinoma and other tumors (Senger et al, 1983; Boocock et al, 1995). Agents which effectively neutralize the biological actions of VEGF can reasonably be anticipated to be of therapeutic benefit in the above and related conditions.

Angiogenesis and vascular remodeling are also hallmarks of blastocyst implantation and placental development (Findlay, 1986). VEGF is strongly expressed both in the maternal decidua and in embryonic trophoblasts, where it is thought to first stimulate expansion and hyperpermeability of the uterine vasculature during the peri-implantation period and subsequently mediate formation of both the maternal and embryonic components of the placental vasculature (Shweiki et al, 1993; Cullinan-Bove and Koos, 1993; Chakraborty et al, 1995; Das et al, 1997). VEGF is also required for luteal angiogenesis and associated progesterone secretion necessary to prepare the uterus for implantation (Ferrara et al, 1998). Thus, agents which inhibit the biological actions of VEGF may prove to be useful as contraceptive agents (by preventing implantation), or as an abortifacients in the early stages of gestation. The latter application might find particular use as a non-surgical intervention for the termination of ectopic pregnancies.

While the expression of VEGF receptors is largely confined to the vascular endothelium in normal reproductive tissues, Flt1 is also expressed by trophoblasts in the placenta in both humans and animals (Clark et al, 1996; He et al, 1999) where it has been proposed to play a role in trophoblast invasion. Interestingly, both Flt1 and KDR (Flk1) are expressed by choriocarcinoma cell line BeWo (Charnock-Jones et al, 1994), and VEGF has been shown to promote DNA synthesis and tyrosine phosphorylation of MAP kinase in these cells. Furthermore, primary and metastatic ovarian carcinomas not only to express high levels of VEGF, but - in addition to the vascular endothelium - the tumor cells themselves express KDR and/ or Flt1 (Boocock et al, 1995). These findings suggest that VEGF may not only be critically involved in the generation and maintenance of tumor vasculature, but that at least in some tumors of reproductive origin VEGF may subserve an autocrine role, directly supporting the survival and proliferation of the tumor cells. Thus agents which block the actions of VEGF may have particularly beneficial applications to the treatment of tumors of reproductive origin.

### Methods and Results

### (a) Assessment of VEGF-Induced Uterine Hyperpermeability

Pregnant mare's serum gonadotrophin (PMSG) was injected subcutaneously (5 IU) to induce ovulation in prepubertal female rats. This results in a surge of estradiol after 2 days which in turn causes an induction of VEGF in the uterus. It is reported that this induction results in hyperpermeability of the uterus and an increase in uterine wet weight 6 hrs. later and, therefore, could potentially be blocked by the modified Flt receptors Flt1(1-3)-Fc (A40), Flt1D2.Flk1D3.FcΔC1(a) and Flt1D2.VEGFR3D3.FcΔC1(a). In this in vivo model, the normal weight of the rat uterus is about 50 mg and this can be induced to 300-350 mg by PMSG. Desiccation of the tissue reveals that this is all water weight. Subcutaneous injection of Flt1(1-3)-Fc (A40), Flt1D2.Flk1D3.FcΔC1(a) and Flt1D2.VEGFR3D3.FcΔC1(a) at 25mg/kg at 1 hr. after PMSG injection results in about a 50% inhibition of the increase in uterine wet weight. Increasing the dose of modified Flt receptor does not further reduce the increase in wet weight suggesting that there is a VEGF-independent component to this model. The results of this experiment are shown in Figure 41.

### (a) Assessment of corpus luteum angiogenesis using progesterone as a readout

Pregnant mare's serum gonadotrophin (PMSG) is injected subcutaneously (5 IU) to induce ovulation in prepubertal female rats. This results in a fully functioning corpus luteum containing a dense network of blood vessels after 4 days that allows for the secretion of progesterone into the blood stream in order to prepare the uterus for implantation. The induction of angiogenesis in the corpus luteum requires VEGF; therefore, blocking VEGF would result in a lack of new blood vessels and thus a lack of progesterone secreted into the blood stream. In this in vivo model, resting levels of progesterone are about 5ng/ml and this can be induced to a level of 25-40ng/ml after PMSG. Subcutaneous injection of Flt1(1-3)-Fc (A40) or Flt1D2.Flk1D3.FcΔC1(a) at 25mg/kg or 5mg/kg at 1hr. after PMSG injection results in a complete inhibition of the progesterone induction on day 4. The results of this experiment are shown in Figure 42A-42B.

### Example 33: Pharmacokinetic Analysis of Flt1(1-3)-Fc (A40) and Pegylated Flt1(1-3)-Fc

Flt1(1-3)-Fc was PEGylated with either 10kD PEG or 20kD PEG and tested in balb/c mice for their pharmacokinetic profile. Both PEGylated forms of Flt1(1-3)-Fc were found to have much better PK profiles than Flt1(1-3)-Fc (A40), with the Tmax occurring at 24 hrs. for the PEGylated molecules as opposed to 6 hrs. for Flt1(1-3)-Fc (A40).

### Example 34: VEGF165 ELISA to Test Affinity of Modified Flt1 Receptor Variants

10pM of VEGF165 was incubated overnight at room temperature with modified Flt1 receptor variants ranging from 160pM to 0.1 pM. The modified Flt1 receptor variants used in this experiment were Flt1(1-3)-Fc, Flt1(1-3)-Fc (A40), transiently expressed Flt1D2Flk1D3.FcΔC1(a), transiently expressed Flt1D2VEFGFR3D3-FcΔC1(a), Flt1-(1-3_{NAS})-Fc, Flt1(1-3_{R->C})-Fc and Tie2-Fc. Flt1(1-3 _{NAS})-Fc is a modified version of Flt1(1-3)-Fc in which the highly basic amino acid sequence KNKRASVRRR is replaced by NASVNGSR, resulting in the incorporation of two new glycosylation sites and a net reduction of five positive charges, both with the purpose of reducing the unfavorable effects of this sequence on PK. Flt1(1-3 _{R->C})-Fc is a modification in which a single arginine (R) residue within the same basic amino acid sequence is changed to a cysteine (C) (KNKRASVRRR -> KNKCASVRRR) to allow for pegylation at that residue, which could then shield the basic region from exerting its unfavorable effects on PK. After incubation the solution was transferred to a plate containing a capture antibody for VEGF165 (R&D). The amount of free VEGF165 was then determined using an antibody to report free VEGF165. This showed that the modified Flt1 receptor variant with the highest affinity for VEGF165 (determined as the lowest amount of free VEGF165) was Flt1D2Flk1D3.FcΔC1(a), followed by Flt1(1-3)-Fc and Flt1(1-3)-Fc (A40) and then by Flt1(1-3_{R->C})-Fc, Flt1(1-3_{NAS})-Fc and Flt1D2VEFGFR3D3-FcΔC1(a). Tie2Fc has no affinity for VEGF165.

## Claims

1. Use of a fusion polypeptide capable of binding vascular endothelial growth factor (VEGF) in the manufacture of a medicament for treating an eye disorder **characterized by** vascular permeability wherein the fusion polypeptide comprises:
(a) a VEGF receptor component consisting essentially of an immunoglobulin-like (Ig) domain 2 of a first VEGF receptor and Ig domain 3 of a second VEGF receptor and
(b) a multimerizing component;
and wherein the first VEGF receptor is Flt1, the second VEGF receptor is Flk1 or Flt4, and the VEGF receptor component is the only VEGF receptor component of the fusion polypeptide.

2. Use according to claim 1, wherein the fusion polypeptide is arranged as 1,2,3; 1,3,2; 2,1,3; 2,3,1; 3,1,2; or 3,2,1, wherein 1 is the first VEGF receptor component, 2 is the second VEGF receptor component, and 3 is the multimerizing component.

3. Use according to claim 1 or 2 wherein the multimerizing component comprises an immunoglobulin domain.

4. Use according to claim 3 wherein the immunoglobulin domain is the Fc domain of IgG or the heavy chain of IgG.

5. Use according to any one of the preceding claims, wherein the fusion polypeptide is encoded by a nucleic acid sequence as set forth in (a) Figures 21A-21C, (b) Figures 22A-22C, (c) Figures 24A-24C or a nucleotide sequence which, as a result of the degeneracy of the genetic code, differs from the nucleotide sequence of (a), (b) or (c).

6. Use according to any one of the preceding claims wherein the fusion polypeptide comprises the amino acid sequence set forth in Figures 21A-21C, Figures 22A-22C or Figures 24A-24C.

7. Use according to claim 6, wherein the fusion polypeptide comprises the amino acid sequence set forth in Figures 24A-24C.

8. Use according to any one of the preceding claims, wherein the eye disorder is age related macular degeneration or diabetic retinopathy.

## Patentansprüche

1. Verwendung eines Fusionspolypeptids, das zur Bindung von vaskulärem endothelialem Wachstumsfaktor (VEGF) fähig ist, in der Herstellung eines Medikaments für die Behandlung einer Augenerkrankung, **gekennzeichnet durch** vaskuläre Permeabilität (Gefäßdurchlässigkeit), wobei das Fusionspolypeptid umfasst:
(a) eine VEGF-Rezeptorkomponente, bestehend im wesentlichen aus einer Immunglobulin-artigen (Ig) Domäne 2 eines ersten VEGF-Rezeptors; und lg Domäne 3 eines zweiten VEGF-Rezeptors; und
(b) eine multimerisierende Komponente;
und wobei der erste VEGF-Rezeptor Flt1 ist, der zweite VEGF-Rezeptor Flk1 ode Flt4 ist, und die VEGF-Rezeptorkomponente die einzige VEGF-Rezeptorkomponente des Fusionspolypeptids ist.

2. Verwendung nach Anspruch 1, wobei das Fusionspolypeptid als 1,2,3; 1,3,2; 2,1,3; 2,3,1; 3,1,2; oder 3,2,1 angeordnet ist, worin 1 die erste VEGF-Rezeptorkomponente ist, 2 die zweite VEGF-Rezeptorkomponente ist und 3 die multimerisierende Komponente ist.

3. Verwendung nach Anspruch 1 oder 2, wobei die multimerisierende Komponente eine Immunglobulin-Domäne umfasst.

4. Verwendung nach Anspruch 3, wobei die Immunglobulin-Domäne die Fc-Domäne von IgG oder die schwere Kette von IgG ist.

5. Verwendung nach einem der vorangehenden Ansprüche, wobei das Fusionspolypeptid durch eine Nukleinsäuresequenz codiert ist, wie angegeben in (a) Figuren 21A-21C, (b) Figuren 22A-22C, (c) Figuren 24A-24C, oder eine Nukleotidsequenz, die, als eine Folge der Degeneriertheit des genetischen Codes, von der Nukleotidsequenz von (a), (b) oder (c) abweicht.

6. Verwendung nach einem der vorangehenden Ansprüche, wobei das Fusionspolypeptid die Aminosäuresequenz umfasst, dargestellt in Figuren 21A-21C, Figuren 22A-22C oder Figuren 24A-24C.

7. Verwendung nach Anspruch 6, wobei das Fusionspolypeptid die Aminosäuresequenz umfasst, dargestellt in Figuren 24A-24C.

8. Verwendung nach einem der vorangehenden Ansprüche, wobei die Augenerkrankung altersbedingte Makuladegeneration oder diabetische Retinopathie ist.

## Revendications

1. Utilisation d'un polypeptide de fusion capable de se lier au facteur de croissance endothélial vasculaire (VEGF) dans la fabrication d'un médicament pour le traitement d'un trouble oculaire **caractérisé par** une perméabilité vasculaire, dans laquelle le polypeptide de fusion comprend :
(a) une constituant récepteur du VEGF constitué essentiellement d'un domaine 2 de type immunoglobuline (Ig) d'un premier récepteur du VEGF ; et d'un domaine 3 d'Ig d'un second récepteur du VEGF ; et
(b) d'un constituant de multimérisation ;
et dans laquelle le premier récepteur du VEGF est Flt1, le second récepteur du VEGF est Flk1 ou Flt4, et le constituant récepteur du VEGF est le seul constituant récepteur du VEGF du polypeptide de fusion.

2. Utilisation selon la revendication 1, dans laquelle le polypeptide de fusion est disposé en tant que 1,2,3 ; 1,3,2 ; 2,1,3 ; 2,3,1 ; 3,1,2 ; ou 3,2,1, où 1 est le premier constituant récepteur du VEGF, 2 est le second constituant récepteur du VEGF, et 3 est le constituant de multimérisation.

3. Utilisation selon la revendication 1 ou 2, dans laquelle le constituant de multimérisation comprend un domaine d'immunoglobuline.

4. Utilisation selon la revendication 3, dans laquelle le domaine d'immunoglobuline est le domaine Fc de l'IgG ou la chaîne lourde de l'IgG.

5. Utilisation selon l'une quelconque des revendications précédentes, dans laquelle le polypeptide de fusion est codé par une séquence d'acides nucléiques telle que présentée (a) dans les Figures 21A à 21C, (b) dans les Figures 22A à 22C, (c) dans les Figures 24A à 24C ou une séquence de nucléotides qui, du fait de la dégénérescence du code génétique, diffère de la séquence de nucléotides de (a), (b) ou (c).

6. Utilisation selon l'une quelconque des revendications précédentes, dans laquelle le polypeptide de fusion comprend la séquence d'acides aminés telle que présentée dans les Figures 21A à 21C, dans les Figures 22A à 22C ou dans les Figures 24A à 24C.

7. Utilisation selon la revendication 6, dans laquelle le polypeptide de fusion comprend la séquence d'acides aminés telle que présentée dans les Figures 24A à 24C.

8. Utilisation selon l'une quelconque des revendications précédentes, dans laquelle le trouble oculaire est une dégénérescence de la macula liée à l'âge ou une rétinopathie diabétique.
